(19) Europäisches Patentamt
European Patent Office
Office européen des brevets

(11) EP 3 212 650 B1

(12) **EUROPEAN PATENT SPECIFICATION**

(45) Date of publication and mention of the grant of the patent:
**17.02.2021 Bulletin 2021/07**

(21) Application number: **15853752.2**

(22) Date of filing: **23.10.2015**

(51) Int Cl.:
***A61K 31/519*** (2006.01) ***A61K 33/24*** (2019.01)
***A61K 31/555*** (2006.01) ***A61K 31/282*** (2006.01)
***A61K 31/52*** (2006.01) ***A61K 31/337*** (2006.01)
***A61P 35/00*** (2006.01)

(86) International application number:
**PCT/US2015/057062**

(87) International publication number:
**WO 2016/069392 (06.05.2016 Gazette 2016/18)**

(54) **ADMINISTRATION OF UBIQUITIN-ACTIVATING ENZYME INHIBITOR AND CHEMOTHERAPEUTIC AGENTS**

VERABREICHUNG EINES UBIQUITIN-AKTIVIERENDEN ENZYMHEMMERS UND CHEMOTHERAPEUTIKA

ADMINISTRATION D'INHIBITEUR D'ENZYME ACTIVANT L'UBIQUITINE ET AGENTS CHIMIOTHÉRAPEUTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **29.10.2014 US 201462072311 P**

(43) Date of publication of application:
**06.09.2017 Bulletin 2017/36**

(73) Proprietor: **Millennium Pharmaceuticals, Inc.**
**Cambridge, MA 02139 (US)**

(72) Inventors:
- **BENCE, Neil**
  **Belmont, CA 94002 (US)**
- **HUCK, Jessica**
  **Malden, MA 02148 (US)**
- **HYER, Marc**
  **Swampscott, MA 01907 (US)**
- **MILHOLLEN, Michael**
  **Foxborough, MA 02035 (US)**
- **SHI, Judy Qiuju**
  **Concord, MA 01742 (US)**
- **TRAORE, Tary**
  **Woburn, MA 01801 (US)**

(74) Representative: **Harris, Jennifer Lucy et al**
**Kilburn & Strode LLP**
**Lacon London**
**84 Theobalds Road**
**London WC1X 8NL (GB)**

(56) References cited:
**US-A9- 2014 088 096** **US-B2- 8 008 307**
**US-B2- 8 207 177**

- **W.J. GRADISHAR: "Taxanes for the Treatment of Metastatic Breast Cancer", BREAST CANCER : BASIC AND CLINICAL RESEARCH, vol. 6, 1 January 2012 (2012-01-01), XP55474363, New Zealand ISSN: 1178-2234, DOI: 10.4137/BCBCR.S8205**
- **SHALOAM DASARI ET AL: "Cisplatin in cancer therapy: Molecular mechanisms of action", EUROPEAN JOURNAL OF PHARMACOLOGY, vol. 740, 21 July 2014 (2014-07-21), pages 364-378, XP55474371, NL ISSN: 0014-2999, DOI: 10.1016/j.ejphar.2014.07.025**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 212 650 B1

**Description**

FIELD

This present disclosure relates to oncology and to combinations for the treatment of cancer. In particular, the present disclosure provides combinations for use in the treatment of various cancers by administering a ubiquitin-activating enzyme (UAE) inhibitor in combination with one or more chemotherapeutic agents.

BACKGROUND

Cancer is the second most common cause of death in the U.S. and accounts for one of every eight deaths globally (American Cancer Society, Cancer Facts and Figures, 2014). The American Cancer Society expects that in 2014 at least 1,665,540 new cancer cases will be diagnosed in the US and 585,720 Americans are expected to die of cancer, almost 1,600 people per day. Currently available paradigms for treating solid tumors may include systemic treatment such as chemotherapy, hormonal therapy, use of targeted agents and biological agents, either as single agents or in combination. These treatments can be delivered in combination with localized treatments such as surgery or radiotherapy. These anti-cancer paradigms can be use in the curative setting as adjuvant or neo-adjuvant treatments or in the metastatic setting as palliative case for prolonged survival and to help manage symptoms and side-effects. In hematological cancers, stem cell transplants may also be an option in certain cancers as well as chemotherapy and/or radiation. Although medical advances have improved cancer survival rates, there remains a continuing need for new and more effective treatments.

Ubiquitin is a small 76-amino acid protein that is the founding member of a family of posttranslational modifiers known as the ubiquitin-like proteins (Ubls). Ubls play key roles in controlling many biological processes including cell division, cell signaling and the immune response. There are 8 known human Ubl activating enzymes (known as E1s) (Schulman, B.A., and J.W. Harper, 2009, Ubiquitin-like protein activation by E1 enzymes: the apex for downstream signalling pathways, Nat Rev Mol Cell Biol 10:319-331). Ubiquitin and other ubls are activated by a specific E1 enzyme which catalyzes the formation of an acyl-adenylate intermediate with the C-terminal glycine of the ubl. The activated ubl molecule is then transferred to the catalytic cysteine residue within the E1 enzyme through formation of a thioester bond intermediate. The E1-ubl intermediate and an E2 interact, resulting in a thioester exchange wherein the ubl is transferred to the active site cysteine of the E2. The ubl is then conjugated to the target protein, either directly or in conjunction with an E3 ligase, through isopeptide bond formation with the amino group of a lysine side chain in the target protein. Eukaryotic cells possess ~35 ubiquitin E2 enzymes and >500 ubiquitin E3 enzymes. The E3 enzymes are the specificity factors of the ubiquitin pathway which mediate the selective targeting of specific cellular substrate proteins (Deshaies, R.J., and C.A. Joazeiro, 2009, RING domain E3 ubiquitin ligases, Annu Rev Biochem 78:399-434; Lipkowitz, S., and A.M. Weissman, 2011, RINGs of good and evil: RING finger ubiquitin ligases at the crossroads of tumour suppression and oncogenesis, Nat Rev Cancer 11:629-643; Rotin, D., and S. Kumar, 2009, Physiological functions of the HECT family of ubiquitin ligases, Nat Rev Mol Cell Biol 10:398-409).

Two E1 enzymes have been identified for ubiquitin, UAE (ubiquitin-activating enzyme) and UBA6 (Jin, J., et al., 2007, Dual E1 activation systems for ubiquitin differentially regulate E2 enzyme charging, Nature 447:1135-1138). UAE is the E1 responsible for the majority of ubiquitin flux within the cell. UAE is capable of charging each of the approximately ~35 E2 enzymes with the exception of Use1, which is the only E2 known to exclusively work with UBA6 (Jin et al., 2007). Inhibition of UAE is sufficient to dramatically impair the great majority of ubiquitin-dependent cellular processes (Ciechanover, A., et al., 1984, Ubiquitin dependence of selective protein degradation demonstrated in the mammalian cell cycle mutant ts85, Cell 37:57-66; Finley, D., A. et al., 1984, Thermolability of ubiquitin-activating enzyme from the mammalian cell cycle mutant ts85, Cell 37:43-55).

The cellular signals generated by ubiquitin are diverse. Ubiquitin can be attached to substrates as a single entity or as polyubiquitin polymers generated through isopeptide linkages between the C-terminus of one ubiquitin and one of the many lysines on a second ubiquitin. These varied modifications are translated into a variety of cellular signals. For example, conjugation of a lysine 48-linked polyubiquitin chain to a substrate protein is predominantly associated with targeting the protein for removal by the 26S proteasome. A single ubiquitin modification, or monoubiquination, typically affects protein localization and/or function. For example, monoubiquitination modulates the function of Histones 2a and 2b (Chandrasekharan, M.B., et al., 2010, Histone H2B ubiquitination and beyond: Regulation of nucleosome stability, chromatin dynamics and the trans-histone H3 methylation, Epigenetics 5:460-468), controls the nucleocytoplasmic shuttling of PTEN (Trotman, L.C., et al., 2007, Ubiquitination regulates PTEN nuclear import and tumor suppression, Cell 128:141-156), drives localization of the FANCD2 protein to sites of DNA damage (Gregory, R.C., et al., 2003, Regulation of the Fanconi anemia pathway by monoubiquitination, Semin Cancer Biol 13:77-82) and promotes the internalization and endosomal/lysosomal turnover of some cell surface receptors like EGFR (Mosesson, Y., and Y. Yarden, 2006, Monoubiquitylation: a recurrent theme in membrane protein transport. Isr Med Assoc J 8:233-237). Other

forms of polyubiquitination include lysine 11, 29 and 63 chains which play various roles in the cell including the cell cycle, DNA repair and autophagy (Behrends, C., and J.W. Harper, 2011, Constructing and decoding unconventional ubiquitin chains, Nat Struct Mol Biol 18:520-528; Bennett, E.J., and J.W. Harper, 2008, DNA damage: ubiquitin marks the spot, Nat Struct Mol Biol 15:20-22; Komander, D., 2009, The emerging complexity of protein ubiquitination, Biochem Soc Trans 37:937-953).

UAE-initiated ubiquitin conjugation plays an important role in protein homeostasis, cell surface receptor trafficking, transcription factor turnover and cell cycle progression. Many of these processes are important for cancer cell survival and it is believed that tumor cells may have increased sensitivity to UAE inhibition as a result of their rapid growth rate, increased metabolic demands and oncogene fueled protein stress. Preclinical studies with PYZD-4409, a UAE inhibitor, showed that it induced cell death in both leukemia and myeloma cell lines and demonstrated anti-tumor activity in a mouse acute myeloid leukemia (AML model). (Xu, W.G., et al., 2010, The ubiquitin-activating enzyme E1 as a therapeutic target for the treatment of leukemia and multiple myeloma, Blood, 115:2251-59). Thus, UAE represents a novel protein homeostasis target opportunity for the treatment of cancer. US 2014/0088096 discloses ubiquitin activating enzyme (UAE) inhibitors, such as exemplified ((1R,2R,3S,4R)-2,3-dihydroxy-4-(2-(3-(trifluoromethylthio)phenyl)pyrazolo[1,5-a]pyrimidin-7-ylamino)cyclopentyl)methyl sulfamate, which can be used in combination with cytotoxic agents for the treatment of proliferative diseases.

New combinations of therapeutic agents that provide a beneficial effect in the treatment of cancers are desirable in order to prolong patient's lives while maintaining a high quality of life. Further, new combinations may provide an increased benefit as compared to each of the agents alone. This is especially true in the case where the cancers may be resistant or refractory to currently available therapeutic regimens.

## SUMMARY

In one aspect, the present disclosure relates to combinations for use in treating cancer comprising administering an UAE inhibitor and one or more chemotherapeutic agents in combination to a subject in need of such treatment.

In one aspect, the present disclosure relates to a compound for use in treating cancer by administration in combination with one or more of: i) a platin; or ii) a taxane; wherein the compound is ((1R,2R,3S,4R)-2,3-dihydroxy-4-(2-(3 (trifluoromethylthio)phenyl)pyrazolo[1,5-a]pyrimidin-7-ylamino)cyclopentyl)methyl sulfamate (Compound 1) or a pharmaceutically acceptable salt thereof; wherein the platin is carboplatin or oxaliplatin; and wherein the taxane is docetaxel.

In some embodiments, the cancer is breast cancer, colorectal cancer, ovarian cancer, lung cancer, prostate cancer or head and neck cancer.

In some embodiments, the cancer is acute myeloid leukemia, myelodysplastic syndromes, multiple myeloma, or non-Hodgkin's lymphoma.

In some embodiments, ((1R,2R,3S,4R)-2,3-dihydroxy-4-(2-(3-(trifluoromethylthio)phenyl)pyrazolo[1,5-a]pyrimidin-7-ylamino)cyclopentyl)methyl sulfamate (Compound 1) or a pharmaceutically acceptable salt thereof is administered on each of days 1, 4, 8, and 11 of a 21 day schedule.

In some embodiments, ((1R,2R,3S,4R)-2,3-dihydroxy-4-(2-(3-(trifluoromethylthio)phenyl)pyrazolo[1,5-a]pyrimidin-7-ylamino)cyclopentyl)methyl sulfamate (Compound 1) or a pharmaceutically acceptable salt thereof is administered on each of days 1, 8, and 15 of a 21 day schedule.

In some embodiments, ((1R,2R,3S,4R)-2,3-dihydroxy-4-(2-(3-(trifluoromethylthio)phenyl)pyrazolo[1,5-a]pyrimidin-7-ylamino)cyclopentyl)methyl sulfamate (Compound 1) or a pharmaceutically acceptable salt thereof is administered on each of days 1, 8, and 15 of a 28 day schedule.

In some embodiments, ((1R,2R,3S,4R)-2,3-dihydroxy-4-(2-(3-(trifluoromethylthio)phenyl)pyrazolo[1,5-a]pyrimidin-7-ylamino)cyclopentyl)methyl sulfamate (Compound 1) or a pharmaceutically acceptable salt thereof is administered in combination with docetaxel.

In some embodiments, docetaxel is administered on day 1 of a 21 day schedule.

In some embodiments, docetaxel is administered on each of days 1, 8, and 15 of a 21 day schedule.

In some embodiments, docetaxel is administered on each of days 1, 8, and 15 of a 28 day schedule.

In some embodiments, ((1R,2R,3S,4R)-2,3-dihydroxy-4-(2-(3-(trifluoromethylthio)phenyl)pyrazolo[1,5-a]pyrimidin-7-ylamino)cyclopentyl)methyl sulfamate (Compound 1) or a pharmaceutically acceptable salt thereof is administered in combination with carboplatin or oxaliplatin.

In some embodiments, ((1R,2R,3S,4R)-2,3-dihydroxy-4-(2-(3-(trifluoromethylthio)phenyl)pyrazolo[1,5-a]pyrimidin-7-ylamino)cyclopentyl)methyl sulfamate (Compound 1) or a pharmaceutically acceptable salt thereof is administered in combination with carboplatin.

In some embodiments, carboplatin or oxaliplatin is administered on day 1 of a 21 day schedule.

In some embodiments, carboplatin or oxaliplatin is administered on each of days 1, 8, and 15 of a 21 day schedule.

In some embodiments, carboplatin or oxaliplatin is administered on each of days 1, 8, and 15 of a 28 day schedule.

Also disclosed is a combination of ((1R,2R,3S,4R)-2,3-dihydroxy-4-(2-(3-(trifluoromethylthio)phenyl)pyrazolo[1,5-a]pyrimidin-7-ylamino)cyclopentyl)methyl sulfamate (Compound 1) or a pharmaceutically acceptable salt thereof and carboplatin and paclitaxel.

In some embodiments, ((1R,2R,3S,4R)-2,3-dihydroxy-4-(2-(3-(trifluoromethylthio)phenyl)pyrazolo[1,5-a]pyrimidin-7-ylamino)cyclopentyl)methyl sulfamate (Compound 1) or a pharmaceutically acceptable salt thereof is administered in combination with carboplatin and docetaxel.

In one aspect, the present disclosure relates to a kit comprising a medicament for use in treating cancer in a subject in need of such treatment. The kit comprises a medicament comprising an UAE inhibitor, and instructions for administering the UAE inhibitor and the one or more chemotherapeutic agents, as defined in claims; or the kit comprises a medicament comprising the one or more chemotherapeutic agents, and instructions for administering the one or more chemotherapeutic agents and a UAE inhibitor, as defined in claims. The kit can contain both a medicament comprising an UAE inhibitor and a medicament comprising one or more chemotherapeutic agents, and instructions for administering the UAE inhibitor and the one or more chemotherapeutic agents, as defined in claims.

In one aspect, the present disclosure relates to a medicament for use in treating cancer in a subject in need of such treatment. The medicament comprises an UAE inhibitor and one or more chemotherapeutic agents, as defined in the claims.

The present invention is defined in the appended claims. Subject matters which are not encompassed by the scope of the claims do not form part of the present invention.

BRIEF DESCRIPTION OF THE FIGURES

FIG. 1 shows a plot of tumor volume as a function of time in a PHTX-132Lu xenograft model following administration of Compound 1 and carboplatin to mice.

FIG. 2a and 2b each shows a plot of tumor volume as a function of time in a SKOV-3 xenograft model following administration of Compound 1 and carboplatin to mice.

FIG. 3 shows a plot of tumor volume as a function of time in a CTG-0486 xenograft model following administration of Compound 1 and carboplatin to mice.

FIG. 4 shows a plot of tumor volume as a function of time in a PHTX-24C xenograft model following administration of Compound 1 and oxaliplatin to mice.

FIG. 5 shows a plot of tumor volume as a function of time in a PHTX-02B xenograft model following administration of Compound 1 and docetaxel to mice.

FIG. 6a and 6b each shows a plot of tumor volume as a function of time in a Calu-6 xenograft model following administration of Compound 1 and docetaxel to mice.

FIG. 7 shows a plot of tumor volume as a function of time in a PHTX-14B xenograft model following administration of Compound 1 and docetaxel to mice.

FIG. 8 shows a plot of tumor volume as a function of time in a CTG-0159 xenograft model following administration of Compound 1 and docetaxel to mice.

FIG. 9 (reference) shows a plot of tumor volume as a function of time in a PHTX-14B xenograft model following administration of Compound 1 and paclitaxel to mice.

DESCRIPTION

**Definitions and Abbreviations.**

| | |
|---|---|
| AUC | area under the plasma concentration versus time curve |
| BSA | body surface area |
| CR | complete response |
| MTD | maximum tolerated dose |
| UAE | Ubiquitin-activating enzyme |
| PR | partial response |
| BIW | twice weekly |
| QD | once daily |
| NSCLC | non-small cell lung cancer |
| SCLC | small cell lung cancer |

As used herein, the term "cancer" refers to a cellular disorder characterized by uncontrolled or dysregulated cell proliferation, decreased cellular differentiation, inappropriate ability to invade surrounding tissue, and/or ability to establish new growth at ectopic sites. The term "cancer" includes solid tumors and hematological tumors. The term "cancer" encompasses diseases of skin, tissues, organs, bone, cartilage, blood, and vessels. The term "cancer" further encompasses primary and metastatic cancers.

As used herein, "clinically effective amount" means an amount of a therapeutic substance that is sufficient upon appropriate administration to a patient (a) to cause a detectable decrease in the severity of the disorder or disease state being treated; (b) to ameliorate or alleviate the patient's symptoms of the disease or disorder; or (c) to slow or prevent advancement of, or otherwise stabilize or prolong stabilization of, the disorder or disease state being treated (*e.g.*, prevent additional tumor growth of a cancer).

When more than one therapeutic substance is being administered, the "clinically effective total amount" means that the sum of the individual amounts of each therapeutic substance meets the definition of "clinically effective amount" even if the individual amounts of any number of the individual therapeutic substances would not. For example, if 10 mg of A were not a clinically effective amount, and 20 mg of B were not a clinically effective amount, but the administration of 10 mg A + 20 mg B resulted in at least one of the results enumerated for the definition of "clinically effective amount", then the sum of 10 mg A + 20 mg B would be considered a "clinically effective total amount".

In any form or composition, the administered dose(s) or the clinically effective (total) amount can be expressed as amount(s) of therapeutic substance(s) per patient as either based on (i) BSA, *e.g.*, as $mg/m^2$, or (ii) amount e.g. as mg.

As used herein, "patient" means a human being diagnosed with, exhibiting symptoms of or otherwise believed to be afflicted with a disease, disorder or condition.

As used herein, "body surface area" (BSA) is calculated using a standard nomogram, e.g.,

$$\text{BSA}\ (m^2) = \sqrt{\frac{\text{Ht (cm) x Wt (kg)}}{3600}} \quad \text{or} \quad \text{BSA} = \sqrt{\frac{\text{Ht (in) x Wt (lb)}}{3131}}$$

As used herein, dosing for carboplatin is based upon an estimate of the GFR (glomerular filtration rate) and the desired level of drug exposure, according to the area under the curve of concentration X time (AUC, mg/mL x min), rather than the more common dosing calculation based upon the body surface area ($mg/m^2$). For a desired target AUC (which typically varies between 5 and 7 mg/mL x min) and the estimated GFR, the dose of carboplatin is then calculated by use of the Calvert formula:

$$\text{Total carboplatin dose, mg} = \text{Target AUC X (estimated creatinine clearance + 25).}$$

Because of potential changes in weight or renal function, this calculation should be repeated prior to each administered course of carboplatin.

The estimation of the GFR is based upon a calculation of creatinine clearance according to the Cockcroft-Gault

Equation (Cockcroft DW, Gault MH. Prediction of creatinine clearance from serum creatinine. Nephron. 1976; 16(1):31-41):

For males:

$$\text{Creatinine Clearance} = (140\text{-age [years] x weight [kg]})])/72 \text{ x (serum creatinine [mg/dL])}$$

For females:

$$\text{Creatinine Clearance} = 0.85\ (140\text{-age[years] x weight [kg]})\ /72 \text{ x (serum creatinine [mg/dL])}.$$

As used herein, the illustrative terms "include", "such as", "for example" and the like (and variations thereof, e.g., "includes" and "including", "examples"), unless otherwise specified, are intended to be non-limiting. That is, unless explicitly stated otherwise, such terms are intended to imply "but not limited to", e.g., "including" means including but not limited to.

Unless otherwise stated, structures depicted herein are meant to include chemical entities which differ only in the presence of one or more isotopically enriched atoms. For example, chemical entities having the present structure except for the replacement of a hydrogen atom by a deuterium or tritium, or the replacement of a carbon atom by a $^{13}$C- or $^{14}$C-enriched carbon are within the scope of the invention.

Unless stereochemical configuration is denoted, structures depicted herein are meant to include all stereochemical forms of the structure, i.e., the *R* and *S* configurations for each asymmetric center. Therefore, unless otherwise indicated, single stereochemical isomers as well as enantiomeric, racemic and diastereomeric mixtures of the present chemical entities are within the scope of the invention. When a stereochemical configuration is denoted for a compound, the diastereoisomeric or enantiomeric excess of the compound is at least 99.0%, 99.5%, 99.6%, 99.7%, 99.8% or 99.9%.

## Detailed Description

In some embodiments, the present disclosure relates to a method of treating cancer in a patient by administering to a patient a combination of an UAE inhibitor or pharmaceutically acceptable salt thereof and one or more chemotherapeutic agents, wherein the one or more chemotherapeutic agents is: (i) a taxane; (ii) a platin; or a pharmaceutically acceptable salt thereof.

UAE inhibitors are disclosed in patent application publications WO2013/123169 and US 2014/0088096. In one embodiment, the UAE inhibitor is a compound having the following structure (Compound 1):

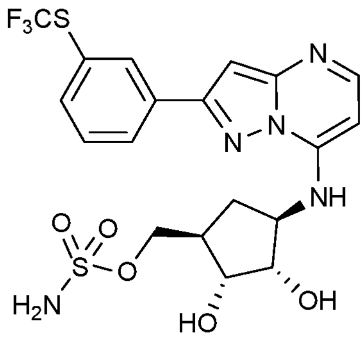

**(Compound 1)**;

or a pharmaceutically acceptable salt thereof. The Compound 1 is named ((1R,2R,3S,4R)-2,3-dihydroxy-4-(2-(3-(trifluoromethylthio)phenyl)pyrazolo[1,5-a]pyrimidin-7-ylamino)cyclopentyl)methyl sulfamate.

In some embodiments, the UAE inhibitor is ((1R,2R,3S,4R)-2,3-dihydroxy-4-(2-(3-(trifluoromethylthio)phenyl)pyrazolo[1,5-a]pyrimidin-7-ylamino)cyclopentyl)methyl sulfamate (Compound 1) or a crystalline form thereof.

In some embodiments, the present disclosure relates to a combination for use in treating cancer in a patient by administering to a patient a combination of Compound 1 or pharmaceutically acceptable salt thereof and one or more chemotherapeutic agents, wherein the one or more chemotherapeutic agents is: (i) docetaxel; (ii) carboplatin or oxaliplatin; or a pharmaceutically acceptable salt thereof.

In some embodiments, the present disclosure relates to a combination for use in treating cancer in a patient

by administering to a patient a combination of Compound 1 or pharmaceutically acceptable salt thereof and one or more chemotherapeutic agents, wherein the one or more chemotherapeutic agents is docetaxel or a pharmaceutically acceptable salt thereof.

In some embodiments, the present disclosure relates to a combination for use in treating cancer in a patient by administering to a patient a combination of Compound 1 or pharmaceutically acceptable salt thereof and one or more chemotherapeutic agents, wherein the one or more chemotherapeutic agents is carboplatin or oxaliplatin or a pharmaceutically acceptable salt thereof.

In some embodiments, the present disclosure relates to a combination for use in treating cancer in a patient by administering to a patient a combination of Compound 1 or pharmaceutically acceptable salt thereof and one or more chemotherapeutic agents, wherein the one or more chemotherapeutic agents is docetaxel or a pharmaceutically acceptable salt thereof, and carboplatin or oxaliplatin or a pharmaceutically acceptable salt thereof.

In another aspect, the present disclosure relates to the combination of Compound 1 or a pharmaceutically acceptable salt and one or more chemotherapeutic agents, wherein the one or more chemotherapeutic agents is: (i) a taxane; (ii) a platin; or a pharmaceutically acceptable salt thereof for the treatment of cancer, as defined in the claims.

In another aspect, the present disclosure relates to a kit for treating cancer comprising at least one medicament comprising at least one dose of Compound 1 or a pharmaceutically acceptable salt thereof, and at least one medicament comprising at least one dose of one or more of: (i) docetaxel; or (ii) carboplatin or oxaliplatin; or a pharmaceutically acceptable salt thereof, said kit for treating cancer further comprising dosing instructions for administering the medicaments for treatment of the subject in recognized need thereof.

Compound 1 or a pharmaceutically acceptable salt thereof can be administered in combination with the one or more chemotherapeutic agents, as defined in the claims, in a single dosage form or as a separate dosage forms. In one embodiment, when administered as a separate dosage form, the one or more chemotherapeutic agents can be administered prior to, at the same time as, or following administration of Compound 1. In some embodiments, when administered as a separate dosage form, one or more doses of Compound 1 or a pharmaceutically acceptable salt thereof, may be administered prior to the one or more chemotherapeutic agents. In some embodiments, the one or more chemotherapeutic agents is administered prior to the administration of Compound 1 or a pharmaceutically acceptable salt thereof. As used herein, the administration in "combination" of Compound 1 and a chemotherapeutic agent refers not only to simultaneous or sequential administration of the two agents, but also to the administration of both compounds during a single treatment cycle, as understood by one skilled in the art. When Compound 1 or a pharmaceutically acceptable salt thereof is administered in combination with the one or more chemotherapeutic agents a clinically effective total amount is administered.

In some embodiments, Compound 1 or a pharmaceutically acceptable salt is administered intravenously (IV). Pharmaceutical compositions suitable for IV administration of Compound 1 or a pharmaceutically acceptable salt are described in patent application publications WO2013/123169 and US 2013/0217682. In some embodiments, the one or more chemotherapeutic agents is administered intravenously (IV).

In some embodiments, the one or more chemotherapeutic agent is one chemotherapeutic agent. In some embodiments, the one or more chemotherapeutic agents is two chemotherapeutic agents. In some embodiments, the one or more chemotherapeutic agents is three chemotherapeutic agents.

In some embodiments, the chemotherapeutic agent is a platinum containing compound ("platin"). Platinum containing compounds include agents such as cisplatin, carboplatin, oxaliplatin, satraplatin, picoplatin, nedaplatin and triplatin. Platinum containing chemotherapeutic agents cause crosslinking of DNA as monoadduct, interstrand crosslinks, intrastrand crosslinks or DNA protein crosslinks. The resulting crosslinking inhibits DNA repair and/or DNA synthesis in cancer cells. These agents are sometimes described as being alkylating-like agents despite the fact that they do not have an alkyl group. Cisplatin was the first platinum containing compound to be discovered and was first approved by the U.S. Food and Drug Administration in 1978. Carboplatin was introduced in the 1980s and has been demonstrated to have lower side-effects than cisplatin in ovarian cancer and lung cancer (Hartmann and Lipp, Exper. Opin. Pharmacother. 2003, 4(6) 889-901).

In some embodiments, the platin is carboplatin. In some embodiments, the platin is oxaliplatin.

In some embodiments, the chemotherapeutic agent is docetaxel. Taxanes are diterpenes produced by the plants of the genus Taxus (yew trees). Taxanes were first discovered and isolated from this natural source but are mostly now produced by synthetic or semi-synthetic methods. The principle mechanism by which taxanes exert their effect is the disruption of microtubule function during cell division, thereby preventing effective growth and division of cancer cells.

Taxane agents include paclitaxel, docetaxel and nab-paclitaxel. Paclitaxel was originally isolated from the bark of the Pacific yew tree and was subsequently produced in a semi-synthetic manner. Paclitaxel was first approved by the U.S. Food and Drug Administration in 1992. Docetaxel is also derived semi-synthetically from the needles of the yew tree. Docetaxel is approved by the U.S. Food and Drug Administration for the treatment of advanced breast, lung, and ovarian cancer. An alternative formulation of paclitaxel where the paclitaxel is bound to albumin nano-particles, known as nab-paclitaxel [marketed as Abraxane (Celgene Corporation)] is also approved by the U.S. Food and Drug Administration for certain types of metastatic breast cancer. In some embodiments, the taxane is docetaxel.

In some embodiments, the one or more chemotherapeutic agents is carboplatin and docetaxel.

In some embodiments, the cancer is a solid tumor. Examples of solid tumors include pancreatic cancer; bladder cancer, including invasive bladder cancer; colorectal cancer; thyroid cancer; gastric cancer; breast cancer, including metastatic breast cancer; prostate cancer, including androgen-dependent and androgen-independent prostate cancer; renal cancer, including, e.g., metastatic renal cell carcinoma; liver cancer including e.g. hepatocellular cancer and intrahepatic bile duct; lung and bronchus cancer including non-small cell lung cancer (NSCLC), squamous lung cancer, brochioloalveolar carcinoma (BAC), adenocarcinoma of the lung, and small cell lung cancer (SCLC); ovarian cancer including, e.g., progressive epithelial and primary peritoneal cancer; cervical cancer; uterine cancer including e.g. uterine corpus and uterine cervix; endometrial cancer; esophageal cancer; gastro-esophageal (GE) junction cancer; head and neck cancer, including, e.g., squamous cell carcinoma of the head and neck, nasopharyngeal caner, oral cavity and pharynx; skin cancer, including squamous cell skin cancer, basal cell skin cancer, Merkel cell carcinoma and melanoma; neuroendocrine cancer, including metastatic neuroendocrine tumors; brain cancer, including, e.g., glioma/glioblastoma, anaplastic oligodendroglioma, adult glioblastoma multiforme, and adult anaplastic astrocytoma; neuroendocrine cancer, including metastatic neuroendocrine tumors; bone cancer; penile cancer; anal cancer; and soft tissue sarcoma.

In some embodiments, the cancer is breast cancer, ovarian cancer, endometrial cancer, colorectal cancer, cervical cancer, lung cancer, gastric cancer, prostate cancer, testicular cancer, bladder cancer, esophageal cancer, melanoma, soft tissue sarcoma or head and neck cancer. In some embodiments, the cancer is breast cancer, colorectal cancer, ovarian cancer, lung cancer, prostate cancer, head and neck cancer, gastric cancer, esophageal cancer, or gastro-esophageal junction cancer. In some embodiments, the cancer is breast cancer, colorectal cancer, ovarian cancer, lung cancer, prostate cancer or head and neck cancer. In some embodiments, the cancer is breast cancer, colorectal cancer, ovarian cancer, or lung cancer. In some embodiments, the cancer is breast cancer, colorectal cancer, ovarian cancer, or non-small cell lung cancer. In some embodiments, the cancer is gastric cancer, esophageal cancer, or gastro-esophageal junction cancer.

In some embodiments, the cancer is lung cancer. Lung cancer includes different sub-types such as small cell lung cancer (SCLC); non-small cell lung cancer (NSCLC) including squamous NSCLC; bronchioloalveolar carcinoma (BAC); and adenocarcinoma. In some embodiments, the cancer is small cell lung cancer. In some embodiments, the cancer is non-small cell lung cancer.

In some embodiments, the cancer is breast cancer. Breast cancer includes different sub-types such as luminal A, luminal B, triple-negative (basal-like) and HER-2 type. In some embodiments, the cancer is triple-negative breast cancer.

In some embodiments, the cancer is ovarian cancer. Ovarian cancer includes different sub-types such as epithelial, germ-cell and sex-cord stromal. Primary peritoneal carcinoma is a related cancer that starts in the lining of the pelvis and abdomen. In some embodiments, the cancer is epithelial ovarian cancer.

In some embodiments, the cancer is prostate cancer. Prostate cancer includes androgen-dependent and androgen independent prostate cancer and adenocarcinomas.

In some embodiments, the cancer is colorectal cancer. Adenocarcinoma is the most common type of colorectal cancer. Other colorectal cancers may include gastrointestinal carcinoid tumors, gastrointestinal stromal tumors, and squamous cell carcinoma.

In some embodiments, the cancer is gastric cancer. Adenocarcinoma is the most common type of gastric cancer. Other gastric cancers may include gastrointestinal carcinoid tumors, gastrointestinal stromal tumors, and lymphomas.

In some embodiments, the cancer is esophageal cancer. The most common types of esophageal cancer are squamous cell carcinoma and adenocarcinoma. Gastro-esophageal cancer is a related cancer that develops at the point where the esophagus joins the stomach.

In some embodiments, the cancer is head and neck cancer. Head and neck cancer are those that arise in the head and neck region and the cancer may be found in areas such as nasal cavities, sinuses, lips, mouth, salivary glands, pharynx or larynx. 90% of head and neck cancers are squamous cell carcinomas (SCCHN), which originate from the mucosal lining (epithelium) of these regions.

In some embodiments, the cancer is a hematological cancer. Non-limiting examples of hematologic malignancies include acute myeloid leukemia (AML); chronic myelogenous leukemia (CML), including accelerated CML and CML blast phase (CML-BP); acute lymphoblastic leukemia (ALL); chronic lymphocytic leukemia (CLL); Hodgkin's disease (HD); non-Hodgkin's lymphoma (NHL), including follicular lymphoma and mantle cell lymphoma; B-cell lymphoma including diffuse large B-cell lymphoma (DLBCL); T-cell lymphoma; multiple myeloma (MM); amyloidosis; Waldenstrom's macroglobulinemia; myelodysplastic syndromes (MDS), including refractory anemia (RA), refractory anemia with ringed siderblasts (RARS), (refractory anemia with excess blasts (RAEB), and RAEB in transformation (RAEB-T); and myeloproliferative syndromes.

In some embodiments, the cancer is acute myeloid leukemia, myelodysplastic syndromes, multiple myeloma,

or non-Hodgkin's lymphoma.

In some embodiments, Compound 1 or a pharmaceutically acceptable salt thereof is administered on a twice-weekly schedule. In some embodiments, Compound 1 or a pharmaceutically acceptable salt thereof is administered on each of days 1, 4, 8, and 11 of a 21 day schedule. In some embodiments, Compound 1 or a pharmaceutically acceptable salt thereof is administered on a weekly schedule. In some embodiments, Compound 1 or a pharmaceutically acceptable salt thereof is administered on each of days 1, 8, and 15 of a 21 day schedule. In some embodiments, Compound 1 or a pharmaceutically acceptable salt thereof is administered on each of days 1, 8, and 15 of a 28 day schedule.

In some embodiments, the amount of Compound 1 or a pharmaceutically acceptable salt thereof that is administered on each day of dosing is about 4 mg to about 65 mg. In some embodiments, the amount of Compound 1 or a pharmaceutically acceptable salt thereof that is administered on each day of dosing is about 5 mg to about 65 mg. In some embodiments, the amount of Compound 1 or a pharmaceutically acceptable salt thereof that is administered on each day of dosing is about 15 mg to about 55 mg. In some embodiments, the amount of Compound 1 or a pharmaceutically acceptable salt thereof that is administered on each day of dosing is between about 18 mg to about 43 mg. In some embodiments, the amount of Compound 1 or a pharmaceutically acceptable salt thereof that is administered on each day of dosing is about 18 mg, about 24 mg, about 30 mg, about 36 mg, about 43 mg, about 51 mg, or about 61 mg. All dosing amounts refer to the amount of Compound 1 administered, and do not include the weight amount of any pharmaceutically acceptable salt.

In some embodiments, docetaxel is administered on day 1 of a 21 day schedule. In some embodiments, carboplatin or oxaliplatin is administered on day 1 of a 21 day schedule. In some embodiments, carboplatin or oxaliplatin and docetaxel are administered on day 1 of a 21 day schedule.

In some embodiments, docetaxel is administered on day 1, 8, and 15 of a 21 day schedule. In some embodiments, carboplatin or oxaliplatin is administered on each of days 1, 8, and 15 of a 21 day schedule. In some embodiments, docetaxel and carboplatin or oxaliplatin are administered on each of days 1, 8, and 15 of a 21 day schedule.

In some embodiments, docetaxel is administered on day 1, 8, and 15 of a 28 day schedule. In some embodiments, carboplatin or oxaliplatin is administered on each of days 1, 8, and 15 of a 28 day schedule. In some embodiments, carboplatin or oxaliplatin and docetaxel are administered on each of days 1, 8, and 15 of a 28 day schedule.

In some embodiments, docetaxel is administered on day 1 of a 21 day schedule. In some embodiments, the amount of docetaxel that is administered on day 1 of a 21 day schedule is about 60 $mg/m^2$. In some embodiments, the amount of docetaxel that is administered on day 1 of a 21 day schedule is about 75 $mg/m^2$. In some embodiments, the amount of docetaxel that is administered on day 1 of a 21 day schedule is about 100 $mg/m^2$.

In some embodiments, docetaxel is administered on each of days 1, 8, and 15 of a 21 day schedule. In some embodiments, the amount of docetaxel that is administered on each of days 1, 8, and 15 of a 21 day schedule is about 25 $mg/m^2$ to about 36 $mg/m^2$. In some embodiments, docetaxel is administered on each of days 1, 8, and 15 of a 28 day schedule. In some embodiments, the amount of docetaxel that is administered on each of days 1, 8, and 15 of a 28 day schedule is about 25 $mg/m^2$ to about 36 $mg/m^2$.

In some disclosures, paclitaxel is administered of day 1 of a 21 day schedule. In some disclosures, the amount of paclitaxel that is administered on day 1 of a 21 day schedule is about 135 $mg/m^2$ to about 200 $mg/m^2$. In some disclosures, the amount of paclitaxel that is administered on day 1 of a 21 day schedule is about 135 $mg/m^2$. In some disclosures, the amount of paclitaxel that is administered on day 1 of a 21 day schedule is about 175 $mg/m^2$. In some disclosures, the amount of paclitaxel that is administered on day 1 of a 21 day schedule is about 200 $mg/m^2$.

In some disclosures, paclitaxel is administered on each of days 1, 8, and 15 of a 21 day schedule. In some disclosures, the amount of paclitaxel that is administered on each of days 1, 8, and 15 of a 21 day schedule is about 60 $mg/m^2$ to about 100 $mg/m^2$. In some disclosures, the amount of paclitaxel that is administered on each of days 1, 8, and 15 of a 21 day schedule is about 60 $mg/m^2$, about 70 $mg/m^2$, about 80 $mg/m^2$, about 90 $mg/m^2$, or about 100 $mg/m^2$. In some disclosures, paclitaxel is administered on each of days 1, 8, and 15 of a 28 day schedule. In some disclosures, the amount of paclitaxel that is administered on each of days 1, 8, and 15 of a 28 day schedule is about 60 $mg/m^2$ to about 100 $mg/m^2$. In some disclosures, the amount of paclitaxel that is administered on each of days 1, 8, and 15 of a 28 day schedule is about 60 $mg/m^2$, about 70 $mg/m^2$, about 80 $mg/m^2$, about 90 $mg/m^2$, or about 100 $mg/m^2$.

In some embodiments, carboplatin is administered on day 1 of a 21 day schedule. In some embodiments, the amount of carboplatin that is administered on day 1 of a 21 day schedule is AUC 6 (calculated as per the Calvert calculation above). In some embodiments, the amount of carboplatin that is administered on day 1 of a 21 day schedule is AUC 5.

In some embodiments, carboplatin is administered on each of days 1, 8, and 15 of a 21 day schedule. In some embodiments, the amount of carboplatin that is administered on each of days 1, 8, and 15 of a 21 day schedule is AUC 2. In some embodiments, carboplatin is administered on each of days 1, 8, and 15 of a 28 day schedule. In some embodiments, the amount of carboplatin that is administered on each of days 1, 8, and 15 of a 28 day schedule is AUC 2.

In some disclosures, cisplatin is administered on day 1 of a 21 day schedule. In some disclosures, the amount of cisplatin administered on day 1 of a 21 day schedule is about 75 $mg/m^2$ to about 100 $mg/m^2$. In some disclosures,

the amount of cisplatin administered on day 1 of a 21 day schedule is about 50 mg/$m^2$ to about 70 mg/$m^2$.

In some disclosures, cisplatin is administered on each of days 1, 8, and 15 of a 21 day schedule. In some disclosures, the amount of cisplatin that is administered on each of days 1, 8, and 15 of a 21 day schedule is about 25 mg/$m^2$ to about 50 mg/$m^2$. In some disclosures, cisplatin is administered on each of days 1, 8, and 15 of a 28 day schedule. In some disclosures, the amount of cisplatin that is administered on each of days 1, 8, and 15 of a 28 day schedule is about 25 mg/$m^2$ to about 50 mg/$m^2$.

In some embodiments, Compound 1 or a pharmaceutically acceptable salt thereof is administered on each of days 1, 4, 8, and 11 of a 21 day schedule, and docetaxel is administered on day 1 of a 21 day schedule. In some embodiments, Compound 1 or a pharmaceutically acceptable salt thereof is administered on each of days 1, 4, 8, and 11 of a 21 day schedule, and carboplatin or oxaliplatin is administered on day 1 of a 21 day schedule. In some embodiments, Compound 1 or a pharmaceutically acceptable salt thereof is administered on each of days 1, 4, 8, and 11 of a 21 day schedule, and docetaxel and carboplatin or oxaliplatin are each administered on day 1 of a 21 day schedule.

In some embodiments, Compound 1 or a pharmaceutically acceptable salt thereof is administered on each of days 1, 4, 8, and 11 of a 21 day schedule, and docetaxel is administered on each of days 1, 8, and 15 of a 21 day schedule. In some embodiments, Compound 1 or a pharmaceutically acceptable salt thereof is administered on each of days 1, 4, 8, and 11 of a 21 day schedule, and carboplatin or oxaliplatin is administered on each of days 1, 8, and 15 of a 21 day schedule.

In some embodiments, Compound 1 or a pharmaceutically acceptable salt thereof is administered on each of days 1, 8, and 15 of a 21 day schedule, and docetaxel is administered on day 1 of a 21 day schedule. In some embodiments, Compound 1 or a pharmaceutically acceptable salt thereof is administered on each of days 1, 8, and 15 of a 21 day schedule, and carboplatin or oxaliplatin is administered on day 1 of a 21 day schedule. In some embodiments, Compound 1 or a pharmaceutically acceptable salt thereof is administered on each of days 1, 8, and 15 of a 21 day schedule, and docetaxel and carboplatin or oxaliplatin are each administered on day 1 of a 21 day schedule.

In some embodiments, Compound 1 or a pharmaceutically acceptable salt thereof is administered on each of days 1, 8, and 15 of a 21 day schedule, and docetaxel is administered on each of days 1, 8, and 15 of a 21 day schedule. In some embodiments, Compound 1 or a pharmaceutically acceptable salt thereof is administered on each of days 1, 8, and 15 of a 21 day schedule, and carboplatin or oxaliplatin is administered on each of days 1, 8, and 15 of a 21 day schedule.

In some embodiments, Compound 1 or a pharmaceutically acceptable salt thereof is administered on each of days 1, 8, and 15 of a 28 day schedule, and docetaxel is administered on day 1 of a 28 day schedule. In some embodiments, Compound 1 or a pharmaceutically acceptable salt thereof is administered on each of days 1, 8, and 15 of a 28 day schedule, and carboplatin or oxaliplatin is administered on day 1 of a 28 day schedule. In some embodiments, Compound 1 or a pharmaceutically acceptable salt thereof is administered on each of days 1, 8, and 15 of a 28 day schedule, and docetaxel and carboplatin or oxaliplatin are each administered on day 1 of a 28 day schedule.

In some embodiments, Compound 1 or a pharmaceutically acceptable salt thereof is administered on each of days 1, 8, and 15 of a 28 day schedule, and docetaxel is administered on each of days 1, 8, and 15 of a 28 day schedule. In some embodiments, Compound 1 or a pharmaceutically acceptable salt thereof is administered on each of days 1, 8, and 15 of a 28 day schedule, and carboplatin or oxaliplatin is administered on each of days 1, 8, and 15 of a 28 day schedule.

In some disclosures, Compound 1 or a pharmaceutically acceptable salt thereof is administered on each of days 1, 4, 8, and 11 of a 21 day schedule, and paclitaxel is administered on day 1 of a 21 day schedule. In some disclosures, Compound 1 or a pharmaceutically acceptable salt thereof is administered on each of days 1, 8, and 15 of a 28 day schedule, and paclitaxel is administered on each of days 1, 8, and 15 of a 28 day schedule.

In some embodiments, Compound 1 or a pharmaceutically acceptable salt thereof is administered on each of days 1, 4, 8, and 11 of a 21 day schedule, and carboplatin is administered on day 1 of a 21 day schedule. In some embodiments, Compound 1 or a pharmaceutically acceptable salt thereof is administered on each of days 1, 8, and 15 of a 28 day schedule, and carboplatin is administered on each of days 1, 8, and 15 of a 28 day schedule.

In some disclosures, Compound 1 or a pharmaceutically acceptable salt thereof is administered on each of days 1, 4, 8, and 11 of a 21 day schedule, and carboplatin and paclitaxel are administered on day 1 of a 21 day schedule. In some disclosures, Compound 1 or a pharmaceutically acceptable salt thereof is administered on each of days 1, 8, and 15 of a 28 day schedule, and carboplatin and paclitaxel are administered on day 1 of a 21 day schedule.

In some disclosures, wherein the cancer is breast cancer, colorectal cancer, ovarian cancer, or lung cancer, the method comprises administering to a patient in need of such treatment a combination of Compound 1 or a pharmaceutically acceptable salt thereof and paclitaxel.

In some embodiments, wherein the cancer is breast cancer, colorectal cancer, ovarian cancer, or lung cancer, the method comprises administering to a patient in need of such treatment a combination of Compound 1 or a pharmaceutically acceptable salt thereof and carboplatin.

In some disclosures, wherein the cancer is breast cancer, colorectal cancer, ovarian cancer, or lung cancer,

the method comprises administering to a patient in need of such treatment a combination of Compound 1 or a pharmaceutically acceptable salt thereof, carboplatin and paclitaxel.

**Therapeutic Substance; Pharmaceutical Compositions.**

Any of therapeutic agents described herein can be in the form of a pharmaceutically acceptable salt. In some embodiments, such salts are derived from inorganic or organic acids or bases. For reviews of suitable salts, *see, e.g.,* Berge et al., J. Pharm. Sci., 1977, 66, 1-19 and Remington: The Science and Practice of Pharmacy, 20th Ed., A. Gennaro (ed.), Lippincott Williams & Wilkins (2000).

Examples of suitable acid addition salts include acetate, adipate, alginate, aspartate, benzoate, benzene sulfonate, bisulfate, butyrate, citrate, camphorate, camphor sulfonate, cyclopentanepropionate, digluconate, dodecylsulfate, ethanesulfonate, fumarate, lucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate and undecanoate.

Examples of suitable base addition salts include ammonium salts; alkali metal salts, such as sodium and potassium salts; alkaline earth metal salts, such as calcium and magnesium salts; salts with organic bases, such as dicyclohexylamine salts, *N*-methyl-D-glucamine; and salts with amino acids such as arginine, lysine, and the like.

For example, Berge lists the following FDA-approved commercially marketed salts: anions acetate, besylate (benzenesulfonate), benzoate, bicarbonate, bitartrate, bromide, calcium edetate (ethylenediaminetetraacetate), camsylate (camphorsulfonate), carbonate, chloride, citrate, dihydrochloride, edetate (ethylenediaminetetraacetate), edisylate (1,2-ethanedisulfonate), estolate (lauryl sulfate), esylate (ethanesulfonate), fumarate, gluceptate (glucoheptonate), gluconate, glutamate, glycollylarsanilate (glycollamidophenylarsonate), hexylresorcinate, hydrabamine (*N*,*N'*-di(dehydroabietyl)ethylenediamine), hydrobromide, hydrochloride, hydroxynaphthoate, iodide, isethionate (2-hydroxyethanesulfonate), lactate, lactobionate, malate, maleate, mandelate, mesylate (methanesulfonate), methylbromide, methylnitrate, methylsulfate, mucate, napsylate (2-naphthalenesulfonate), nitrate, pamoate (embonate), pantothenate, phosphate/diphosphate, polygalacturonate, salicylate, stearate, subacetate, succinate, sulfate, tannate, tartrate, teoclate (8-chlorotheophyllinate) and triethiodide; organic cations benzathine (*N,N'*-dibenzylethylenediamine), chloroprocaine, choline, diethanolamine, ethylenediamine, meglumine (*N*-methylglucamine) and procaine; and metallic cations aluminum, calcium, lithium, magnesium, potassium, sodium and zinc.

Berge additionally lists the following non-FDA-approved commercially marketed (outside the United States) salts: anions adipate, alginate, aminosalicylate, anhydromethylenecitrate, arecoline, aspartate, bisulfate, butylbromide, camphorate, digluconate, dihydrobromide, disuccinate, glycerophosphate, hemisulfate, hydrofluoride, hydroiodide, methylenebis(salicylate), napadisylate (1,5-naphthalenedisulfonate), oxalate, pectinate, persulfate, phenylethylbarbiturate, picrate, propionate, thiocyanate, tosylate and undecanoate; organic cations benethamine (*N*-benzylphenethylamine), clemizole (1-*p*-chlorobenzyl-2-pyrrolildine-1'-ylmethylbenzimidazole), diethylamine, piperazine and tromethamine (tris(hydroxymethyl)aminomethane); and metallic cations barium and bismuth.

As used herein, "pharmaceutically acceptable carrier" refers to a material that is compatible with a recipient subject (a human) and is suitable for delivering an active agent to the target site without terminating the activity of the agent. The toxicity or adverse effects, if any, associated with the carrier preferably are commensurate with a reasonable risk/benefit ratio for the intended use of the active agent.

The pharmaceutical compositions for use in the methods of the present disclosure can be manufactured by methods well known in the art such as conventional granulating, mixing, dissolving, encapsulating, lyophilizing, or emulsifying processes, among others. Compositions can be produced in various forms, including granules, precipitates, or particulates, powders, including freeze dried, rotary dried or spray dried powders, amorphous powders, tablets, capsules, syrup, suppositories, injections, emulsions, elixirs, suspensions or solutions. Formulations can contain stabilizers, pH modifiers, surfactants, solubilizing agents, bioavailability modifiers and combinations of these.

Pharmaceutically acceptable carriers that can be used in these compositions include ion exchangers, alumina, aluminum stearate, lecithin, serum proteins, such as human serum albumin, buffer substances such as phosphates or carbonates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

These pharmaceutical compositions are formulated for pharmaceutical administration to a human being. Such compositions can be administered orally, parenterally, by inhalation spray, topically, rectally, nasally, buccally, vaginally or via an implanted reservoir. The term "parenteral" as used herein includes subcutaneous, intravenous, intraperitoneal, intramuscular, intra-articular, intra-synovial, intrasternal, intrathecal, intrahepatic, intralesional and intracranial injection

or infusion techniques. In some embodiments, the compositions are administered orally, intravenously or subcutaneously. In some embodiments, the compositions are administered orally. In some embodiments, the compositions are administered intravenously. These formulations can be designed to be short-acting, fast-releasing, or long-acting. Furthermore, the compositions can be administered in a local rather than systemic means, such as administration (e.g., by injection) at a tumor site.

Pharmaceutical formulations can be prepared as liquid suspensions or solutions using a liquid, such as an oil, water, an alcohol, and combinations of these. Solubilizing agents such as cyclodextrins can be included. Pharmaceutically suitable surfactants, suspending agents, or emulsifying agents, can be added for oral or parenteral administration. Suspensions can include oils, such as peanut oil, sesame oil, cottonseed oil, corn oil and olive oil. Suspension preparations can also contain esters of fatty acids such as ethyl oleate, isopropyl myristate, fatty acid glycerides and acetylated fatty acid glycerides. Suspension formulations can include alcohols, such as ethanol, isopropyl alcohol, hexadecyl alcohol, glycerol and propylene glycol; ethers, such as poly(ethyleneglycol); petroleum hydrocarbons such as mineral oil and petrolatum; and water.

Sterile injectable forms of these pharmaceutical compositions can be aqueous or oleaginous suspensions. These suspensions may be formulated according to techniques known in the art using suitable dispersing or wetting agents and suspending agents. The sterile injectable preparation can also be a sterile injectable solution or suspension in a non-toxic parenterally acceptable diluent or solvent, for example as a solution in 1,3-butanediol. Among the acceptable vehicles and solvents that can be employed are water, Ringer's solution and isotonic sodium chloride solution. In addition, sterile, fixed oils are conventionally employed as a solvent or suspending medium. For this purpose, any bland fixed oil can be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as sorbitan alkyl esters, such as Tweens or Spans, and other emulsifying agents or bioavailability enhancers which are commonly used in the manufacture of pharmaceutically acceptable solid, liquid, or other dosage forms can also be used for the purposes of formulation. Compounds can be formulated for parenteral administration by injection such as by bolus injection or continuous infusion. A unit dosage form for injection can be in ampoules or in multi-dose containers.

These pharmaceutical compositions can be orally administered in any orally acceptable dosage form including capsules, tablets, aqueous suspensions or solutions. When aqueous suspensions are required for oral use, the active ingredient is combined with emulsifying and suspending agents. If desired, certain sweetening, flavoring or coloring agents can also be added. For oral administration in a capsule form, useful diluents include lactose and dried cornstarch. In the case of tablets for oral use, carriers that are commonly used include lactose and corn starch. Lubricating agents, such as magnesium stearate, are also typically added. Coatings may be used for a variety of purposes, *e.g.*, to mask taste, to affect the site of dissolution or absorption, or to prolong drug action. Coatings can be applied to a tablet or to granulated particles for use in a capsule.

Alternatively, these pharmaceutical compositions can be administered in the form of suppositories for rectal administration. These can be prepared by mixing the agent with a suitable non-irritating excipient which is solid at room temperature but liquid at rectal temperature and therefore will melt in the rectum to release the drug. Such materials include cocoa butter, beeswax and polyethylene glycols.

These pharmaceutical compositions can also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract may be effected in a rectal suppository formulation (see above) or in a suitable enema formulation. Topically-transdermal patches can also be used. For topical applications, the pharmaceutical compositions can be formulated in a suitable ointment containing the active component suspended or dissolved in one or more carriers. Carriers for topical administration of the compounds of the present disclosure include mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active component(s) suspended or dissolved in one or more pharmaceutically acceptable carriers. Suitable carriers include mineral oil, sorbitan monostearate, polysorbate 60, cetyl esters wax, cetearyl alcohol, 2-octyldodecanol, benzyl alcohol and water.

For ophthalmic use, the pharmaceutical compositions can be formulated as micronized suspensions in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions can be formulated in an ointment such as petrolatum.

The pharmaceutical compositions can also be administered by nasal aerosol or inhalation. Such compositions

are prepared according to techniques well known in the art of pharmaceutical formulation and can be prepared as solutions in saline, employing benzyl alcohol or other suitable preservatives, absorption promoters to enhance bioavailability, fluorocarbons, and/or other conventional solubilizing or dispersing agents.

The methods of the present disclosure are directed to treating diseases, disorders and conditions in which inhibition of UAE enzyme activity is detrimental to survival and/or expansion of diseased cells or tissue (*e.g.*, cells are sensitive to UAE inhibition; inhibition of UAE activity disrupts disease mechanisms; reduction of UAE activity stabilizes protein which are inhibitors of disease mechanisms; reduction of UAE activity results in inhibition of proteins which are activators of disease mechanisms). The diseases, disorders and conditions are also intended to include those which require effective cullin and/or ubiquitination activity, which activity can be regulated by diminishing NAE enzyme activity.

In some embodiments, the methods of the present disclosure further comprise administering a anti-cancer agent. As used herein, the term "anticancer agent" refers to any agent that is administered to a subject with cancer for purposes of treating the cancer. The administration of the further anti-cancer agent includes administration concurrently or sequentially with the combinations of the present disclosure. Alternatively, the further anti-cancer agent can be combined into one composition with the combinations of the present disclosure which is administered to the patient.

Examples of anti-cancer agents include DNA damaging chemotherapeutic agents such as topoisomerase I inhibitors (e.g., irinotecan, topotecan, camptothecin and analogs or metabolites thereof); topoisomerase II inhibitors (e.g., etoposide, and teniposide); anthracyclines (e.g. doxorubicin, daunorubicin and idarubicin) alkylating agents (e.g., melphalan, chlorambucil, busulfan, thiotepa, ifosfamide, carmustine, lomustine, semustine, streptozocin, decarbazine, methotrexate, pemetrexed, mitomycin C, and cyclophosphamide); DNA intercalators; DNA intercalators and free radical generators such as bleomycin; and nucleoside mimetics (e.g., 5-fluorouracil, capecitibine, fludarabine, cytarabine, mercaptopurine, thioguanine, pentostatin, and hydroxyurea). Chemotherapeutic agents that disrupt cell replication include: vincristine, vinblastin, and related analogs; thalidomide, lenalidomide, and related analogs (e.g., CC-5013 and CC-4047); protein tyrosine kinase inhibitors (e.g., imatinib mesylate, erlotonib, croztinib and gefitinib); proteasome inhibitors (e.g., bortezomib); NF-κB inhibitors, including inhibitors of IκB kinase; antibodies which bind to proteins overexpressed in cancers and thereby downregulate cell replication (e.g., trastuzumab, panitumumab, rituximab, cetuximab, and bevacizumab); and other inhibitors of proteins or enzymes known to be upregulated, over-expressed or activated in cancers, the inhibition of which downregulates cell replication.

Kits

In some embodiments, one or more of the therapeutic agents described herein can be manufactured for inclusion in a kit. A "kit" is any article of manufacture (e.g., a package or container) comprising at least one reagent or chemotherapeutic agent. A kit for use in the methods herein can comprise an UAE inhibitor, such as Compound 1 or a pharmaceutically acceptable salt thereof. In some embodiments, the kit can further include a taxane. In some embodiments, the kit can further include a platin. In some embodiments, the kit can further include a taxane and a platin. In some embodiments, the kit can include Compound 1 or a pharmaceutically acceptable salt thereof and paclitaxel. In some embodiments, the kit can include Compound 1 or a pharmaceutically acceptable salt thereof and carboplatin. In some embodiments, the kit can include Compound 1 or a pharmaceutically acceptable salt thereof, carboplatin and paclitaxel.

In some embodiments, a kit comprising Compound 1 or a pharmaceutically acceptable salt thereof and another chemotherapeutic agent can further include another component or reagent. In some embodiments, a reagent in the kit can be a diluent for preparing the Compound 1 or a pharmaceutically acceptable salt thereof for administration. In some embodiments, a reagent in the kit can be a diluent for preparing the chemotherapeutic agent for administration. In some embodiments, a component in the kit can be a vessel for mixing the combination of Compound 1 and the chemotherapeutic agent. In some embodiments, the kit can include instructions for calculating the dose of each therapeutic component of the kit. In some embodiments, the instructions can include the Calvert formula.

**Methods of Synthesis**

In another aspect, the present disclosure provides a process for making Compound 1 or a pharmaceutically acceptable salt thereof. A process suitable for the large scale production of Compound 1 or a pharmaceutically acceptable salt is disclosed herein.

In some embodiments, the present disclosure provides a process for making Compound 1:

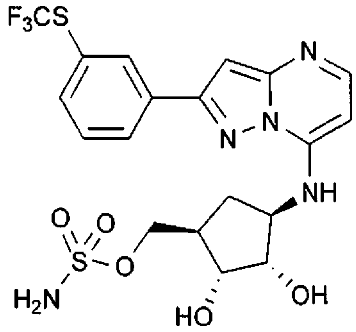

Compound 1;

or pharmaceutically acceptable salt thereof, comprising the steps of:

a) contacting Compound 9 or a salt, solvate or hydrate thereof with 2,2-dimethyl-1,3-dioxane-4,6-dione (Meldrum's acid):

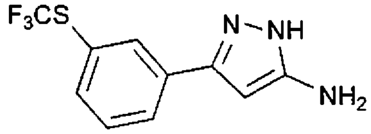

; Compound 9

under coupling conditions to provide compound 8 or a salt, solvate or hydrate thereof:

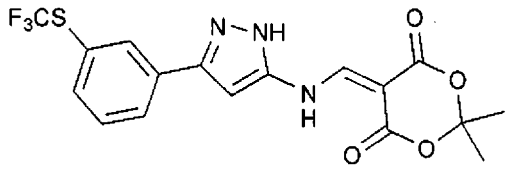

; Compound 8

b) subjecting compound 8 or a salt, solvate or hydrate thereof to cyclization conditions to provide compound 7 or a salt, solvate or hydrate thereof

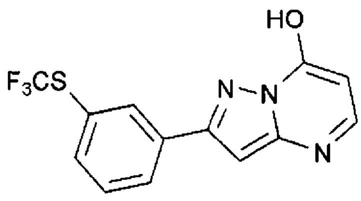

; Compound 7

c) contacting Compound 7 or a salt, solvate or hydrate thereof with benzotriazole under chlorination/displacement conditions to provide Compound 5 or a salt, complex, solvate or hydrate thereof

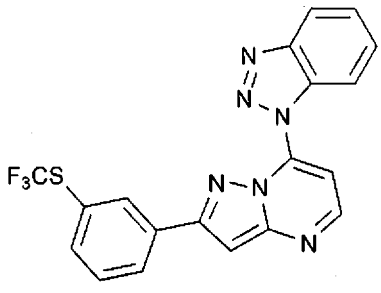

; Compound 5

d) contacting Compound 5 or a salt, complex, solvate or hydrate thereof with Compound 6 or a salt, solvate or

hydrate thereof:

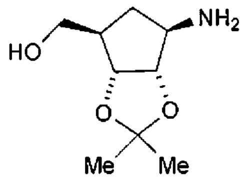

; **Compound 6**

under displacement reaction conditions to provide Compound 3 or a salt, solvate or hydrate thereof

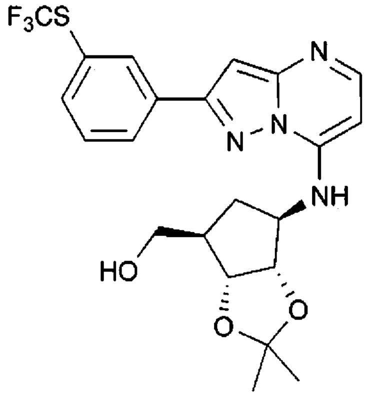

; **Compound 3**

e) contacting Compound 3 or a salt, solvate or hydrate thereof with Compound 4

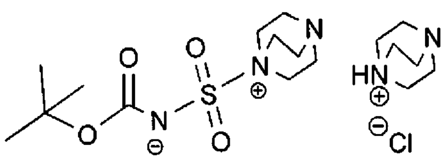

; **Compound 4**

under sulfamoylating reaction conditions to provide Compound 2 or a salt, solvate or hydrate thereof

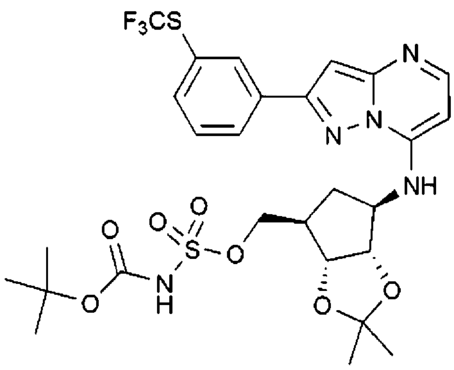

; **Compound 2**

f) contacting Compound 2 or a salt, solvate or hydrate thereof with an acid under sulfamoylation conditions to provide Compound 1 or a pharmaceutically acceptable salt thereof

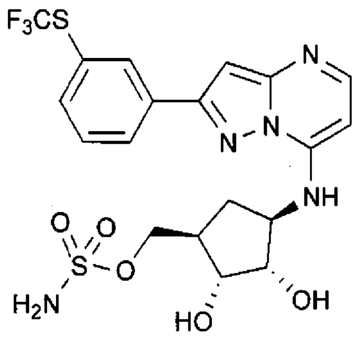

Compound 1;

g) and optionally recrystallizing Compound 1 or a pharmaceutically acceptable salt under recrystallization conditions.

In some embodiments, the present disclosure provide a process for recrystallizing Compound 1 or a pharmaceutically acceptable salt thereof to provide Compound 1 or a pharmaceutically acceptable salt using recrystallization conditions. The recrystallizing may be done to remove impurities.

In some embodiments, Compound 1 or a pharmaceutical salt thereof is Compound 1 Form 1; a anhydrous crystalline form that is described in patent applications WO2013/123169 and US 2013/0217682. In some embodiments, Compound 1 or a pharmaceutical salt thereof is Compound 1 Form 2; a monohydrated crystalline form that is described in patent applications WO2013/123169 and US 2013/0217682.

In one embodiment, the recrystallization conditions of the disclosure comprise an aqueous solvent mixture. In some embodiments, the solvent is acetonitrile, ethyl acetate, dimethylformamide, dimethylacetamide, N-methylpyrrolidone or dimethylsulfoxide. In some embodiments, the solvent is acetonitrile. In some embodiments, the amount of water in the aqueous solvent mixture is about 5% to about 70%. In some embodiments, the amount of water in the aqueous solvent mixture is about 45% to about 65%.

In some embodiments, the present disclosure provides a process for making Compound 1:

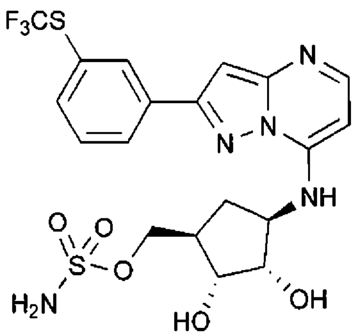

Compound 1;

or pharmaceutically acceptable salt, solvate or hydrate thereof comprising contacting compound 2 or a pharmaceutically acceptable salt thereof with an acid:

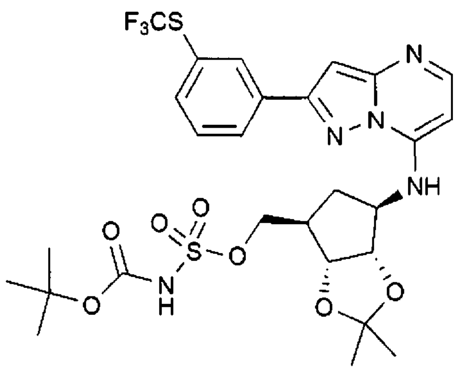

Compound 2;

under deprotection reaction conditions to provide Compound 1 or a pharmaceutically acceptable salt thereof.

In one embodiment, the deprotection reaction conditions of the disclosure comprise an aqueous acid. A variety of acids may be suitable for deprotecting the protecting groups in Compound 2. Non-limiting examples of suitable acids include phosphoric acid, sulfuric acid, trifluoracetic acid, acetic acid, and hydrochloric acid. In some embodiments, the acid is phosphoric acid. In some embodiments, the acid is sulfuric acid.

In one embodiment, the deprotection temperature is about 0 °C to about 25 °C. In other embodiments, the deprotection temperature is about 0 °C to room temperature.

In one embodiment, the deprotection reaction conditions comprise a solvent. Non-limiting examples of suitable solvents include dimethylformamide, dimethylacetamide, N-methylpyrrolidone, acetonitrile, isopropanol, ethanol, methanol, dimethylsulfoxide, water, tetrahydrofuran, 2-methyltetrahydrofuran, dichloromethane or mixtures thereof. In some embodiments, the solvent is acetonitrile.

In some embodiments, the present disclosure provides a process for making Compound 2:

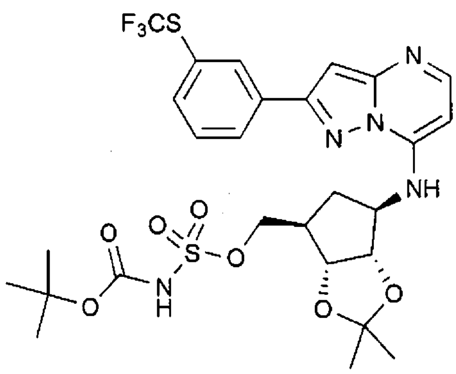

; **Compound 2**

or a salt, solvate or hydrate thereof comprising contacting Compound 3 or a salt, solvate or hydrate thereof with Compound 4:

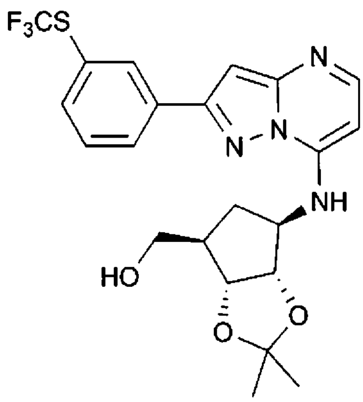

; **Compound 3**

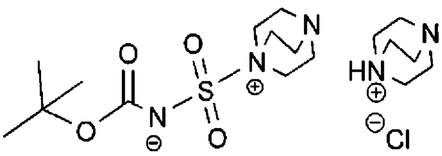

; **Compound 4**

under sulfamoylating reaction conditions to provide Compound 2 or a pharmaceutically acceptable salt thereof.

In one embodiment, the sulfamoylating reaction conditions of the disclosure comprise a weak acid or a weak base. In some embodiments, the weak acid is pyridinium p-toluenesulfonate, tosic acid, citric acid, benzoic acid, camphorsulfonic acid, nosic acid or mesic acid. In some embodiments, the weak acid is pyridinium p-toluenesulfonate. In some embodiments, the weak base is pyridine, dimethylaminopyridine, tetramethylpyridine or 2,6-lutidine.

Compound 4 can be prepared by methods such as those described in Armitage, I. et al, Org. Lett.; 2012, 14, 2626-2629, and Armitage, I. et al, US Patent Appl. Publication 2009/0036678. In some embodiments, about 1 molar equivalents to about 5 molar equivalents of the Compound 4 (with respect to Compound 3) are used. In some embodiments, about 2.5 molar equivalents to about 3 molar equivalents of Compound 4 (with respect to Compound 3) are used.

In one embodiment, the sulfamoylating reaction conditions of the disclosure comprise a solvent. Non-limiting examples of suitable solvent include dimethylformamide, dimethylacetamide, N-methylpyrrolidone, acetonitrile, tetrahydrofuran, dimethylsulfoxide, toluene, 2-methyltetrahydrofuran or mixtures thereof. In some embodiments, the solvent is acetonitrile.

In some embodiments, the present disclosure provides a process for making Compound 3:

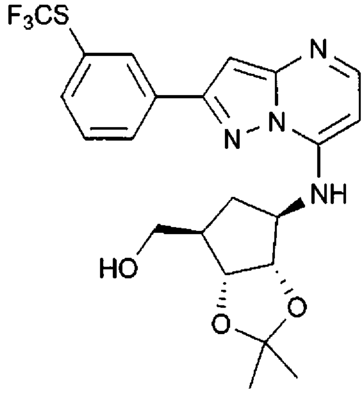

; **Compound 3**

or a salt, solvate or hydrate thereof comprising contacting Compound 5 or a salt, complex, solvate or hydrate thereof with Compound 6 or a salt, solvate or hydrate thereof:

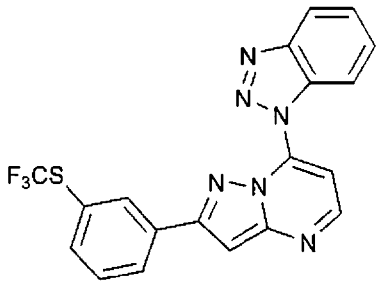

; **Compound 5**

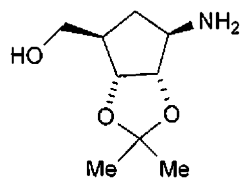

; **Compound 6**

under displacement reaction conditions.

In one embodiment, the displacement reaction conditions of the disclosure comprise a neat base. Non-limiting examples of bases that can be use neat for the displacement reaction include triethylamine, diisopropylethylamine, triispropylamine, tributylamine, pyridine, and pyrrolidine. In some embodiments, the neat base is triethylamine.

In one embodiment, the displacement reaction conditions of the disclosure comprise a base and solvent. In some embodiments, the base is triethylamine, diisopropylethylamine, triispropylamine, tributylamine, pyridine, pyrrolidine, triethylenediamine (DABCO) and diazabicycloundecene (DBU). In some embodiments, the solvent is isopropanol, ethanol, acetonitrile, dimethylsulfoxide, tetrahydrofuran, 2-methyltetrahydrofuran, dimethylformamide, N-methylpyrrolidine and toluene. In some embodiments, the base and solvent are triethylamine and isopropanol.

In one embodiment, the displacement reaction conditions of the disclosure comprise an elevated temperature. In some embodiments, the elevated temperature is about 50 °C to about 110 °C. In some embodiments, the elevated temperature is about 70 °C to about 90 °C.

In one embodiment Compound 6 is the hydrochloride salt. Compound 6 can be prepared by methods known in the scientific literature [See for example: A) Saksena, A. K. Tetrahedron Lett, 1980, 21, 133-136. B) Hutchinson, E. J.; Taylor, B. F.; Blackburn, G. M. J. Chem. Soc., Chem. Commun. 1997, 1859-1860. C) Tokoro, Y. Chem. Commun, 1999, 807-809. D) Dominguez, B. M.; Cullis, P. M. Tetrahedron Lett., 1999, 40, 5783-5786]. Compound 6 hydrochloride salt can be prepared in an analogous manner to that described in Armitage, I. et al, US Patent Appl. Publication 2009/0036678.

In another embodiment, the present disclosure provides Compound 5:

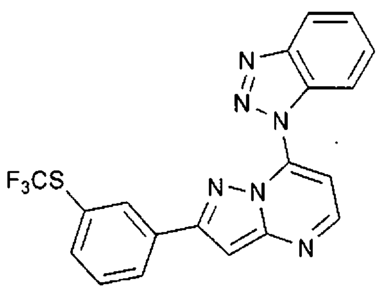

; **Compound 5**

or a salt, solvate or hydrate thereof. In one embodiment, Compound 5 is a Compound 5-hydrochloride-tertiary amine complex. In some embodiments, the tertiary amine is triethylamine. In some embodiments the amount of hydrochloride-triethylamine present in the complex is about 0% to about 200% based on the amount of Compound 5 present in the complex. In some embodiments the amount of hydrochloride-triethylamine present in the complex is about 0% to about 130% based on the amount of Compound 5 present in the complex.

In some embodiments, the present disclosure provides a process for making Compound 5:

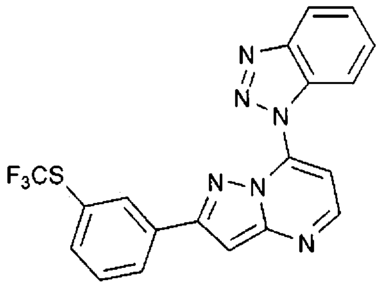

; **Compound 5**

or a salt, complex, solvate or hydrate thereof comprising contacting Compound 7 or a salt, solvate or hydrate thereof with benzotriazole:

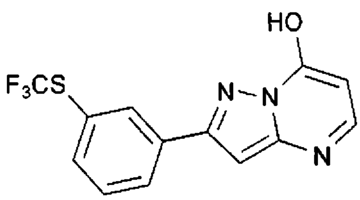

; **Compound 7**

under chlorination/displacement conditions.

In one embodiment, the chlorination/displacement reaction conditions of the disclosure comprise a chlorinating reagent. In some embodiments, the chlorinating reagent is thionyl chloride or phosphoryl chloride. In some embodiments, the chlorinating reagent is phosphoryl chloride.

In one embodiment, the chlorination/displacement reaction conditions of the disclosure comprise a solvent. In some embodiments, the solvent is acetonitrile, tetrahydrofuran, toluene or dichloroethane. In some embodiments, the solvent is acetonitrile.

In one embodiment, the chlorination/displacement reaction conditions of the disclosure comprise a base. In some embodiments, the base is triethylamine, diisopropylethylamine, triispropylamine, tributylamine, or tripropylamine. In some embodiments, the base is triethylamine.

In one embodiment, the chlorination/displacement reaction conditions of the disclosure comprise an elevated temperature. In some embodiments, the elevated temperature is about 50 °C to about 110 °C. In some embodiments, the elevated temperature is about 70 °C to about 90 °C.

In another embodiment, the present disclosure provides Compound 7:

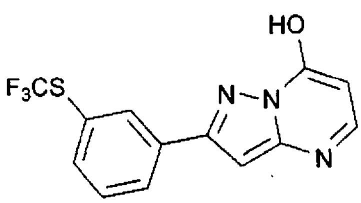

; **Compound 7**

Or a salt, solvate or hydrate thereof.

In some embodiments, the present disclosure provides a process for making Compound 7:

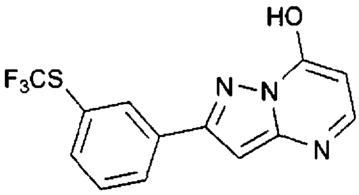

; Compound 7

or a salt, solvate or hydrate thereof comprising subjecting Compound 8 or a salt, solvate or hydrate thereof:

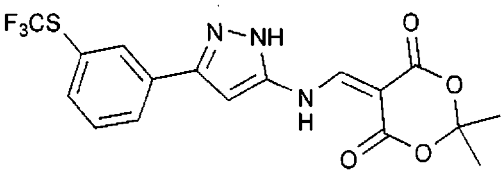

; Compound 8

to cyclization conditions.

In one embodiment, the cyclization reaction conditions of the disclosure comprise a solvent. Suitable solvents include 1,2-dichlorobenzene, chlorobenzene, nitrobenzene, quinuclidine and N-methylpyrrolidone. In some embodiments, the solvent is heat transfer fluid which is a eutectic mixture of biphenyl and diphenyl oxide (DOWTHERM A). In some embodiments, the solvent is 1,2-dichlorobenzene.

In one embodiment, the cyclization reaction conditions of the disclosure comprise an elevated temperature. In some embodiments, the elevated temperature is about 140 °C to about 180 °C. In some embodiments, the elevated temperature is about 145 °C to about 165 °C.

In one embodiment, the cyclization reaction conditions of the disclosure provide a selective cyclization that prevents formation of the alternative regio-isomer (Compound 10):

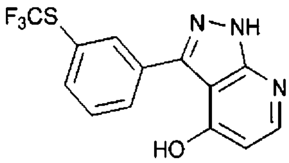

; Compound 10

In another embodiment, the present disclosure provides Compound 8:

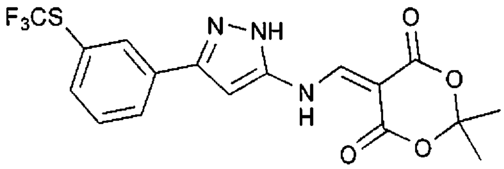

; Compound 8

Or a salt, solvate or hydrate thereof.

In some embodiments, the present disclosure provides a process for making Compound 8:

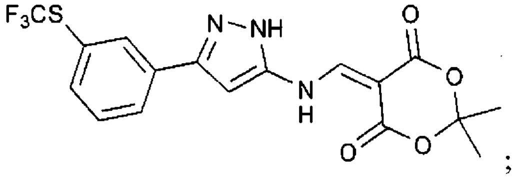

; Compound 8

or a salt, solvate or hydrate thereof comprising contacting Compound 9 or a salt, solvate or hydrate thereof with 2,2-dimethyl-1,3-dioxane-4,6-dione (Meldrum's acid):

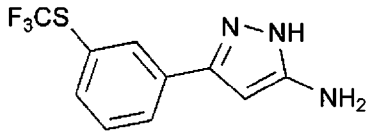

; **Compound 9**

to coupling reaction conditions.

In one embodiment, the coupling reaction conditions of the disclosure comprise a solvent. Suitable solvents include isopropanol, acetonitrile, tetrahydrofuran, 2-methyltetrahydrofuran and dichloromethane.

In one embodiment, the coupling reaction conditions of the disclosure comprise an orthoester. In some embodiments, the orthoester is trimethylorthoformate or triethylorthoformate. In some embodiments, the orthoester is trimethylorthoformate. In one embodiment, the coupling reaction conditions of the disclosure comprise dimethylformamide dimethylacetal.

In one embodiment, the coupling temperature is about room temperature to about 25 °C. In other embodiments, the coupling temperature is about 70 °C to about 90 °C.

In another embodiment, the present disclosure provides Compound 9:

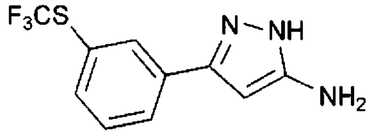

; **Compound 9**

Or a salt, solvate or hydrate thereof.

In order that this present disclosure be more fully understood, the following examples are set forth. These examples are illustrative.

## EXAMPLES (All below-mentioned examples with cisplatin or paclitaxel are considered to be reference examples.)

### Abbreviations

| | |
|---|---|
| h | hour |
| min | minutes |
| HPLC | High-pressure liquid chromatography |
| UPLC | Ultra-pressure liquid chromatography |
| NMR | Nuclear Magnetic Resonance |
| THF | tetrahydrofuran |
| TGI | Tumor Growth Inhibition |

### General Analytical Methods

Unless otherwise stated 1H NMR spectra were obtained using a Varian 300 MHz. Unless otherwise stated HPLC were obtained on Agilent 1100 Series and UPLC were obtained by Water Acuity Systems.

### Example 1: Synthesis of 3-{3-[4(trifluoromethyle)sulfanyl]phenyl}-1H-pyrazol-5-amine

#### Step A: 3-((trifluoromethyl)thio)benzoate

To dimethylcarbonate (68 mL) was added 3-((trifluoromethyl)thio)benzoic acid (100 g, Beta Pharma Scientific) and a catalytic amount of sulfuric acid (2.4 mL). The mixture was then heated to 90°C for 5h. The reaction was then

cooled to room temperature and quenched with sodium bicarbonate (1.0 L). To the aqueous layer was with ethyl acetate (1.0 L). The phases were separated and this process was repeated with ethyl acetate (1.0 L). The organic layers were combined and concentrated with a rotovap to give a light orange oil. The methyl 3-((trifluoromethyl)thio)benzoate (105g, 99%) was taken on crude to the next reaction. $^1$H NMR (300 MHz, CHLOROFORM-*d*) $\delta$ ppm 3.99 (s, 3 H) 7.49 - 7.58 (m, 1 H) 7.85 (d, *J*=7.62 Hz, 1 H) 8.17 (dt, *J*=7.69, 1.43 Hz, 1 H) 8.32 - 8.44 (m, 1 H).

**Step B: 3-oxo-3-(3-((trifluoromethyl)thio)phenyl)propanenitrile**

Methyl 3-((trifluoromethyl)thio)benzoate (100.0 g) in tetrahydrofuran (1.0 L) was added acetonitrile (44.2 mL, 847 mmol) and 1M (in THF) potassium tert-butoxide (95.01 g). The reaction was complete in 10 min by HPLC analysis. The reaction was quenched with 1M HCl (1.0 L) and then extracted with three times with (1.0 L) of ethyl acetate. The organic layers with 3-oxo-3-(3-((trifluoromethyl)thio)phenyl)propanenitrile were then concentrated to dryness. This material (100.0g, 96.3%) was taken on crude with further purification. $^1$H NMR (300 MHz, CHLOROFORM-*d*) $\delta$ ppm 4.12 (s, 2 H) 7.51 -7.75 (m, 1 H) 7.89 - 8.01 (m, 1 H) 8.01 - 8.10 (m, 1 H) 8.20 (s, 1 H)

**Step C: 3-{3-[(trifluoromethyl)sulfanyl]phenyl}-1H-pyrazol-5-amine**

To 3-oxo-3-{3-[(trifluoromethyl)sulfanyl]phenyl}propanenitrile (100.0 g,) in ethanol (1000.0 mL) was added hydrazine hydrate (59.52 mL). The reaction was heated to 100°C for 1h at which point HPLC analysis showed the reaction was complete. The reaction was concentrated to dryness on a rotovap to give a brown oil. The oil was taken up in ethyl acetate (1.0 L) and extracted with water (1.0 L). The phases were separated and the organic phase was concentrated. Upon concentration 3-{3-[(trifluoromethyl)sulfanyl]phenyl}-1H-pyrazol-5-amine was obtained (80.8 g; Yield = 76.4%) . $^1$H NMR (300 MHz, CHLOROFORM-*d*) $\delta$ ppm 5.95 (s, 1 H) 6.73 (br s, 1 H) 7.13 - 7.34 (m, 2 H) 7.42 - 7.74 (m, 3 H) 7.85 (s, 1 H).

**Example 2: ((1R,2R,3S,4R)-2,3-dihydroxy-4-((2-(3-((trifluoromethyl)thio)phenyl)pyrazolo[1,5-a]pyrimidin-7-yl)amino)cyclopentyl)methyl sulfamate**

**Step 1: (2,2-dimethyl-5-(((3-(3-((trifluoromethyl)thio)phenyl)-1H-pyrazol-5-yl)amino)methylene)-1,3-dioxane-4,6-dione)**

To trimethoxy orthoformate (2.0 L), at 20°C and under a blanket of nitrogen, was added 2,2-dimethyl-1,3-dioxane-4,6-dione (361.35 g). The resulting white suspension went clear within minutes and was heated to 85°C over 15 minutes. The reaction was held at 85°C for 120 minutes. While the reaction was heated and stirred another solution of 3-(3-((trifluoromethyl)thio)phenyl)-1H-pyrazol-5-amine (500.0 g) was made. To a 4L RBF was added 3-(3-((trifluoromethyl)thio)phenyl)-1H-pyrazol-5-amine (500.0 g) and then trimethoxy orthoformate (1.4 L) added into this solid. This solution was mixed to dissolve the solids and resulted a dark brown solution. The solution of 3-(3-((trifluoromethyl)thio)phenyl)-1H-pyrazol-5-amine (~1.8L in trimethoxy orthoformate) was added to the reactor over 30 minutes while maintaining the reaction temperature at 85°C. The reaction was then stirred for 20 minutes with white solids forming in the solution. After 20 minutes the reaction was sampled and the UPLC showed the complete conversion of 3-(3-((trifluoromethyl)thio)phenyl)-1H-pyrazol-5-amine to 2,2-dimethyl-5-(((3-(3-((trifluoromethyl)thio)phenyl)-1H-pyrazol-5-yl)amino)methylene)-1,3-dioxane-4,6-dione. The reaction was cooled to 20 °C over 20 minutes and maintained at that temperature for 20 additional minutes. At this point, a thick white slurry had formed and the reaction was filtered using a Nutche Filter over 15 minutes. The reactor was washed with 1L of ethyl acetate and this solution was then mixed with the filter cake and removed by filtration. The cake was dried for ~40 minutes on the filter and then transferred to a vacuum oven and heated at 40°C under full vacuum overnight (16 hours). The reaction was then analyzed by HPLC and NMR to give 2,2-dimethy]-5-(((3-(3-((trifluoromethyl)thio)phenyl)-1H-pyrazol-5-yl)amino)methylene)-1,3-dioxane-4,6-dione (635.3 g, 79%) $^1$H NMR (300 MHz, DMSO-$d_6$) $\delta$ ppm 1.68 (s, 6 H) 7.05 (d, *J*=2.05 Hz, 1 H) 7.64 - 7.77 (m, 2 H) 7.77 - 8.03 (m, 1 H) 8.12 (s, 1 H) 8.72 (d, *J*=14.36 Hz, 1 H) 11.35 (d, *J*=14.66 Hz, 1 H) 13.47 (s, 1 H).

**Step 2: 2-(3-((trifluoromethyl)thio)phenyl)pyrazolo[1,5-a]pyrimidin-7-ol**

A solution of 2,2-dimethyl-5-(((3-(3-((trifluoromethyl)thio)phenyl)-1H-pyrazol-5-yl)amino)methylene)-1,3-dioxane-4,6-dione (615.00 g) in 1,2-dichlorobenzene (6.3 L) was stirred at ambient temperature for 10 minutes. The solution was then heated to 150°C over 75 minutes. The reaction was maintained at this temperature for 16 hours. An sample was taken after 16 hours and the UPLC analysis showed the complete conversion of 2,2-dimethyl-5-(((3-(3-((trifluoromethyl)thio)phenyl)-1H-pyrazol-5-yl)amino)methylene)-1,3-dioxane-4,6-dione to 2-(3-((trifluoromethyl)thio)phenyl)pyrazolo[1,5-a]pyrimidin-7-ol. The reaction was cooled to 20°C over 130 minutes. At this point, a thick white slurry

had formed and the reaction was filtered using a Nutche Filter over 15 minutes. The reactor was washed with 1.8 L of acetonitrile and this solution was then mixed with the filter cake and then the solvent was removed by filtration. The cake was dried for -40 minutes on the filter and then transferred to a vacuum oven and heated at 40°C under full vacuum overnight (16 hours). The reaction was then analyzed by HPLC and NMR to give 2-(3-((trifluoromethyl)thio)phenyl)pyrazolo[1,5-a]pyrimidin-7-ol (331.2 g, 72%) $^1$H NMR (300 MHz, METHANOL-$d_4$) δ ppm 6.55 (d, *J*=7.33 Hz, 1 H) 7.59 (s, 1 H) 8.40 - 8.52 (m, 1 H) 8.53 - 8.64 (m, 1 H) 8.69 (d, *J*=7.62 Hz, 1 H) 9.01 (dt, *J*=7.77, 1.39 Hz, 1 H) 9.12 (s, 1 H) 13.34 (s, 1 H)

**Step 3: 1-(2-(3-((trifluoromethyl)thio)phenyl)pyrazolo[1,5-a]pyrimidin-7-yl)-1H-benzo[d][1,2,3]triazole: triethylamine: hydrochloride complex (1:1.25:1.25 moles:moles:moles)**

To a solution of 2-(3-((trifluoromethyl)thio)phenyl)pyrazolo[1,5-a]pyrimidin-7-ol (30.00 g), benzotriazole (287.02 g) in acetonitrile (3000 mL) and triethylamine (403.00 mL) at 0°C, was added phosphoryl chloride (108 mL) slowly under a blanket of nitrogen, maintaining < 10°C. The reaction was then warmed to 80°C over 45 minutes and stirred for 240 minutes. HPLC indicated complete consumption of starting material. To the reaction mixture was added acetonitrile (3000 mL) while maintaining the temperature at 80°C. The reaction was then cooled to 20°C over 80 minutes. The reaction was then stirred at ambient temperature for 14 hours. At this point, a thick slurry had formed and the reaction was filtered using a Nutche filter over 15 minutes. The reactor was washed twice with 900 mL of acetonitrile and this solution was then mixed with the filter cake and then the solvent was removed by filtration. The cake was dried for ~40 minutes on the filter and then transferred to a vacuum oven and heated at 40°C under full vacuum overnight (16h). The reaction was then analyzed by HPLC and NMR to give 1-(2-(3-(trifluoromethyl)thio)phenyl)pyrazolo[1,5-a]pyrimidin-7-yl)-1H-benzo[d][1,2,3]triazole: triethylamine: hydrochloride complex (1:1.25:1.25 moles:moles:moles) (438.1 g, 83%). 1H NMR (300 MHz, DMSO-$d_6$) δ ppm 1.19 (t, *J*=7.33 Hz, 12 H) 3.07 (qd, *J*=7.28, 4.84 Hz, 8 H) 7.60 - 7.78 (m, 6 H) 7.80 - 7.87 (m, 1 H) 8.15 (dt, *J*=7.99, 1.28 Hz, 1 H) 8.24 (s, 1 H) 8.33 (dt, *J*=8.14, 0.92 Hz, 1 H) 8.85 (d, *J*=4.69 Hz, 1 H).

**Step 4: ((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-yl)methanol**

To the reactor was added 1-(2-(3-((trifluoromethyl)thio)phenyl)pyrazolo[1,5-a]pyrimidin-7-yl)-1H-benzo[d][1,2,3]triazole: triethylamine: hydrochloride complex (1:1.25:1.25 moles:moles:moles) (430.0 g) and ((3aR,4R,6R,6aS)-6-amino-2,2-dimethyltetrahydro-3aH-cyclopenta[d][1,3]dioxol-4-yl)methanol hydrochloride (209.0 g) and then triethylamine (2103 mL) was added. The reaction was then heated to 80°C, under a blanket of nitrogen. After 360 minutes, HPLC analysis indicated that the reaction mixture contained <1% starting material and the reaction was cooled to 20°C over 60 minutes. To the reaction was added ethyl acetate (3.5 L) and water (3.5 L). After stirring for 10 minutes the phases were separated and the aqueous layer was back extracted with ethyl acetate (3.5 L). The organic layers were combined and concentrated to form a dark, brown oil. Acetonitrile (4.5 L) was added and the solution was concentrated to dryness to give an orange solid. The solids was transferred back to the reaction with water (4.3 L), heated to 50°C, and stirred for 20 minutes. White solids formed in this hot solution and were isolated by filtration using a Nutche Filter over 15 minutes. The solids were dried under vacuum for 15 minutes on the filter and then dissolved in acetonitrile (4.0 L) at 50°C. The solution was stirred for 15 minutes. The solution was then filtered through a fritted funnel to remove the hydrolysis solid by product and the solution was concentrated to dryness. The solids were dried in a vacuum oven at full vacuum overnight (40°C, 16 hours). The reaction was then analyzed by HPLC and NMR to give ((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-yl)methanol (349.2 g, 88%). $^1$H NMR (300 MHz, DMSO-$d_6$) δ ppm 1.25 (s, 3 H) 1.47 (s, 3 H) 1.76 - 1.90 (m, 1 H) 2.25 (br d, *J*=3.22 Hz, 1 H) 2.33 - 2.47 (m, 1 H) 3.46 - 3.67 (m, 2 H) 4.08 (br d, *J*=5.57 Hz, 1 H) 4.48 - 4.64 (m, 2 H) 5.19 (t, *J*=4.40 Hz, 1 H) 6.28 (d, *J*=5.28 Hz, 1 H) 7.06 (s, 1 H) 7.58 - 7.71 (m, 1 H) 7.72 - 7.80 (m, 1 H) 8.12 - 8.24 (m, 2 H) 8.31 (d, *J*=7.62 Hz, 1 H) 8.42 (s, 1 H).

**Step 5: ((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-yl)methyl tert-butoxycarbonylsulfamate**

((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-yl)methanol (6.0 g) was dissolved in 2-methyltetrahedrafuran (60.0 mL) and to this solution was added pyridinium p-toluenesulfonate (5.9 g). This formed a precipitated and to this white slurry was added (4-aza-1-azoniabicyclo[2.2.2]oct-1-ylsulfonyl)(tert-butoxycarbonyl)azanide-1,4-diazabicyclo[2.2.2]octane (1:1) hydrochloride[i] (17.0 g). The mixture was stirred at ambient temperature until the HPLC showed <1% ((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-yl)methanol remaining starting material (~300 minutes). The reaction was quenched with water (60 mL) and the phases were separated. To the organic layer was added acetonitrile (60 mL) and the mixture was concentrated

using a rotovap at 50°C to ~60 mL. The mixture was allowed to cool to room temperature and stirred overnight. During this time a white slurry formed. White solids were filtered using a medium fritted filter. The solid was dried in a vacuum oven at full vacuum overnight (40 °C). The reaction was then analyzed by HPLC and NMR to give ((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-yl)methyl tert-butoxycarbonylsulfamate (5.03 g, 68%). $^1$H NMR (300 MHz, DMSO-$d_6$) δ ppm 1.26 (s, 3 H) 1.42 (s, 9 H) 1.51 (s, 3 H) 2.33 - 2.48 (m, 2 H) 3.30 (br s, 1 H) 4.06 - 4.21 (m, 1 H) 4.29 (d, *J*=5.28 Hz, 2 H) 4.52 (dd, *J*=7.18, 5.13 Hz, 1 H) 4.76 (dd, *J*=7.18, 4.54 Hz, 1 H) 6.35 (d, *J*=5.57 Hz, 1 H) 7.08 (s, 1 H) 7.63 - 7.72 (m, 1 H) 7.74 - 7.82 (m, 1 H) 8.01 (d, *J*=7.92 Hz, 1 H) 8.21 (d, *J*=5.28 Hz, 1 H) 8.31 (dt, *J*=7.84, 1.36 Hz, 1 H) 8.48 (s, 1 H) 11.92 (br s, 1 H)

**Step 6: ((1R,2R,3S,4R)-2,3-dihydroxy-4-((2-(3-((trifluoromethyl)thio)phenyl)pyrazolol[1,5-a]pyrimidin-7-yl)amino)cyclopentyl)methyl sulfamate**

To a solution of ((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-yl)methyl tert-butoxycarbonylsulfamate (2.0 g) in acetonitrile (11 mL) at 0°C was added phosphoric acid (11 mL) while maintaining the temperature below 10°C. This mixture was warmed to ambient temperature and stirred for 4 hours. At this time HPLC analysis showed that <1% ((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-yl)methyl tert-butoxycarbonylsulfamate starting material or reaction intermediates remained. To the reaction was added ethyl acetate (11 mL) and water (11 mL) and saturated $Na_2CO_3$ (10 mL) dropwise. After this addition was complete saturated $Na_2CO_3$ was added until the pH was between 6-7. The phases were separated and to the organic layer was added acetonitrile (30 mL) and the mixture was concentrated on a rotovap to ~16 mL. The mixture was stirred overnight. During this time a white slurry formed. The white solids were filtered using a medium filtted filter. The solid was dried in a vacuum oven at full vacuum overnight (40°C). The reaction was then analyzed by HPLC and NMR to give ((1R,2R,3S,4R)-2,3-dihydroxy-4-((2-(3-((trifluoromethyl)thio)phenyl)pyrazolo[1,5-a]pyrimidin-7-yl)amino)cyclopentyl)methyl sulfamate (1.5g ,84%). $^1$H NMR (300 MHz, DMSO-$d_6$) δ ppm 1.44 - 1.61 (m, 1 H) 2.20 - 2.42 (m, 2 H) 3.78 (q, *J*=4.50 Hz, 1 H) 3.90 - 4.09 (m, 3 H) 4.09 - 4.22 (m, 1 H) 4.80 (d, *J*=5.28 Hz, 1 H) 5.03 (d, *J*=5.28 Hz, 1 H) 6.31 (d, *J*=5.57 Hz, 1 H) 7.05 (s, 1 H) 7.48 (s, 2 H) 7.62 - 7.72 (m, 1 H) 7.77 (d, *J*=7.92 Hz, 2 H) 8.17 (d, *J*=5.28 Hz, 1 H) 8.31 (dt, *J*=7.70, 1.43 Hz, 1 H) 8.47 (s, 1 H).

**Example 3: ((1R,2R,3S,AR)-2,3-dihydroxy-4-((2-(3-((trifluoromethyl)thio)phenyl)pyrazolo[1,5-a]pyrimidin-7-yl)amino)cyclopentyl)methyl sulfamate**

**Step 1: (2,2-dimethyl-5-(((3-(3-((trifluoromethyl)thio)phenyl)-1H-pyrazol-5-yl)amino)methylene)-1,3-dioxane-4,6-dione)**

Under a blanket of nitrogen at 20°C, Meldrum's acid (18.6 Kg) and isopropanol (33 L) were placed in a 100 L glass-lined reactor. Trimethyl orthoformate (15.5 Kg (16.0L)) and isopropanol (11 L) were added and the mixture was heated to 80 °C for 40 min, whereby a small amount of methanol distilled off (<0.5 L). The mixture was stirred for 2 h at 80 °C. In a separate 160 L glass-lined reactor under nitrogen at 20 °C, 3-(3-((trifluoromethyl)thio)phenyl)-1H-pyrazol-5-amine (prepared in the manner described above) was mixed with isopropanol (10.9 kg, 42.0 mmol) and heated up to 80 °C within 60 min. The content of the 100 L reactor was transferred into the reaction mixture in the 160 L reactor at 80 °C, which was completed after 3 min. The reaction mixture was stirred for 30 min at 78 °C, the reaction was then cooled to 60 °C. HPLC analysis showed the reaction was 99.56% complete (product%/(product%+starting material%). The reaction mixture was cooled to 20 °C within 100 min, then the mixture was stirred for further 100 min at 20 °C. The suspension was then transferred onto a pressure filter. At 1.2 bar nitrogen, the solids were collected on the filter. The filter cake was washed 4 x with ethyl acetate (18 L each time). The wet cake was dried on the filter for 17 h at 20°C using a slight stream of nitrogen/vacuum (200-100 mbar). The wet product (14.7 kg) was further dried at the rotavap for approx. 24 h at 40-50 °C. 11.75 kg of the crude title compound was obtained (68% yield). NMR spectrum was consistent with that described above in Example 2.

**Step 2: 2-(3-((trifluoromethyl)thio)phenyl)pyrazolo[1,5-a]pyrimidin-7-ol**

Under nitrogen at 20 °C, (2,2-dimethyl-5-(((3-(3-((trifluoromethyl)thio)phenyl)-1H-pyrazol-5-yl)amino)methylene)-1,3-dioxane-4,6-dione) was placed in the reactor. 1,2-Dichlorobenzene (117 L) was added. The suspension was heated to 147°C for 90 min to give a solution, then it was stirred at 147°C for 18 h. Before sampling, the reaction was cooled to 60°C. HPLC analysis showed the reaction was 92.28% completion (product%/(product%+starting material%). The mixture was heated up again to 147°C and stirred for further 5 h at this temperature. HPLC analysis showed the

reaction was 96.51% complete (product%/(product%+starting material%). The mixture was then stirred for 48 hours at 20°C, then it was heated again to 147°C und stirred at this temperature for 5 h. Before sampling, the reaction was cooled to 60°C. HPLC analysis showed the reaction was 98.47% completion (product%/(product%+starting material%). The mixture was heated up again to 146°C and stirred for further 5 h at this temperature. Before sampling, the reaction was cooled to 60°C. HPLC analysis showed the reaction was 99.35% complete (product%/(product%+starting material%). The reaction was cooled to 20°C and the suspension was transferred in a pressure filter. The solids were collected on the filter at 1.8-3 bar $N_2$ over a greater than 10 hour period. The filter cake was washed 4 x with acetonitrile (17 L), then it was dried on the filter for 18 h at 20°C/200-100 mbar, using a slight stream of $N_2$. The material was transferred to a 50 L flask and dried on a rotavap at 50-60°C / 24-14 mbar for 2 d. 6.118 kg of the crude title compound was obtained (70% yield). NMR spectrum was consistent with that described above in Example 2.

**Step 3: 1-(2-(3-((trifluoromethyl)thio)phenyl)pyrazolo[1,5-a]pyrimidin-7-yl)-1H-benzo[d][1,2,3]triazole: triethylamine: hydrochloride complex (1:0.21:0.21 moles:moles:moles)**

Under $N_2$ at 20°C, acetonitrile (30 L) was placed in the reactor, 2-(3-((trifluoromethyl)thio)phenyl)pyrazolo[1,5-a]pyrimidin-7-ol (6.00 kg) and IH-benzotriazol (5.83 kg) was added. A further portion of acetonitrile (30 L) was added, then the mixture was stirred at 20°C. Stirring proceeded over night. Triethylamine (8.16 L) was added at 20°C over 6 min. The yellow suspension was heated up to 45°C for 40 min. While stirring at 150 rpm, phosphoryl chloride (4.562 kg) was slowly added for 45 min. By controlling the addition, the reagent was dropped directly into the mixture to avoid the formation of lumps. The addition was exothermic, a maximum temperature of 53°C was observed. The brown suspension was heated up to 80°C over 1 h, then the reaction mixture was stirred for 5 h at this temperature. Acetonitrile (30 L) was added over 20 min keeping the internal temperature between 75-80°C. HPLC analysis showed the reaction was 98.31% completion (product%/(product%+starting material%).The mixture (brown suspension) was further stirred at 80°C for 70 min. HPLC analysis showed the reaction was 99.48% completion (product%/(product%+starting material%). Acetonitrile (61 L) was added over 30 min maintaining the temperature between 75-80°C. The pale brown suspension was stirred at 80°C for 90 min, then it was cooled to 20°C over 2.5 h. The mixture was stirred for 12 h at 20°C. The mixture was transferred in a pressure filter. The filter cake was washed twice with acetonitrile (18 L). Both wash steps were done at 3.5-4 bar $N_2$. Each of these filtrations took overnight to go to completion. The filter cake was dried on the filter for 7.5 h. The material was transferred in a 50 L flask and dried at the rotavap at Ta 40-50°C / 50-11 mbar for 3 d to get a dry mass of 99.88% . The yield of 1-(2-(3-((trifluoromethyl)thio)phenyl)pyrazolo[1,5-a]pyrimidin-7-yl)-1H-benzo[d][1,2,3]triazole: triethylamine: hydrochloride complex (1:0.21:0.21 moles:moles:moles) was 7.948 kg (75%). NMR spectrum was consistent with that described above in Example 2.

**Step 4: ((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-y)methanol**

Under $N_2$ in a 160 L glasslined reactor, triethylamine (21%) compound with 1-(2-(3-((trifluoromethyl)thio)phenyl)pyrazolo[1,5-a]pyrimidin-7-yl)-1H-benzo[d][1,2,3]triazole (21%) hydrochloride (7.86 kg) was dissolved in triethylamine (23.3 L) at 20°C. ((3aR,4R,6R,6aS)-6-amino-2,2-dimethyltetrahydro-3aH-cyclopenta[d][1,3]dioxol-4-yl)methanol hydrochloride (4.49 kg) was added, followed by triethylamine (23 L). The reaction mixture was heated up to 80°C over 1 h, and then the mixture was stirred for 8 h at 80°C. The mixture was then cooled to 20°C. HPLC analysis showed the reaction was 99.97% complete (product%/(product%+starting material%). Water (66 L) was then added over 30 min at 20-25°C (exotherm), whereby a brown suspension was obtained. The mixture was concentrated at 60°C, 150-95 mbar, until 42 L solvent was distilled off. The suspension was heated to 50°C, and the solids were collected on a 90 L pressure filter (1.2 bar $N_2$), which took 40 min. During this process, the material on the filter was not actively heated. The remaining solids in the reactor were rinsed with 15 L of the mother liquor. The wet filter cake was transferred back in the reactor. Water (64 L) was added. The mixture was heated up to 50°C over 30 min. The washed solids were collected on the 90 L pressure filter. Remaining mother liquor in the filter cake was pressed off at 1.2 bar $N_2$ for 50 min (50 L mother liquor was used to rinse the reactor). The filter cake was dried on the pressure filter for 13.5 h, applying a slight stream of $N_2$ / vac at 20°C to afford 10.247 kg of crude ((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-yl)methanol. The wet filter cake was isolated. The wet filter cake was loaded into the reactor. Acetonitrile (65 L) was added, followed by activated charcoal (6.59 kg). The mixture was heated to 50°C for 30 min and stirred for 2 h at 50°C. Meanwhile a bed of celite (4.25 kg) had been prepared in the 90 L pressure filter, using acetonitrile (20 L) for conditioning. The bed was heated at 50°C. The black suspension was transferred on the filter and pushed through the Celite plug at 2 bar. The filtrate was transferred to a 200 L stirring tank via a heat resistant tube and a 0.45 $\mu$m inline filter. The operation needed 18 min for completion. For washing, acetonitrile (50 L) which had been warmed up in the reactor to 50°C and transferred over the warmed filter cake and pushed through at 2 bar. Again, the filtrate was transferred in the 200 L stirring tank via a heat resistant tube

and a 0.45 μm inline filter. The operation needed 10 min for completion. The reactor was cleaned to remove attached charcoal (abrasive cleaning, using NaCl /acetone). The filtrate in the stirring tank was transferred in the reactor and concentrated at 50°C / 120 mbar until 63 L were distilled off. While well stirring (300 rpm) and 50°C, Water (110 L) was slowly added over 2 h. A pale yellow suspension was formed. The concentrate was cooled to 20°C for 3 h, then stirred at this temperature for 13 h. The solids were collected on a 50 L filter, using 1.2 bar $N_2$ to push the filtrate through. The filter cake was washed twice with water (18 L), then dried on the filter for 24 h at 200-100 mbar, using a slight stream of $N_2$. 4.563 kg of the title compound was obtained 55% yield. NMR spectrum was consistent with that described above in Example 2.

**Step 5: ((3aR.4R,6R.6aS)-2,2-dimethyl-6-((2-(3-((trifluoromethyl)thio)phenyl)pyrazolo]1,5-a]pyrimidin-7-yl)amino)tetrahydro-3aH-cyclopenta[d][1,3]dioxol-4-yl)methy tert-butoxycarbonylsulfamate**

Under $N_2$ at 20°C, ((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-yl)methanol (4.019 kg) was placed in a 160 L glasslined reactor, then 2-methyl-tetrahydrofuran (40 L) was added. The mixture was stirred at 150 rpm for 30 min at 20°C, whereby a clear solution was formed. A KF measurement was taken and showed the water content to be 0.036% $H_2O$. The solution was stirred over night at 20 °C. The next morning, PPTS (2.2 kg) was loaded into the reactor. At 20°C, (4-aza-1-azoniabicyclo[2.2.2]oct-1-ylsulfonyl)(tert-butoxycarbonyl)azanide-1,4-diazabicyclo[2.2.2]octane (1:1) hydrochloride (10.2 kg) was added. Stirring of the heterogeneous mixture was started at 130 rpm. The reaction was stirred with 200 rpm for 1 h at 20°C, then with increased speed of 250 rpm for an additional hour. HPLC analysis showed the conversion to be 87.3%. The reaction mass was stirred with 300 rpm for 2 h at 20°C. HPLC analysis showed the conversion to be 95.6%. The reaction mass was stirred with 300 rpm for 2 h at 20°C. HPLC analysis showed the conversion to be 97.7%. $NaHCO_3$ 3.7% (40 L) was added to the mixture at 20°C and the reaction was stirred at 300 rpm for 10 min. Most of the solids from the reaction mixture went into solution. To dissolve remaining material which was attached at the top of the reactor, the bilayered mixture was stir up shortly by a $N_2$ stream from the bottom. The layers were separated, which was completed after 13 min. The aqueous layer was discharged, the organic layer remained in the reactor. The org. layer was a brown solution, the aqueous layer was colorless and turbid. The pH of aqueous layer was approx. 8 (pH stick). $NaHCO_3$ 3.7% (40 L) was added to the mixture at 20°C and it was stirred at 300 rpm for 10 min. The layers were separated, which was completed after 27 min. The aqueous layer was discharged, the organic layer remained in the reactor. The organic layer was a brown solution, the aqueous layer was colorless and turbid. The pH of aqueous layer was approx. 8-9 (pH stick) and the pH of organic layer was approx. 8 (pH stick, wet). The product in organic layer was transferred in the feeding tank and stored temporarily (approx. 30 min) at 20°C. The reactor was optically cleaned using a mixture of 2-methyltetrahydrofuran (30 L) and $H_2O$ (20 L). The org. layer was placed in the reactor and stored at -20°C for 14.5 h . While stirring at 150 rpm, the org. layer (suspension) was diluted with acetonitrile (16 L) and water (15 L) and warmed up to 5°C. At 5°C, acetic acid (0.172 kg) was added over 5 min. to a pH of 6; resulting in a mixture that was a pale brown solution. ((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-yl)methyl tert-butoxycarbonylsulfamate (2.0 g; prepared in a similar manner to that described above Example 2, Step 5) was added as seed. At 5°C, acetic acid (0.515 kg) was added over 15 min. to pH 4-5; a suspension formed. The feeding tank was rinsed with water (1.6 L). The mixture was stirred at 5°C with 90 rpm for 1.5 h, then it was transferred in a 50 L filter and filtered at 1.2 bar $N_2$, in only 4 min. The filter cake was washed 4 x with cold acetonitrile (8 L, 0-5°C), then it was dried on the filter at 20°C for 8 h at 200 mbar, using a slight stream of $N_2$. The yield of the title compound was 3.594 kg (62%). NMR spectrum was consistent with that described above in Example 2.

**Step 6: ((1R,2R,3S,4R)-2,3-dihydroxy-4-((2-(3-((trifluoromethyl)thio)phenyl)pyrazolo[1,5-a]pyrimidin-7-yl)amino)cyclopentyl)methyl) sulfamate Compound 1**

3.538 kg of ((3aR,4R,6R,6aS)-2,2-dimethyl-6-((2-(3-((trifluoromethyl)thio)phenyl)pyrazolo[1,5-a]pyrimidin-7-yl)amino)tetrahydro-3aH-cyclopenta[d][1,3]dioxol-4-yl)methyl tert-butoxycarbonylsulfamate was suspended in 13.5 kg of acetonitrile and cooled to 5°C. To this mixture was added 27.3 kg of $H_3PO_4$ over 1 hour and 50 minutes. The reaction was warmed to 20°C over 50 minutes and then stirred for 8h at 22°C. HPLC analysis showed the reaction was 99.69% complete. To the first portion (50% of the reaction mixture) was added 8.9 kg of water and 7.95 kg of ethyl acetate. The pH was then adjusted to 6.5 with 48 L of saturated sodium carbonate. 7.7 kg of ethyl acetate was added and the phases were separated. To the second portion (50% of the reaction mixture) was added 8.9 kg of water and 7.95 kg of ethyl acetate. The pH was then adjusted to 6.15 with 48 L of saturated sodium carbonate. 7.7 kg of ethyl acetate was added and the phases were separated. The organic phases were combined in a vessel (rinsed with 1.8 kg of ethyl acetate) and washed with 17.8 kg of water. The phases were separated and 17.8 kg of water and 0.237 kg of NaCl were added and the phases were separated. A repeat of wash with 17.8 kg of water and 0.237 kg of NaCl was added and the phases

were separated. The organic layers were then combined and the temperature of the mixture was raised to 40°C and the pressure was reduced to 300-142 mbar. 27 L of liquid was distilled off over 4h. 31.7 kg of acetonitrile were then added to the solution and the temperature of the mixture was raised to 38°C and the pressure was reduced to 320-153 mbar. 26 L of liquid was distilled over 3h. 31.7 kg of acetonitrile were then added to the solution and the temperature of the mixture was raised to 37°C and the pressure was reduced to 320-153 mbar. 34 L of liquid was distilled over 2h. The suspension was stirred for 1h at 50°C and then cooled to 20-25°C over 3h. The reaction was stirred overnight and the product was filtered and washed with 8.9 kg of acetonitrile twice. The cake was dried for 2h at 20°C (33 mbar) then at 40-45°C (1 mbar) to afford 2.08 kg (75.8%) of the title compound. 2.066 kg of ((1R,2R,3S,4R)-2,3-dihydroxy-4-((2-(3-((trifluoromethyl)thio)phenyl)pyrazolo[1,5-a]pyrimidin-7-yl)amino)cyclopentyl)methyl sulfamate was loaded into a reactor with 9.76 kg of acetronitrile and 4.12 kg of water and heated at a temperature of 56°C for 1 hour and 10 minutes until dissolved. The solution was polished filtered and the filter was rinsed with 3.16 kg acetonitrile and 1.37 kg of water. To the resulting solution was added with 11.0 kg of water over 45 minutes while maintaining the reaction temperature between 52-55°C. 0.009 kg of ((1R,2R,3S,4R)-2,3-dihydroxy-4-((2-(3-((trifluoromethyl)thio)phenyl)pyrazolo[1,5-a]pyrimidin-7-yl)amino)cyclopentyl)methyl sulfamate was added as seed (prepared in a similar manner to that described above Example 2, Step 5). A suspension was visible after 10 minutes of stirring. To the solution was added 9.62 kg of water over 3h while maintaining the reaction temperature between 50-55°C. The suspension was then cooled over 3h to 20°C and stirred for 12h at 22-23°C. The suspension was then filtered and washed twice with 13.7 kg of water. The product was dried at 40°C. 1.605 kg of the title compound was obtained in 78% yield. NMR spectrum was consistent with that described above in Example 2.

**Example 4: In vivo tumor efficacy models**

**Xenograft models**

The PHTX-132Lu non-small cell lung xenograft model was established from a patient-derived tumor collected during surgery from a 56 year female with lung cancer (Poorly Differentiated Adenocarcinoma). PHTX-132Lu tumor fragments (approximately 2x2x3 $mm^3$) are implanted into the subcutaneous space in the right dorsal flank of female CB-17 SCID mice (age 10 weeks, Charles River Laboratory, Wilmington, MA) using a 13-gauge trocar. When the mean tumor volume reached approximately 220 $mm^3$, the animals were randomized into 12 treatment groups (n=5/group).

SKOV-3 is an ovarian cell-line derived xenograft. Ten week old female nude mice (Taconic Farms, Inc) were inoculated subcutaneously in flank (cell suspension) with 4.0 x $10^6$ SKOV-3 cells suspended in serum free DMEM + Matrigel. When the mean tumor volume reached approximately 200 $mm^3$, the animals were randomized into 12 treatment groups (n=5/group).

The PHTX-02B breast xenograft model was established from a patient-derived tumor collected during surgery from a 51 year female with invasive ductal carcinoma classified as triple negative breast cancer (ER-/PR-/Her2-) by IHC. PHTX-02B tumor fragments (approximately 2x2x3 $mm^3$) are implanted into the subcutaneous space in the right dorsal flank of female SCID-NOD mice (age 10 weeks, Jackson Laboratory, Bar Harbor, ME) using a 13-gauge trocar. In the study of Compound 1 and docetaxel, when the mean tumor volume reached approximately 220 $mm^3$, the animals were randomized into 11 treatment groups (n=5/group). In the study of Compound 1 and paclitaxel, when the mean tumor volume reached approximately 250 $mm^3$, the animals were randomized into 6 treatment groups (n=5/group).

Calu-6 is a non-small cell lung cancer cell-lined derived xenograft model. Nine week old female nude mice (Taconic Farms, Inc) were inoculated subcutaneously in the flank (cell suspension) with 4.0 x $10^6$ Calu-6 cells suspended in serum free MEM. When the mean tumor volume reached approximately 150 $mm^3$, the animals were randomized into 12 treatment groups (n=6/group).

The PHTX-24C colon xenograft model was established from a patient-derived tumor collected during surgery from a 89 year female with poorly differentiate adenocarcinoma. PHTX-24C tumor fragments (approximately 2x2x3 $mm^3$) are implanted into the subcutaneous space in the right dorsal flank of female CB-17 SCID (age 9 weeks, Charles River lab, Wilmington, MA) using a 13-gauge trocar. When the mean tumor volume reached approximately 200 $mm^3$, the animals were randomized into 7 treatment groups (n=5/group).

The CTG-0256 model of human ovarian xenograft model was established from a patient-derived tumor collected during surgery at primary site from a 77 year female with ovarian cancer (serous adenocarcinoma with resistant response to carboplatin or paclitaxel). CTG-0256 tumor fragments (approximately 2x2x3 $mm^3$) were implanted into the subcutaneous space in the right dorsal flank of female *Mus musculus*/nu/nu mice (age 6-9 weeks, Harlan Laboratories, Madison, WI) using a 13-gauge trocar. When the mean tumor volume reached approximately 150 $mm^3$, the animals were randomized into 7 treatment groups (n=7/group).

The CTG-0486 model of human ovarian xenograft model was established from a patient-derived tumor collected during surgery from a 77 year female with ovarian cancer (serous adenocarcinoma, metastases to the small bowel). CTG-0486 tumor fragments (approximately 2x2x3 $mm^3$) are implanted into the subcutaneous space in the right dorsal

flank of female Mus musculus/nu/nu mice (age 6-9 weeks, Harlan Laboratories, Madison, WI, USA) using a 13-gauge trocar needle. When the mean tumor volume reached approximately 260 mm3, the animals were randomized into 8 treatment groups (n=5/group).

The PHTX-17C colorectal carcinoma xenograft model was established from a patient-derived tumor collected during surgery from a 71 year female with moderately differentiated adenocarcinoma.PHTX-17C tumor fragments (approximately 2x2x3 $mm^3$) were implanted into the subcutaneous space in the right dorsal flank of female nude mice (age 5-7 weeks, Balb/c Nude Mice, Shanghai SINO-British SIPPR/BK Lab Animal Ltd, Shanghai, China) using a 13-gauge trocar. When the mean tumor volume reached approximately 185 $mm^3$, the animals were randomized into 12 treatment groups (n=5/group).

The CTG-0165 human lung NSCLC xenograft model was established from a patient-derived tumor collected during lymph node biopsy from a 71 year female with NSCLC cancer (poorly differentiated adenocarcinoma with poor response to carboplatin + taxotere). CTG-0165 tumor fragments (approximately 2x2x3 $mm^3$) were implanted into the subcutaneous space in the right dorsal flank of female *Mus musculus*/nu/nu mice (age 6-9 weeks, Harlan Laboratories, Madison, WI, USA) using a 13-gauge trocar. When the mean tumor volume reached approximately 260 $mm^3$, the animals were randomized into 8 treatment groups (n=5/group).

The PHTX-14B breast xenograft model was established from a patient-derived tumor collected during surgery from a 43 year female with invasive ductal adenocarcinoma classified as triple negative breast cancer (ER-/PR-/Her2-) by IHC. In the study of Compound 1 and docetaxel, PHTX-14B tumor fragments (approximately 2x2x3 $mm^3$) are implanted into the subcutaneous space in the right dorsal flank of female nude mice (age 9 weeks, Taconic Farms, Inc, 273 Hover Ave Germantown NY) using a 13-gauge trocar. When the mean tumor volume reached approximately 180 $mm^3$, the animals were randomized into 9 treatment groups (n=5/group). In the study of Compound 1 and paclitaxel, PHTX-14B tumor fragments (approximately 2x2x3 $mm^3$) are implanted into the subcutaneous space in the right dorsal flank of female nude mice (age 5-7 weeks, Balb/c Nude Mice, Shanghai SINO-British SIPPR/BK Lab Animal Ltd, Shanghai, China) using a 13-gauge trocar needle. When the mean tumor volume reached approximately 150 $mm^3$, the animals were randomized into 10 treatment groups (n=5/group).

The CTG-0159 human lung NSCLC xenograft model was established from a patient-derived tumor collected during surgery from a 65 year old female with NSCLC cancer. CTG-0159 tumor fragments (approximately 2x2x3 $mm^3$) were implanted into the subcutaneous space in the left flank of female *Mus musculus*/nu/nu mice (age 6-9 weeks, Harlan Laboratories, Madison, WI, USA) using a 13-gauge trocar. When the mean tumor volume reached approximately 195 $mm^3$, the animals were randomized into 7 treatment groups (n=7/group).

The CTG-0012 model of human primary triple negative breast cancer model was established from a patient-derived tumor collected during surgery from a 36 year female with triple negative breast cancer (ER-/PR-/Her2-) by IHC. CTG-0012 tumor fragments (approximately 2x2x3 mm3) are implanted into the subcutaneous space in the right dorsal flank of female Mus musculus/nu/nu mice (age 6-9 weeks, Harlan Laboratories, Madison, WI, USA) using a 13-gauge trocar needle. When the mean tumor volume reached approximately 180 $mm^3$, the animals were randomized into 7 treatment groups (n=7/group).

**Test agents:**

Docetaxel, paclitaxel, oxaliplatin, cisplatin and carboplatin were clinical grade purchased from commercial sources and are administered as outlined below.

Docetaxel (Henry Schein, Inc., Pittsburgh PA) is formulated in 0.9% saline and administered by intravenous injection (IV) once weekly (QW) at 5 mg/kg or 10 mg/kg. The once weekly schedule is also described as every 7 days (Q7D).

Paclitaxel (Bristol-Myers Squibb Company) is formulated in 0.9% saline and administered by intravenous injection (IV) on every 4 days (Q4D) or once weekly (QW) at 10 and 20 mg/kg. The once weekly schedule is also described as every 7 days (Q7D).

Oxaliplatin (Jiang Su Geng Rui Pharmaceuticals, Inc.) is formulated in 0.9% saline and administered by IP injection once weekly (QW) at 7.5 or 12.5 mg/kg. The once weekly schedule is also described as every 7 days (Q7D).

Cisplatin (Teva Pharmaceuticals) is formulated in 0.9% saline and administered by IV injection once weekly (QW) at 7.5 mg/kg. The once weekly schedule is also described as every 7 days (Q7D).

Carboplatin (Teva Pharmaceuticals) is formulated in 0.9% saline and administered by IP injection once weekly (QW) at 25, 50, 60, and 75 mg/kg. The once weekly schedule is also described as every 7 days (Q7D).

Compound 1 [((1R,2R,3S,4R)-2,3-dihydroxy-4-(2-(3-(trifluoromethylthio)-phenyl)pyrazolo[1,5-a]pyrimidin-7-ylamino)cyclopentyl)methyl sulfamate] is formulated in 10-20% HPbCD in sterile water and administered intravenously (IV) or subcutaneously (SC) on a BIW (twice/week schedule; Monday and Thursday or Tuesday and Friday) for either 2 - 4 weeks.

**Tumor measurements:**

Tumors were measured twice weekly using vernier calipers. Tumor volumes were calculated using the standard formula V = $W^2$ x L /2.). When mean tumor volumes reached approximately 200 $mm^3$ for PHTX-24C, 220 $mm^3$ for PHTX-Lu132, 150 $mm^3$ for CTG-0256, 260 $mm^3$ for CTG-0486, 185 $mm^3$ for PHTX-17C, 260 $mm^3$ for CTG-0165, 150 $mm^3$ for PHTX-14B in the study of Compound 1 and paclitaxel, 180 $mm^3$ for PHTX-14B in the study of Compound 1 and docetaxel, 200 $mm^3$ for SKOV-3, 220 $mm^3$ for PHTX-02B in the study of Compound 1 and docetaxel, 250 $mm^3$ for PHTX-02B in the study of Compound 1 and paclitaxel, 195 $mm^3$ for CTG-0159, 180 $mm^3$ for CTG-0012 or 150 $mm^3$ for Calu-6, mice were randomized into groups of n=5-7/arm as described in the tables below, and injected with vehicle, Compound 1 or agents (docetaxel, paclitaxel, oxaliplatin, cisplatin or carboplatin), or the combination of Compound 1 plus one of the agents, at various doses and schedules as described in tables below. Tumor size and body weight were measured approximately twice a week for the duration of the study. Mice are euthanized when their tumor volume reached 10% of their body weight, or when the average tumor volume of a treatment or control group reached approximately 2000 $mm^3$. Tumor growth continued to be monitored after the dosing period in some studies. Tumor volumes during study days 14-28 are shown in tables below. Average tumor volume is reported as a function of time for selected arms of selected studies in the Figures.

**Statistical analyses of combination effect for tumor growth in subcutaneous xenograft models**

Measurements from day 0 to day 14, day 19, day 21, or day 28 are analyzed as specified in Tables below. All tumor volumes have a value of 1 added to them before $\log_{10}$ transformation. For each animal, the log tumor volume at day 0 is subtracted from the log tumor volume on the subsequent days. This difference vs. time is used to calculate an area under the curve (AUC) for each animal using the trapezoid rule. In instances when an animal in a treatment group is removed early from the study, the last observed tumor value is carried forward through all subsequent time points. The synergy score for the combination of agents A and B is defined as

$$100 * (\text{mean}(AUC_{AB}) - \text{mean}(AUC_A) - \text{mean}(AUC_B) + \text{mean}(AUC_{ctl})) / \text{mean}(AUC_{ctl}):$$

where $AUC_{AB}$, $AUC_A$, $AUC_B$, and $AUC_{ctl}$ are the AUC values for animals in the combination group, the A group, the B group, and the control group, respectively. The standard error of the synergy score is computed based on the variation in the AUC values among the animals. A two sided t-test is used to determine if the synergy score is significantly different from zero. If the P-value is above 0.05, then the combination is considered to be additive. If the P-value is below 0.05, and the synergy score is less than zero, then the combination is considered to be synergistic. If the P-value is below 0.05 and the synergy score was greater than zero, but the mean AUC for the combination was lower than the lowest mean AUC among the two single agent treatments, then the combination was sub-additive. If the P-value is below 0.05 and if the synergy score was greater than zero, and the mean AUC for the combination was greater than the mean AUC for at least one of the single agent treatments, then the combination was antagonistic.

**Results**

Mouse xenograft models, performed as described in the general methods above, were used to assess the combination effect in vivo of Compound 1 and docetaxel, Compound 1 and paclitaxel, Compound 1 and oxaliplatin, Compound 1 and cisplatin, and Compound 1 and carboplatin. The details for each study are as shown below in tables below. The results were analyzed using the statistical analysis described above and the classification of the combination is shown tables below.

**Compound 1 and carboplatin**

**PHTX-132Lu xenograft model**

In the PHTX-132Lu model dosing of the single agents Compound 1 (IV at 5, 10 and 15 mg/kg BIW) and carboplatin (25 mg/kg IP Q7D) yielded modest anti-tumor activity compared to vehicle control. Combining Compound 1 with 25 mg/kg of carboplatin yielded an additive and antagonistic combination effect. 75 mg/kg of carboplatin (Q7Dx3) administered as either a single agent or in combination with Compound 1 (IV at 5, 10 and 15 mg/kg BIW) was not tolerated (study groups 2, 7, 8 and 9; these groups are not shown in Table 1a). All tolerated study groups from the study are shown in **Table 1a.** The combination effect for this combination is shown in **Table 1b.** Mean tumor growth curves are shown in **Figure 1.** The error bars shown in Figure 1 indicate the standard error of the mean (SEM).

**Table 1a: Combination of carboplatin and Compound 1 in the PHTX-132Lu xenograft model**

| Study Group | Treatment | Dosing Regimen | Route | Tumor Volume Day 21 | SEM Tumor Volume Day 21 | Number of mice in group (number on day 21) |
|---|---|---|---|---|---|---|
| 1 | 20% HPbCD, 0.9% saline | BIWx3, Q7Dx3 | IV, IP | 988 | 153.4 | 5 |
| 3 | 25 mg/kg carboplatin | Q7Dx3 | IP | 727 | 42.8 | 5 |
| 4 | 15 mg/kg Compound 1 | BIWx3 | IV | 483.7 | 80.4 | 5 |
| 5 | 10 mg/kg Compound 1 | BIWx3 | IV | 653.5 | 210 | 5 |
| 6 | 5 mg/kg Compound 1 | BIWx3 | IV | 882.7 | 131 | 5 |
| 10 | 15 mg/kg Compound 1, 25 mg/kg carboplatin | BIWx3, Q7Dx3 | IV, IP | 455.1 | 109 | 4 |
| 11 | 10 mg/kg Compound 1, 25 mg/kg carboplatin | BIWx3, Q7Dx3 | IV, IP | 823.3 | 345.9 | 5 |
| 12 | 5 mg/kg Compound 1, 25 mg/kg carboplatin | BIWx3, Q7Dx3 | IV, IP | 773.5 | 302.8 | 5 |

**Table 1b: Classification for in vivo combination of carboplatin and Compound 1 in the PHTX-132Lu xenograft model**

| Treatment | Synergy score | SEM | P-value | Classification |
|---|---|---|---|---|
| 5 mg/kg Compound 1 + 25 mg/kg carboplatin | 29.6 | 14.9 | 0.082 | Additive |
| 10 mg/kg Compound 1+ 25 mg/kg carboplatin | 46.9 | 13.7. | 0.007 | Antagonistic |
| 15 mg/kg Compound 1 + 25 mg/kg carboplatin | 29.8 | 17.5 | 0.118 | Additive |

**SKOV-3 xenograft model**

In the SKOV-3 model, dosing of the single agents Compound 1 (IV at 5, 10 and 15 mk/kg BIW) and carboplatin (25 and 75 mg/kg IP Q7D) yielded modest anti-tumor activity compared to vehicle control. The combination arms of Compound 1 with carboplatin yielded additive to synergistic anti-tumor effects. All treatment groups from the study are shown in **Table 2a.** Day 14 was the last day of the study. The combination benefit for this combination is shown in **Table 2b.** Mean tumor growth curves of arms 1, 2, 4 and 7 are shown in **Figure 2a.** Mean tumor growth curves of all arms are shown in **Figure 2b.** The error bars shown on the Figure 1 indicate the standard error measurements (SEM).

Table 2a: Combination of carboplatin and Compound 1 in the SKOV-3 xenograft model

| Study Group | Treatment | Dosing Regimen | Route | Tumor Volume Day 14 | SEM Tumor Volume Day 14 | Number of mice in group (number on day 14) |
|---|---|---|---|---|---|---|
| 1 | 20% HPbCD, 0.9% saline | BIWx3, Q7Dx2 | IV, IP | 974.4 | 461.9 | 5 |
| 2 | 75 mg/kg carboplatin | Q7Dx2 | IP | 798.5 | 441.2 | 5 |
| 3 | 25 mg/kg carboplatin | Q7Dx2 | IP | 932.8 | 435.2 | 5 |
| 4 | 15 mg/kg Compound 1 | BIWx2 | IV | 919.2 | 521 | 5 |
| 5 | 10 mg/kg Compound 1 | BIWx2 | IV | 883 | 529 | 5 |
| 6 | 5 mg/kg Compound 1 | BIWx2 | IV | 857.2 | 297.6 | 5 |
| 7 | 15 mg/kg Compound 1, 75 mg/kg carboplatin | BIWx2, Q7Dx2 | IV, IP | 219.8 | 63.9 | 5 |
| 8 | 10 mg/kg Compound 1, 75 mg/kg carboplatin | BIWx2, Q7Dx2 | IV, IP | 361.9 | 242.4 | 5 |
| 9 | 5 mg/kg Compound 1, 75 mg/kg carboplatin | BIWx2, Q7Dx2 | IV, IP | 457.8 | 127.7 | 5 |
| 10 | 15 mg/kg Compound 1, 25 mg/kg carboplatin | BIWx2, Q7Dx2 | IV, IP | 518.2 | 204.1 | 5 |
| 11 | 10 mg/kg Compound 1, 25 mg/kg carboplatin | BIWx2, Q7Dx2 | IV, IP | 732 | 318.6 | 5 |
| 12 | 5 mg/kg Compound 1, 25 mg/kg carboplatin | BIWx2, Q7Dx2 | IV, IP | 731.3 | 323.8 | 5 |

Table 2b: Classification for in vivo combination of carboplatin and Compound 1 in the SKOV-3 xenograft model

| Treatment | Synergy score | SEM | P-value | Classification |
|---|---|---|---|---|
| 5 mg/kg Compound 1 + 25 mg/kg carboplatin | -17 | 16 | 0.307 | Additive |
| 10 mg/kg Compound 1 + 25 mg/kg carboplatin | -20 | 18 | 0.0.279 | Additive |
| 15 mg/kg Compound 1 + 25 mg/kg carboplatin | -37 | 19 | 0.079 | Additive |
| 5 mg/kg Compound 1 + 75 mg/kg carboplatin | -30 | 14 | 0.044 | Synergy |
| 10 mg/kg Compound 1 + 75 mg/kg carboplatin | -69 | 24 | 0.02 | Synergy |
| 15 mg/kg Compound 1 + 75 mg/kg carboplatin | -78 | 19 | 0.001 | Synergy |

## CTG-0256 xenograft model

In the CTG-0256 model dosing of the single agents Compound 1 (IV at 20, 15, 6.25 mg/kg BIW) and carboplatin (IP at 50 mg/kg Q7D) yielded modest to weak anti-tumor activity compared to vehicle control. Combining Compound 1 at 15 and 6.25 mg/kg with carboplatin yielded additive combination effect. All treatment groups from the study are shown in **Table 3a.** The combination effect for this combination is shown in **Table 3b.**

**Table 3a: Combination of carboplatin and Compound 1 in the CTG-0256 xenograft model**

| Study Group | Treatment | Dosing Regimen | Route | Tumor Volume Day 14 | SEM Tumor Volume Day 14 | Number of mice in group (number on day 14) |
|---|---|---|---|---|---|---|
| 1 | 10% HPbCD, 0.9% saline | BIWx2 | IV | 1858.2 | 328.6 | 7 |
| 2 | 20mg/kg Compound 1 | BIWx2 | IV | 1406.7 | 164.6 | 6 |
| 3 | 15mg/kg Compound 1 | BIWx2 | IV | 1638.6 | 272.9 | 7 |
| 4 | 6.25mg/kg Compound 1 | BIWx2 | IV | 1291.5 | 288.4 | 7 |
| 5 | 50mg/kg carboplatin | Q7Dx2 | IP | 1898.1 | 348.4 | 7 |
| 6 | 15mg/kg Compound 1<br>50mg/kg carboplatin | BIWx2<br>Q7Dx2 | IV<br>IP | 1396.8 | 275.4 | 7 |
| 7 | 6.25mg/kg Compound 1<br>50mg/kg carboplatin | BIWx2<br>Q7Dx2 | IV<br>IP | 1902.8 | 386.9 | 7 |

**Table 3b: Classification for in vivo combination of carboplatin and Compound 1 in the CTG-0256 xenograft model**

| Treatment | Synergy score | SEM | P-value | Classification |
|---|---|---|---|---|
| Compound 1 (15 mg/kg) andcarboplatin (50 mg/kg) | -28.7 | 21.1 | 0.204 | Additive |
| Compound 1 (6.25 mg/kg) and carboplatin (50 mg/kg) | 2.5 | 26.0 | 0.926 | Additive |

## CTG-0486 xenograft model

In the CTG-0486 model dosing of the single agents Compound 1 (SC at 6.25, 15, 20 and 25 mg/kg on a BIW schedule for 3 weeks) and carboplatin (IP at 60 mg/kg on a Q7D for 3 weeks) yielded modest to strong anti-tumor activity compared to vehicle control (**Figure 3**). The 20 and 25 mg/kg doses of Compound 1 were not tolerated. Combining Compound 1 at 6.25 and 15 mg/kg with carboplatin yielded an additive combination effect. All treatment groups from the study are shown in **Table 4a.** The combination effect for this combination is shown in **Table 4b.** Mean tumor growth curves of all arms are shown in **Figure 3.** The error bars shown on the Figure 3 indicate the standard error measurements (SEM).

**Table 4a: Combination of carboplatin and Compound 1 in the CTG-0486 xenograft model**

| Study Group | Treatment | Dosing Regimen | Route | Tumor Volume Day 21 | SEM Tumor Volume Day 21 | Number of mice in group (number on day 21) |
|---|---|---|---|---|---|---|
| 1 | 10% HPbCD, 0.9% saline | BIWx3, Q7Dx3 | SC, IV | 491.4 | 63.1 | 5 |
| 2 | 25mg/kg Compound 1 | BIWx3 | SC | 151.7 | 34 | 2 |
| 3 | 20mg/kg Compound 1 | BIWx3 | SC | 92.7 | 25.7 | 2 |
| 4 | 15mg/kg Compound 1 | BIWx3 | SC | 131.9 | 32.5 | 5 |
| 5 | 6.25mg/kg Compound 1 | BIWx3 | SC | 476.9 | 88.4 | 5 |
| 6 | 60mg/kg carboplatin | Q7Dx3 | IV | 400.6 | 43.8 | 5 |
| 7 | 15 mg/kg Compound 1, 60mg/kg carboplatin | BIWx3, Q7Dx3 | SC, IV | 101 | 10 | 4 |
| 8 | 6.25 mg/kg Compound 1, 60mg/kg carboplatin | BIWx3, Q7Dx3 | SC, IV | 378.5 | 37.4 | 5 |

**Table 4b: Classification for in vivo combination of carboplatin and Compound 1 in the CTG-0486 xenograft model**

| Treatment | Synergy score | SEM | P-value | Classification |
|---|---|---|---|---|
| Compound 1 (6.25 mg/kg) + carboplatin (60 mg/kg) | 42.9 | 40.5 | 0.313 | Additive |
| Compound 1 (15 mg/kg) + carboplatin (60 mg/kg) | 56.4 | 49.6 | 0.277 | Additive |

**Compound 1 and oxaliplatin**

**PHTX-24C xenograft model**

In the PHTX-24C model, dosing of the single agents Compound 1 (IV at 10 mk/kg BIW) and oxaliplatin (7.5 mg/kg IP Q7D) yielded modest anti-tumor activity compared to vehicle control. In this experiment several different dosing strategies were explored. Both simultaneous and sequencing of agents (Compound 1 and oxaliplatin) were evaluated. For simultaneous treatment, both agents were dosed within 5 minutes of each other (indicated as time=0). For sequencing treatments, 3 different dosing schedules were evaluated: 1) Compound 1 was dosed at time=0 and oxaliplatin was dosed at time=8 hours, 2) oxaliplatin was dosed at time=0 and Compound 1 dosed at time=8 hours, and 3) oxaliplatin was dosed at time=0 and Compound 1 was dosed time=24 hours. The combination arms of Compound 1 with oxaliplatin yielded synergistic anti-tumor effects. All treatment groups from the study are shown in **Table 5a.** Day 21 was the last day of the study. The combination benefit for this combination is shown in **Table 5b.** Mean tumor growth curves are shown in **Figure 4.** The error bars shown on the figure indicate the standard error measurements (SEM).

**Table 5a: Combination of oxaliplatin and Compound 1 in the PHTX-24C xenograft model**

| Study Group | Treatment | Dosing Regimen | Route | Tumor Volume Day 21 | SEM Tumor Volume Day 21 | Number of mice in group (number on day 21) |
|---|---|---|---|---|---|---|
| 1 | 10% HPbCD, 0.9% saline | BIWx3, Q7Dx3 | IV, IP | 1,009.5 | 160.4 | 5 |
| 2 | 10 mg/kg Compound 1 | BIWx3 | IV | 609.3 | 95.7 | 5 |
| 3 | 7.5 mg/kg oxaliplatin | Q7Dx2 | IP | 720.6 | 76.5 | 5 |
| 4 | 10 mg/kg Compound 1 at time=0, 7.5 mg/kg oxaliplatin at time=0 | BIWx3 Q7Dx3 | IV, IP | 305.8 | 54.8 | 5 |
| 5 | 10 mg/kg Compound 1 at time=0, 7.5 mg/kg oxaliplatin at time=8 hours | BIWx3, Q7Dx3 | IV, IP | 287.5 | 59.6 | 5 |
| 6 | 10 mg/kg Compound 1 at time=8 hours, 7.5 mg/kg oxaliplatin at time=0 | BIWx3, Q7Dx3 | IV, IP | 200.4 | 41.6 | 5 |
| 7 | 10 mg/kg Compound 1 at time=24 hours, 7.5 mg/kg oxaliplatin at time=0 | BIWx3, Q7Dx3 | IV, IP | 311 | 25.4 | 5 |

**Table 5b: Classification for in vivo combination of oxaliplatin and Compound 1 in the PHTX-24C xenograft model**

| Treatment | Synergy score | SEM | P-value | Classification |
|---|---|---|---|---|
| 10mg/kg Compound 1 at time=0 + 7.5 mg/kg oxaliplatin at time=0 | -39.8 | 13.1 | 0.014 | Synergy |
| 10 mg/kg Compound 1 at time=0 + 7.5 mg/kg oxaliplatin at time=8 | -45.2 | 14 | 0.011 | Synergy |
| 10 mg/kg Compound 1 at time=8 hours + 7.5 mg/kg oxaliplatin at time=0 | -63.9 | 11.2 | <0.001 | Synergy |
| 10 mg/kg Compound 1 at time=24 hours + 7.5 mg/kg oxaliplatin at time=0 | -35 | 9.2 | 0.008 | Synergy |

## PHTX-17c xenograft model

In the PHTX-17c model dosing of the single agents Compound 1 (IV at 20, 25, 30 mg/kg BIW) and oxaliplatin (IP at 7.5 or 12.5 mg/kg Q7D) yielded modest anti-tumor activity compared to vehicle control. Combining Compound 1 at 20, 25 and 30 mg/kg with oxaliplatin at 7.5 or 12.5 mg/kg yielded additive combination effect. Combination of 30 mg/kg of Compound 1 and 12.5 mg/kg of oxaliplatin was not tolerated. All treatment groups from the study are shown in **Table 6a.** The combination effect for this combination is shown in **Table 6b.**

**Table 6a: Combination of oxaliplatin and Compound 1 in the PHTX-17c xenograft model**

| Study Group | Treatment | Dosing Regimen | Route | Tumor Volume Day 21 | SEM Tumor Volume Day 21 | Number of mice in group (number on day 21) |
|---|---|---|---|---|---|---|
| 1 | 20% HPbCD, 0.9% saline | BIW x 3<br>Q7D x 3 | IV,<br>IP | 2826.8 | 331.6 | 5 |
| 2 | 20mg/kg Compound 1 | BIWx3 | IV | 1875.6 | 393.1 | 5 |
| 3 | 25mg/kg Compound 1 | BIWx3 | IV | 2159.3 | 211.9 | 5 |
| 4 | 30mg/kg Compound 1 | BIWx3 | IV | 1612.7 | 356 | 5 |
| 5 | 7.5mg/kg oxaliplatin | Q7Dx3 | IP | 2017.5 | 303.9 | 5 |
| 6 | 12.5mg/kg oxaliplatin | Q7Dx3 | IP | 1758.9 | 184.7 | 5 |
| 7 | 20mg/kg Compound 1, 7.5mg/kg oxaliplatin | BIW x 3<br>Q7Dx3 | IV,<br>IP | 2210.6 | 182.1 | 5 |
| 8 | 20mg/kg Compound 1, 12.5mg/kg oxaliplatin | BIW x 3<br>Q7D x 3 | IV,<br>IP | 1687.6 | 207.6 | 5 |
| 9 | 25mg/kg Compound 1, 7.5mg/kg oxaliplatin | BIW x 3<br>Q7D x 3 | IV,<br>IP | 1800 | 238.4 | 5 |
| 10 | 25mg/kg Compound 1, 12.5mg/kg oxaliplatin | BIW x 3<br>Q7D x 3 | IV,<br>IP | 1505.9 | 335.8 | 5 |
| 11 | 30mg/kg Compound 1, 7.5mg/kg oxaliplatin | BIW x 3<br>Q7D x 3 | IV<br>IP | 1382.2 | 163 | 5 |
| 12 | 30mg/kg Compound 1, 12.5mg/kg oxaliplatin | BIW x 3<br>Q7D x 3 | IV<br>IP | 1560.8 | 411.1 | 3 |

**Table 6b: Classification for in vivo combination of oxaliplatin and Compound 1 in the PHTX-17c xenograft model**

| Treatment | Synergy score | SEM | P-value | Classification |
|---|---|---|---|---|
| Compound 1 (20 mg/kg, BIW x 3) + oxaliplatin (7.5 mg/kg, Q7D x 3) | 11.7 | 12.8 | 0.382 | Additive |

(continued)

| Treatment | Synergy score | SEM | P-value | Classification |
|---|---|---|---|---|
| Compound 1 (20 mg/kg, BIW x 3) + oxaliplatin (12.5 mg/kg, Q7D x 3) | 8.7 | 8.0 | 0.322 | Additive |
| Compound 1 (25 mg/kg, BIW x 3) + oxaliplatin (7.5 mg/kg, Q7D x 3) | -5.1 | 14.2 | 0.725 | Additive |
| Compound 1 (25 mg/kg, BIW x 3) + oxaliplatin (12.5 mg/kg, Q7D x 3) | -13.0 | 10.8 | 0.265 | Additive |
| Compound 1 (30 mg/kg, BIW x 3) + oxaliplatin (7.5 mg/kg, Q7D x 3) | -2.4 | 11.0 | 0.830 | Additive |
| Compound 1 (30 mg/kg, BIW x 3) + oxaliplatin (12.5 mg/kg, Q7D x 3) | -2.3 | 6.9 | 0.749 | Additive |

**Compound 1 and cisplatin (All below-mentioned examples with cisplatin are considered to be reference examples.)**

**CTG-0165 xenograft model**

In the CTG-0165 model dosing of the single agents Compound 1 (IV at 20, 15, 6.25 mg/kg BIW) and cisplatin (7.5 mg/kg Q7D) yielded modest to weak anti-tumor activity compared to vehicle control. Combining Compound 1 at 15 and 6.25 mg/kg with cisplatin yielded additive combination effect. All treatment groups from the study are shown in **Table 7a.** The combination effect for this combination is shown in **Table 7b.**

**Table 7a: Combination of cisplatin and Compound 1in the CTG-0165 xenograft model**

| Study Group | Treatment | Dosing Regimen | Route | Tumor Volume Day 21 | SEM Tumor Volume Day 21 | Number of mice in group (number on day 21) |
|---|---|---|---|---|---|---|
| 1 | 10% HPbCD | BIWx3 | IV | 1332 | 262.7 | 5 |
| 2 | 20 mg/kg Compound 1 | BIWx3 | IV | 1476.3 | 441.6 | 5 |
| 3 | 15mg/kg Compound 1 | BIWx3 | IV | 1624.9 | 388.8 | 5 |
| 4 | 6.25mg/kg Compound 1 | BIWx3 | IV | 988.8 | 224.9 | 5 |
| 5 | 7.5mg/kg cisplatin | QWx3 | IV | 489.3 | 152.4 | 4 |
| 6 | 15mg/kg Compound 1 + 7.5mg/kg cisplatin | BIWx3, QWx3 | IV, IV | 607 | 186.5 | 4 |
| 7 | 6.25mg/kg Compound 1 + 7.5mg/kg cisplatin | BIWx3, QWx3 | IV, IV | 506.7 | 262.6 | 5 |

**Table 7b: Classification for in vivo combination of cisplatin and Compound 1 in the CTG-0165 xenograft model**

| Treatment | Synergy score | SEM | P-value | Classification |
|---|---|---|---|---|
| Compound 1 (15 mg/kg) and cisplatin (7.5 mg/kg) | -8.2 | 26.3 | 0.759 | Additive |

(continued)

| Treatment | Synergy score | SEM | P-value | Classification |
|---|---|---|---|---|
| Compound 1 (15 mg/kg) and cisplatin (7.5 mg/kg) | 9.7 | 22.1 | 0.667 | Additive |

**Compound 1 and docetaxel**

**PHTX-02B xenograft model**

In the PHTX-02B breast xenograft model, dosing of the single agents Compound 1 (SC at 7.5, 15 and 20 mg/kg BIW; IV also 20 mg/kg BIW) and docetaxel (5 and 10 mg/kg IV QW) inhibited tumor growth compared to the vehicle control group. The combination treatment using these doses and schedules led to additive combination effects. All treatment groups from the study are shown in **Table 8a.** The combination benefit for this combination in this study was scored and is shown in **Table 8b.** Mean tumor growth curves are shown in **Figure 5.** The error bars shown on the figure indicate the standard error measurements (SEM).

**Table 8a: Combination of docetaxel and Compound 1 in the PHTX-02B xenograft model**

| Study Group | Treatment | Dosing Regimen | Route | Tumor Volume Day 21 | SEM Tumor Volume Day 21 | Number of mice in group (number on day 21) |
|---|---|---|---|---|---|---|
| 1 | 20% HPbCD, 0.9% saline | BIWx3, Q7Dx3 | SC, IV | 1061 | 464.4 | 5 |
| 2 | 20 mg/kg Compound 1 | BIWx3 | IV | 178.8 | 44.1 | 5 |
| 3 | 20 mg/kg Compound 1 | BIWx3 | SC | 185 | 81.4 | 5 |
| 4 | 15 mg/kg Compound 1 | BIWx3 | SC | 324.8 | 158.9 | 5 |
| 5 | 7.5 mg/kg Compound 1 | BIWx3 | SC | 596.5 | 241.4 | 5 |
| 6 | 5 mg/kg docetaxel | Q7Dx3 | IV | 492.3 | 200.4 | 5 |
| 7 | 10 mg/kg docetaxel | Q7Dx3 | IV | 232.2 | 122.6 | 5 |
| 8 | 15 mg/kg Compound 1, 10 mg/kg docetaxel | BIWx3, Q7Dx3 | SC, IV | 48.1 | 32.9 | 5 |
| 9 | 7.5 mg/kg Compound 1, 10 mg/kg docetaxel | BIWx3, Q7Dx3 | SC,IV | 97.8 | 49.2 | 5 |
| 10 | 15 mg/kg Compound 1, 5 mg/kg docetaxel | BIWx3, Q7Dx3 | SC, IV | 145.3 | 57 | 5 |
| 11 | 7.5 mg/kg Compound 1, 5 mg/kg docetaxel | BIWx3, Q7Dx3 | SC, IV | 209.4 | 166.7 | 5 |

**Table 8b: Classification for in vivo combination of docetaxel and Compound 1 in the PHTX-02B xenograft model**

| Treatment | Synergy score | SEM | P-value | Classification |
|---|---|---|---|---|
| 15 mg/kg Compound 1 + 10 mg/kg docetaxel | 29 | 22 | 0.208 | Additive |
| 7.5 mg/kg Compound 1 + 10 mg/kg docetaxel | 22 | 21 | 0.303 | Additive |
| 15 mg/kg Compound 1 + 5 mg/kg docetaxel | 10 | 18 | 0.582 | Additive |
| 7.5 mg/kg Compound 1 + 5 mg/kg docetaxel | 17 | 19 | 0.372 | Additive |

## Calu-6 xenograft model

In the Calu-6 NSCLC xenograft model, dosing of the single agents Compound 1 (IV at 5, 10 and 20 mg/kg IV BIW) and docetaxel (5 and 10 mg/kg IV QW) yielded modest anti-tumor responses compared to the vehicle control. The combination treatment arms using these doses and schedules resulted in additive to synergistic anti-tumor combination effects. All treatment groups from the study are shown in **Table 9a.** The combination benefit for this study was scored and is shown in **Table 9b.** Arms 1, 3, 6 and 9 are shown as an example of a combination exhibiting a synergistic effect in **Figure 6a.** Mean tumor growth curves of all arms are shown in **Figure 6b.** The error bars shown on the Figures indicate the standard error measurements (SEM).

**Table 9a: Combination of docetaxel and Compound 1 in the Calu-6 xenograft model**

| Study Group | Treatment | Dosing Regimen | Route | Tumor Volume Day 21 | SEM Tumor Volume Day 21 | Number of mice in group (number on day 21) |
|---|---|---|---|---|---|---|
| 1 | 20% HPbCD, 0.9% saline | BIWx3, Q7Dx3 | IV, IP | 1258.3 | 259.3 | 6 |
| 2 | 15 mg/kg Compound 1 | BIWx3 | IV | 1072.4 | 216.9 | 6 |
| 3 | 10 mg/kg Compound 1 | BIWx3 | IV | 1346.4 | 216.9 | 6 |
| 4 | 5 mg/kg Compound 1 | BIWx3 | IV | 1302.2 | 272.1 | 6 |
| 5 | 5 mg/kg docetaxel | Q7Dx3 | IV | 743.7 | 158.3 | 6 |
| 6 | 10 mg/kg docetaxel | Q7Dx3 | IV | 882.6 | 265.4 | 6 |
| 7 | 15 mg/kg Compound 1, 10 mg/kg docetaxel | BIWx3, Q7Dx3 | IV, IV | 88.5 | 17 | 6 |
| 8 | 15 mg/kg Compound 1, 5 mg/kg docetaxel | BIWx3, Q7Dx3 | IV, IV | 333.2 | 109.6 | 5 |
| 9 | 10 mg/kg Compound 1, 10 mg/kg docetaxel | BIWx3, Q7Dx3 | IV | 246.5 | 58.6 | 6 |
| 10 | 10 mg/kg Compound 1, 5 mg/kg docetaxel | BIWx3, Q7Dx3 | IV | 505.8 | 108.8 | 6 |
| 11 | 5 mg/kg Compound 1, 10 mg/kg docetaxel | BIWx3, Q7Dx3 | IV | 236 | 53.8 | 6 |
| 12 | 5 mg/kg Compound 1, 5 mg/kg docetaxel | BIWx3, Q7Dx3 | IV | 791.2 | 200.5 | 6 |

**Table 9b: Classification for in vivo combination of docetaxel and Compound 1 in the Calu-6 xenograft model**

| Treatment | Synergy score | SEM | P-value | Classification |
|---|---|---|---|---|
| 15 mg/kg Compound 1, 10 mg/kg docetaxel | -58.2 | 13.8 | 0.001 | Synergy |
| 15 mg/kg Compound 1, 5 mg/kg docetaxel | -15.9 | 19 | 0.417 | Additive |
| 10 mg/kg Compound 1, 10 mg/kg docetaxel | -47.5 | 11.2 | 0.001 | Synergy |
| 10 mg/kg Compound 1, 5 mg/kg docetaxel | -19.9 | 15.6 | 0.225 | Additive |
| 5 mg/kg Compound 1, 10 mg/kg docetaxel | -48.8 | 15.8 | 0.012 | Synergy |
| 5 mg/kg Compound 1, 5 mg/kg docetaxel | -1.5 | 18.3 | 0.937 | Additive |

## PHTX-14B xenograft model

In the PHTX-14B breast xenograft model, dosing of the single agents Compound 1 (SC at 20 and 10 mg/kg BIW) and docetaxel (5 and 10 mg/kg IV QW) inhibited tumor growth compared to the vehicle control group. The combination treatment using these doses and schedules led to additive combination effects. All treatment groups from the study are shown in **Table 10a.** The combination benefit for this combination in this study was scored and is shown in **Table 10b.** Mean tumor growth curves are shown in Figure 7. The error bars shown on the Figure indicate the standard error measurements (SEM).

**Table 10a: Combination of docetaxel and Compound 1 in the PHTX-14B xenograft model**

| Study Group | Treatment | Dosing Regimen | Route | Tumor Volume Day 28 | SEM Tumor Volume Day 28 | Number of mice in group (number on day 28) |
|---|---|---|---|---|---|---|
| 1 | 10% HPbCD, 0.9% saline | BIWx3, Q7Dx3 | SC,IV | 607.1 | 127.3 | 5 |
| 2 | 20 mg/kg Compound 1 | BIWx3 | SC | 399.4 | 114.9 | 5 |
| 3 | 10 mg/kg Compound 1 | BIWx3 | SC | 499.4 | 115.5 | 5 |
| 4 | 10 mg/kg docetaxel | Q7Dx3 | IV | 195.5 | 45.7 | 5 |
| 5 | 5 mg/kg docetaxel | Q7Dx3 | IV | 590.4 | 132.5 | 5 |
| 6 | 20mg/kg Compound 1, + 10 mg/kg docetaxel | BIWx3, Q7Dx3 | SC,IV | 63.6 | 19.7 | 5 |
| 7 | 20 mg/kg Compound 1, + 5mg/kg docetaxel | BIWx3, Q7Dx3 | SC, IV | 199.1 | 58.7 | 5 |
| 8 | 10mg/kg Compound 1, + 10 mg/kg docetaxel | BIWx3, Q7Dx3 | SC, IV | 130.7 | 60.6 | 5 |
| 9 | 10 mg/kg Compound 1, + 5 mg/kg docetaxel | BIWx3, Q7Dx3 | SC, IV | 287.4 | 90.5 | 5 |

**Table 10b: Classification for in vivo combination of docetaxel and Compound 1 in the PHTX-14B xenograft model**

| Treatment | Synergy score | SEM | P-value | Classification |
|---|---|---|---|---|
| 20 mg/kg Compound 1 + 10 mg/kg docetaxel | -70.0 | 46.6 | 0.160 | Additive |
| 20 mg/kg Compound 1 + 5 mg/kg docetaxel | -139.2 | 75.9 | 0.111 | Additive |
| 10 mg/kg Compound 1 + 10 mg/kg docetaxel | -34.5 | 52.4 | 0.526 | Additive |
| 10 mg/kg Compound 1 + 5 mg/kg docetaxel | -62.5 | 47.9 | 0.218 | Additive |

## CTG-0159 xenograft model

In the CTG-0159 NSCLC xenograft model, Compound 1 treatment alone at 20, 15, and 6.25 mg/kg IV on a BIW schedule did not inhibit tumor growth. Combining Compound 1 (15 and 6.25 mg/kg, IV, BIWx3) with docetaxel (10 mg/kg IV, QWx3) yielded additive combination effects. All treatment groups from the study are shown in **Table 11a**. The combination benefit for this study was scored and is shown in **Table 11b**. Mean tumor growth curves are shown in **Figure 8.**

**Table 11a: Combination of docetaxel and Compound 1 in the CTG-0159 xenograft model**

| Study Group | Treatment | Dosing Regimen | Route | Tumor Volume Day 21 | SEM Tumor Volume Day 21 | Number of mice in group (number on day 21) |
|---|---|---|---|---|---|---|
| 1 | 10% HPbCD | BIWx3, | IV, | 1323.8 | 295.9 | 7 |
| 2 | 20 mg/kg Compound 1 | BIWx3 | IV | 1376.2 | 554.7 | 7 |
| 3 | 15 mg/kg Compound 1 | BIWx3 | IV | 2373.2 | 327.7 | 7 |
| 4 | 6.25 mg/kg Compound 1 | BIWx3 | IV | 1618.9 | 465.6 | 7 |
| 5 | 10 mg/kg docetaxel | QWx3 | IV | 103.2 | 42.3 | 7 |
| 6 | 15mg/kg Compound 1, 10 mg/kg docetaxel | BIWx3, QWx3 | IV, IV | 186.3 | 95.5 | 7 |
| 7 | 6.25 mg/kg Compound 1, 10mg/kg docetaxel | BIWx3, QWx3 | IV, IV | 47.6 | 16.8 | 7 |

**Table 11b: Classification for in vivo combination of docetaxel and Compound 1 in the CTG-0159 xenograft model**

| Treatment | Synergy score | SEM | P-value | Classification |
|---|---|---|---|---|
| 15 mg/kg Compound 1 + 10 mg/kg docetaxel | -13.3 | 36.2 | 0.719 | Additive |
| 6.25 mg/kg Compound 1 + 10 mg/kg docetaxel | -45.2 | 33.5 | 0.195 | Additive |

**Compound 1 and paclitaxel (All below-mentioned examples with paclitaxel are considered to be reference examples.)**

**PHTX-02B xenograft model**

In the PHTX-02B model dosing of the single agents Compound 1 (IV at 6.25, and 12.5 mg/kg on a BIW schedule for 3 weeks) and paclitaxel (IV at 10 mg/kg on a Q4D for 3 weeks) yielded minimal to modest anti-tumor activity compared to vehicle control. Dosing 20 mg/kg paclitaxel (IV at 20 mg/kg a Q4D for 3 weeks) yielded strong anti-tumor activity. Combining Compound 1 at 12.5 mg/kg with paclitaxel yielded an additive combination effect. All treatment groups from the study are shown in **Table 12a.** The combination effect for this combination is shown in **Table 12b.**

**Table 12a: Combination of paclitaxel and Compound 1 in the PHTX-02B xenograft model**

| Study Group | Treatment | Dosing Regimen | Route | Tumor Volume Day 19 | SEM Tumor Volume Day 19 | Number of mice in group (number on day 19) |
|---|---|---|---|---|---|---|
| 1 | 20% HPbCD, 0.9% saline | BIWx3, Q4Dx3 | IV, IV | 1864 | 486.5 | 5 |
| 2 | 6.25 mg/kg Compound 1 | BIWx3 | IV | 1850.4 | 516.8 | 5 |
| 3 | 12.5 mg/kg Compound 1 | BIWx3 | IV | 1613.2 3 | 401.3 | 5 |
| 4 | 10 mg/kg paclitaxel | Q4Dx3 | IV | 1093.5 | 216.9 | 5 |
| 5 | 20mg/kg paclitaxel | Q4Dx3 | IV | 245.2 | 36.5 | 5 |
| 6 | 12.5mg/kg Compound 1, 10 mg/kg paclitaxel | BIWx3, Q4Dx3 | IV, IV | 667.1 | 145.7 | 5 |

**Table 12b: Classification for in vivo combination of paclitaxel and Compound 1 in the PHTX-02B xenograft model**

| Treatment | Synergy score | SEM | P-value | Classification |
|---|---|---|---|---|
| 12.5 mg/kg Compound 1 + 10 mg/kg paclitaxel | -12.8 | 13.2 | 0.351 | Additive |

**PHTX-14B xenograft model**

In the PHTX-14B model dosing of the single agents Compound 1 (SC at 10, and 20 mg/kg on a BIW schedule for 4 weeks) and paclitaxel (10 mg/kg IV on a Q7D schedule for 4 weeks) yielded modest anti-tumor activity compared to vehicle control. Dosing paclitaxel (20 mg/kg IV on a Q7D schedule for 4 weeks) and single agents Compound 1(SC at 30 mg/kg on a BIW for 4 weeks) yielded strong anti-tumor activity (**Figure 9**). Combining Compound 1 with paclitaxel yielded additive combination effects. All treatment groups from the study are shown in **Table 13a.** The combination effect for this combination is shown in **Table 13b.** Mean tumor growth curves are shown in **Figure 9.** The error bars shown on the Figure indicate the standard error measurements (SEM).

**Table 13a: Combination of paclitaxel and Compound 1 in the PHTX-14B xenograft model**

| Study Group | Treatment | Dosing Regimen | Route | Tumor Volume Day 28 | SEM Tumor Volume Day 28 | Number of mice in group (number on day 28) |
|---|---|---|---|---|---|---|
| 1 | 20% HPbCD, 0.9% saline | BIWx4, Q7Dx4 | SC, IV | 834.2 | 105.2 | 5 |

(continued)

| Study Group | Treatment | Dosing Regimen | Route | Tumor Volume Day 28 | SEM Tumor Volume Day 28 | Number of mice in group (number on day 28) |
|---|---|---|---|---|---|---|
| 2 | 10mg/kg Compound 1 | BIWx4 | SC | 690.0 | 155.7 | 5 |
| 3 | 20mg/kg Compound 1 | BIWx4 | SC | 480.6 | 100.0 | 5 |
| 4 | 10mg/kg paclitaxel | Q7Dx4 | IV | 584.4 | 208.8 | 5 |
| 5 | 20mg/kg paclitaxel | Q7Dx4 | IV | 283.6 | 88.1 | 5 |
| 6 | 10mg/kg Compound, 110mg/kg paclitaxel | BIWx4, Q7Dx4 | SC, IV | 428.5 | 98.1 | 5 |
| 7 | 10mg/kg Compound 1, 20mg/kg paclitaxel | BIWx4, Q7Dx4 | SC, IV | 281.9 | 41.8 | 5 |
| 8 | 20mg/kg Compound 1, 10mg/kg paclitaxel | BIWx4, Q7Dx4 | SC, IV | 435.6 | 95.2 | 5 |
| 9 | 20mg/kg Compound 1, 20mg/kg paclitaxel | BIWx4, Q7Dx4 | SC, IV | 74.0 | 17.6 | 4 |
| 10 | 30mg/kg Compound 1 | BIWx4 | SC | 165.1 | 64.5 | 5 |

**Table 13b: Classification for in vivo combination of paclitaxel and Compound 1 in the PHTX-14B xenograft model**

| Treatment | Synergy score | SEM | P-value | Classification |
|---|---|---|---|---|
| Compound 1 (10 mg/kg, BIW x 4) + paclitaxel (10 mg/kg, Q7D x 4) | 1.4 | 20.5 | 0.946 | Additive |
| Compound 1 (10 mg/kg, BIW x 4) + paclitaxel (20 mg/kg, Q7D x 4) | 18.4 | 14.9 | 0.255 | Additive |
| Compound 1 (20 mg/kg, BIW x 4) + paclitaxel (10 mg/kg, Q7D x 4) | 20.4 | 16.3 | 0.242 | Additive |
| Compound 1 (20 mg/kg, BIW x 4) + paclitaxel (20 mg/kg, Q7D x 4) | -32.7 | 25.1 | 0.239 | Additive |

**CTG-0012 xenograft model**

In the CTG-0012 model dosing of the single agents Compound 1 (IV at 6.25, 15, and 20 mg/kg on a BIW schedule for 3 weeks) and paclitaxel (IV at 10 mg/kg on a Q4D for 3 weeks) yielded modest to strong anti-tumor activity compared to vehicle control. Combining Compound 1 at 6.25 and 15 mg/kg with paclitaxel yielded an additive combination effect. All treatment groups from the study are shown in **Table 14a.** The combination effect for this combination is shown in **Table 14b.**

**Table 14a: Combination of paclitaxel and Compound 1 in the CTG-0012 xenograft model**

| Study Group | Treatment | Dosing Regimen | Route | Tumor Volume Day 21 | SEM Tumor Volume Day 21 | Number of mice in group (number on day 21) |
|---|---|---|---|---|---|---|
| 1 | 10% HPbCD, 0.9% saline | BIWx3, Q4Dx3 | IV, IV | 1092.8 | 338.8 | 7 |
| 2 | 20mg/kg Compound 1 | BIWx3 | IV | 47.4 | 56.1 | 7 |

(continued)

| Study Group | Treatment | Dosing Regimen | Route | Tumor Volume Day 21 | SEM Tumor Volume Day 21 | Number of mice in group (number on day 21) |
|---|---|---|---|---|---|---|
| 3 | 15mg/kg Compound 1 | BIWx3 | IV | 185 | 161 | 7 |
| 4 | 6.25mg/kg Compound 1 | BIWx3 | IV | 815 | 266.5 | 7 |
| 5 | 10mg/kg paclitaxel | Q4Dx3 | IV | 731.1 | 340.8 | 7 |
| 6 | 15 mg/kg Compound 1 + 10mg/kg paclitaxel | BIWx3, Q4Dx3 | IV, IV | 178.8 | 205.6 | 7 |
| 7 | 6.25 mg/kg Compound 1 + 10mg/kg paclitaxel | BIWx3, Q4Dx3 | IV, IV | 559.7 | 320.8 | 7 |

**Table 14b: Classification for in vivo combination of paclitaxel and Compound 1 in the CTG-0012 xenograft model**

| Treatment | Synergy score | SEM | P-value | Classification |
|---|---|---|---|---|
| 6.25 mg/kg Compound 1+ 10mg/kg paclitaxel | 2.1 | 15.1 | 0.891 | Additive |

## Claims

**1.** A compound for use in treating cancer by administration in combination with one or more of:

i) a platin; or
ii) a taxane;

wherein the compound is ((1R,2R,3S,4R)-2,3-dihydroxy-4-(2-(3 (trifluoromediylthio)phenyl)pyrazolo[1,5-a]pyrimidin-7-ylamino)cyclopentyl)methyl sulfamate (Compound 1) or a pharmaceutically acceptable salt thereof;
wherein the platin is carboplatin or oxaliplatin;
wherein the taxane is docetaxel.

**2.** The compound for use of claim 1, wherein the cancer is breast cancer, colorectal cancer, ovarian cancer, lung cancer, prostate cancer or head and neck cancer.

**3.** The compound for use of claim 1, wherein the cancer is acute myeloid leukemia, myelodysplastic syndromes, multiple myeloma, or non-Hodgkin's lymphoma.

**4.** The compound for use of any of claims 1-3, wherein ((lR,2R,3S,4R)-2,3-dihydroxy-4-(2-(3-(trifluoromethylthio)phenyl)pyrazolo[1,5-a]pyrimidin-7-ylamino)cyclopentyl)methyl sulfamate (Compound 1) or a pharmaceutically acceptable salt thereof is administered on

a) each of days 1, 4, 8, and 11 of a 21 day schedule; or
b) each of days 1, 8, and 15 of a 21 day schedule; or
c) each of days 1, 8, and 15 of a 28 day schedule.

**5.** The compound for use of any of claims 1-4, wherein ((lR,2R,3S,4R)-2,3-dihydroxy-4-(2-(3-(trifluoromethylthio)phenyl)pyrazolo[1,5-a]pyrimidin-7-ylamino)cyclopentyl)methyl sulfamate (Compound 1) or a pharmaceutically acceptable salt thereof, is administered in combination with docetaxel.

**6.** The compound for use of claim 5, wherein docetaxel is administered on:

a) day 1 of a 21 day schedule; or
b) each of days 1, 8, and 15 of a 21 day schedule; or
c) each of days 1, 8 and 15 of a 28 day schedule.

**7.** The compound for use of claim 1-4, wherein ((lR,2R,3S,4R)-2,3-dihydroxy-4-(2-(3-(trifluoromethylthio)phenyl)pyrazolo[1,5-a]pyrimidin-7-ylamino)cyclopentyl)methyl sulfamate (Compound 1) or a pharmaceutically acceptable salt thereof is administered in combination with a platin, wherein the platin is carboplatin or oxaliplatin.

**8.** The compound for use of claim 7, wherein the platin is carboplatin.

**9.** The compound for use of claim 7, wherein the platin is oxaliplatin.

**10.** The compound for use of any one of claims 7-9, wherein carboplatin or oxaliplatin is administered on:

a) day 1 of a 21 day schedule; or
b) each of days 1, 8, and 15 of a 21 day schedule; or
c) each of days 1, 8 and 15 of a 28 day schedule.

**11.** The compound for use of any one of claims 8-10, further comprising administration in combination with docetaxel.

**Patentansprüche**

**1.** Verbindung zur Verwendung bei der Behandlung von Krebs durch Verabreichung in Kombination mit einem oder mehreren von

i) einem Platin oder
ii) einem Taxan;

wobei es sich bei der Verbindung um ((1R,2R,3S,4R)-2,3-Dihydroxy-4-(2-(3-(trifluormethylthio)phenyl)pyrazolo[1,5-a]pyrimidin-7-yl-amino)cyclopentyl)methylsulfamat (Verbindung 1) oder ein pharmazeutisch unbedenkliches Salz davon handelt;
wobei es sich bei dem Platin um Carboplatin oder Oxaliplatin handelt;
wobei sich bei dem Taxan um Docetaxel handelt.

**2.** Verbindung zur Verwendung nach Anspruch 1, wobei es sich bei dem Krebs um Brustkrebs, Kolorektalkrebs, Eierstockkrebs, Lungenkrebs, Prostatakrebs oder Kopf- und Halskrebs handelt.

**3.** Verbindung zur Verwendung nach Anspruch 1, wobei es sich bei dem Krebs um akute myeloische Leukämie, myelodysplastische Syndrome, multiples Myelom oder Non-Hodgkin-Lymphom handelt.

**4.** Verbindung zur Verwendung nach einem der Ansprüche 1-3, wobei ((1R,2R,3S,4R)-2,3-Dihydroxy-4-(2-(3-(trifluormethylthio)phenyl)pyrazolo[1,5-a]-pyrimidin-7-ylamino)cyclopentyl)methylsulfamat (Verbindung 1) oder ein pharmazeutisch unbedenkliches Salz davon

a) an jedem der Tage 1, 4, 8 und 11 eines 21-Tage-Plans oder
b) an jedem der Tage 1, 8 und 15 eines 21-Tage-Plans oder
c) an jedem der Tage 1, 8 und 15 eines 28-Tage-Plans

verabreicht wird.

**5.** Verbindung zur Verwendung nach einem der Ansprüche 1-4, wobei ((1R,2R,3S,4R)-2,3-Dihydroxy-4-(2-(3-(trifluormethylthio)phenyl)pyrazolo[1,5-a]-pyrimidin-7-ylamino)cyclopentyl)methylsulfamat (Verbindung 1) oder ein pharmazeutisch unbedenkliches Salz davon in Kombination mit Docetaxel verabreicht wird.

**6.** Verbindung zur Verwendung nach Anspruch 5, wobei Docetaxel

a) an Tag 1 eines 21-Tage-Plans oder
b) an jedem der Tage 1, 8 und 15 eines 21-Tage-Plans oder
c) an jedem der Tage 1, 8 und 15 eines 28-Tage-Plans

verabreicht wird.

**7.** Verbindung zur Verwendung nach Anspruch 1-4, wobei ((1R,2R,3S,4R)-2,3-Dihydroxy-4-(2-(3-(trifluormethylthio)phenyl)pyrazolo[1, 5-a]pyrimidin-7-yl-amino)cyclopentyl)methylsulfamat (Verbindung 1) oder ein pharmazeutisch unbedenkliches Salz davon in Kombination mit einem Platin verabreicht wird, wobei es sich bei dem Platin um Oxaliplatin handelt.

**8.** Verbindung zur Verwendung nach Anspruch 7, wobei es sich bei dem Platin um Carboplatin handelt.

**9.** Verbindung zur Verwendung nach Anspruch 7, wobei es sich bei dem Platin um Oxaliplatin handelt.

**10.** Verbindung zur Verwendung nach einem der Ansprüche 7-9, wobei Carboplatin bzw. Oxaliplatin

a) an Tag 1 eines 21-Tage-Plans oder
b) an jedem der Tage 1, 8 und 15 eines 21-Tage-Plans oder
c) an jedem der Tage 1, 8 und 15 eines 28-Tage-Plans

verabreicht wird.

**11.** Verbindung zur Verwendung nach einem der Ansprüche 8-10, ferner umfassend eine Verabreichung in Kombination mit Docetaxel.

## Revendications

**1.** Composé pour une utilisation dans le traitement d'un cancer par administration en combinaison avec l'un ou plusieurs parmi :

i) un composé du platine ; et
ii) un taxane ;

le composé étant le sulfamate de ((1R,2R,3S,4R)-2,3-dihydroxy-4(2-(3-(trifluorométhylthio)phényl)pyrazolo[1, 5-a]pyrimidin-7-ylamino)cyclopentyl)méthyle (Composé 1) ou un sel pharmaceutiquement acceptable correspondant ;
le composé du platine étant le carboplatine ou l'oxaliplatine ;
le taxane étant le docétaxel.

**2.** Composé pour une utilisation selon la revendication 1, le cancer étant un cancer du sein, un cancer colorectal, un cancer de l'ovaire, un cancer du poumon, un cancer de la prostate ou un cancer de la tête et du cou.

**3.** Composé pour une utilisation selon la revendication 1, le cancer étant une leucémie myéloïde aiguë, des syndromes myélodysplasiques, un myélome multiple, ou un lymphome non hodgkinien.

**4.** Composé pour une utilisation selon l'une quelconque des revendications 1 à 3, le sulfamate de ((1R,2R,3S,4R)-2,3-dihydroxy-4(2-(3-(trifluorométhylthio)phényl)pyrazolo[1,5-a]pyrimidin-7-ylamino) cyclopentyl) méthyle (Composé 1) ou un sel pharmaceutiquement acceptable correspondant étant administré

a) chacun des jours 1, 4, 8, et 11 d'un schéma d'administration de 21 jours ; ou
b) chacun des jours 1, 8, et 15 d'un schéma d'administration de 21 jours ; ou
c) chacun des jours 1, 8, et 15 d'un schéma d'administration de 28 jours.

**5.** Composé pour une utilisation selon l'une quelconque des revendications 1 à 4, le sulfamate de ((1R,2R,3S,4R)-2,3-dihydroxy-4(2-(3-(trifluorométhylthio)phényl)pyrazolo[1,5-a]pyrimidin-7-ylamino)cyclopentyl)méthyle (Composé 1) ou un sel pharmaceutiquement acceptable correspondant étant administré en combinaison avec le docétaxel.

**6.** Composé pour une utilisation selon la revendication 5, le docétaxel étant administré :

a) le jour 1 d'un schéma d'administration de 21 jours ; ou
b) chacun des jours 1, 8, et 15 d'un schéma d'administration de 21 jours ; ou
c) chacun des jours 1, 8, et 15 d'un schéma d'administration de 28 jours.

**7.** Composé pour une utilisation selon l'une quelconque des revendications 1 à 4, le sulfamate de ((1R,2R,3S,4R)-2,3-dihydroxy-4(2-(3-(trifluorométhylthio)phényl)pyrazolo[1,5-a]pyrimidin-7-ylamino)cyclopentyl)méthyle (Composé 1) ou un sel pharmaceutiquement acceptable correspondant étant administré en combinaison avec un composé du platine, le composé du platine étant le carboplatine ou l'oxaliplatine.

**8.** Composé pour une utilisation selon la revendication 7, le composé du platine étant le carboplatine.

**9.** Composé pour une utilisation selon la revendication 7, le composé du platine étant l'oxaliplatine.

**10.** Composé pour une utilisation selon l'une quelconque des revendications 7 à 9, le carboplatine ou l'oxaliplatine étant administré :

a) le jour 1 d'un schéma d'administration de 21 jours ; ou
b) chacun des jours 1, 8, et 15 d'un schéma d'administration de 21 jours ; ou
c) chacun des jours 1, 8, et 15 d'un schéma d'administration de 28 jours.

**11.** Composé pour une utilisation selon l'une quelconque des revendications 8 à 10, comprenant en outre une administration en combinaison avec le docétaxel.

**FIGURE 1: The anti-tumor activity of Compound 1 and carboplatin in the PHTX-132Lu xenograft model**

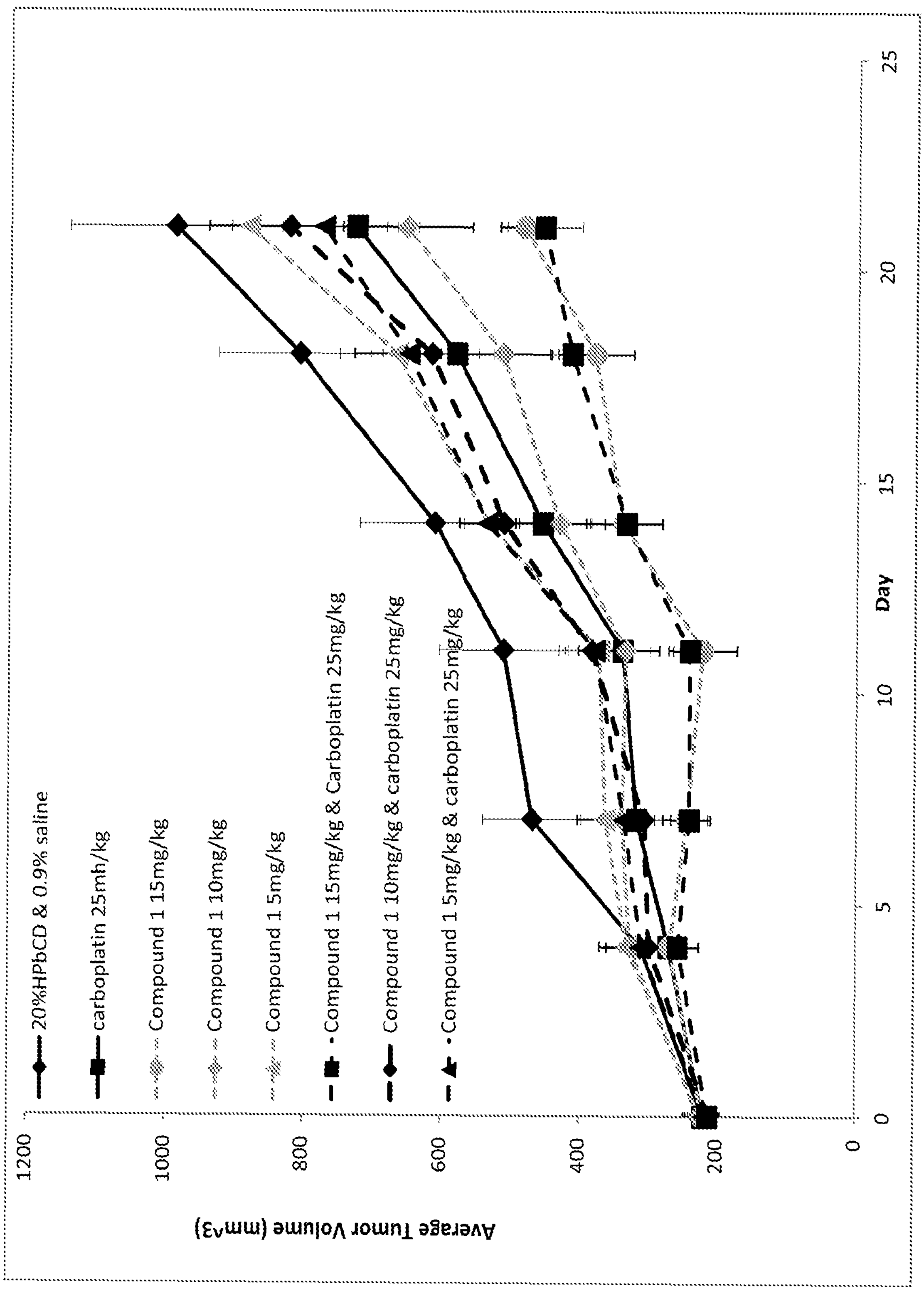

**FIGURE 2a The anti-tumor activity of Compound 1 at 15 mg/kg and carboplatin in the SKOV-3 xenograft model**

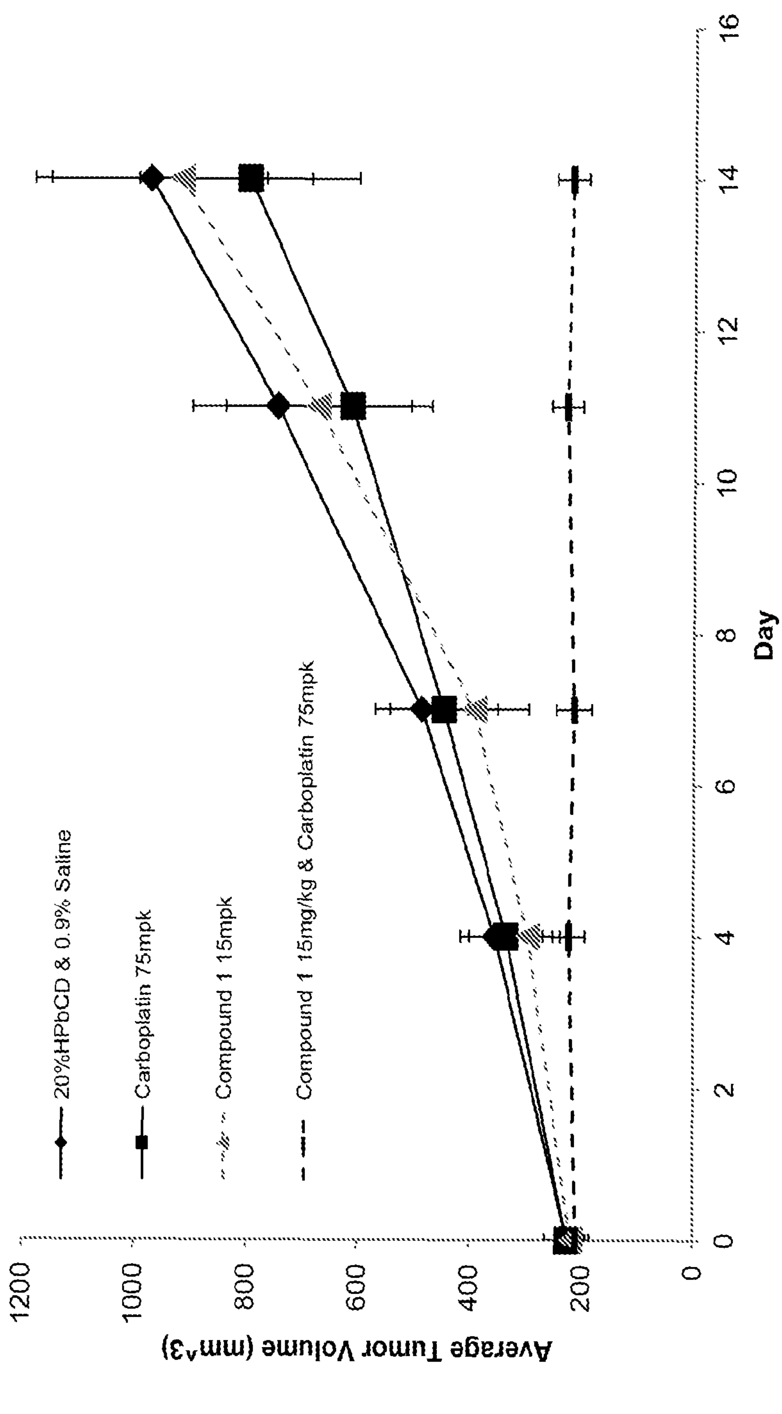

**FIGURE 2b: The anti-tumor activity of Compound 1 and carboplatin in the SKVO-3 xenograft model**

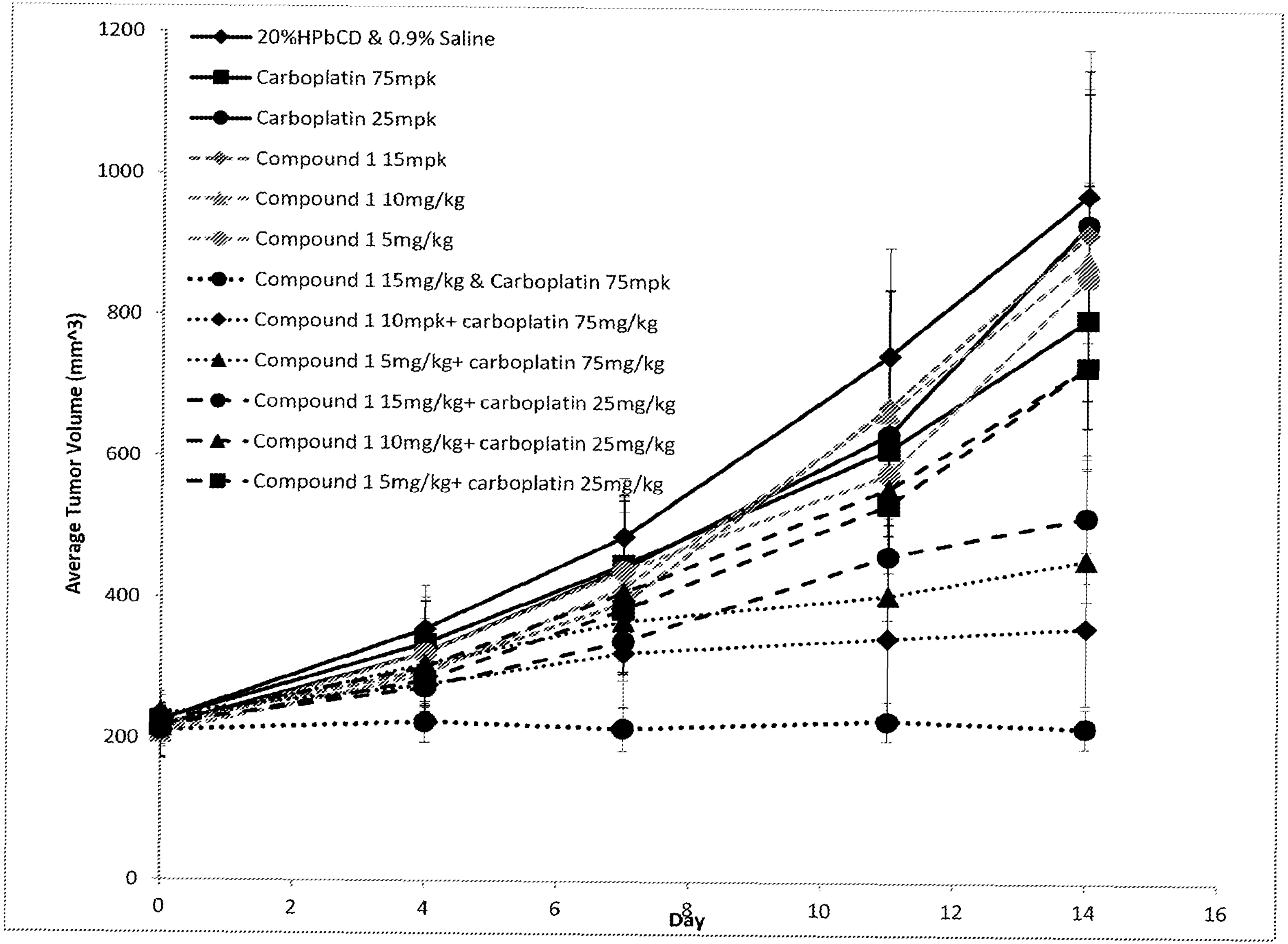

**FIGURE 3: The anti-tumor activity of Compound 1 and carboplatin in the CTG-0486 xenograft model.**

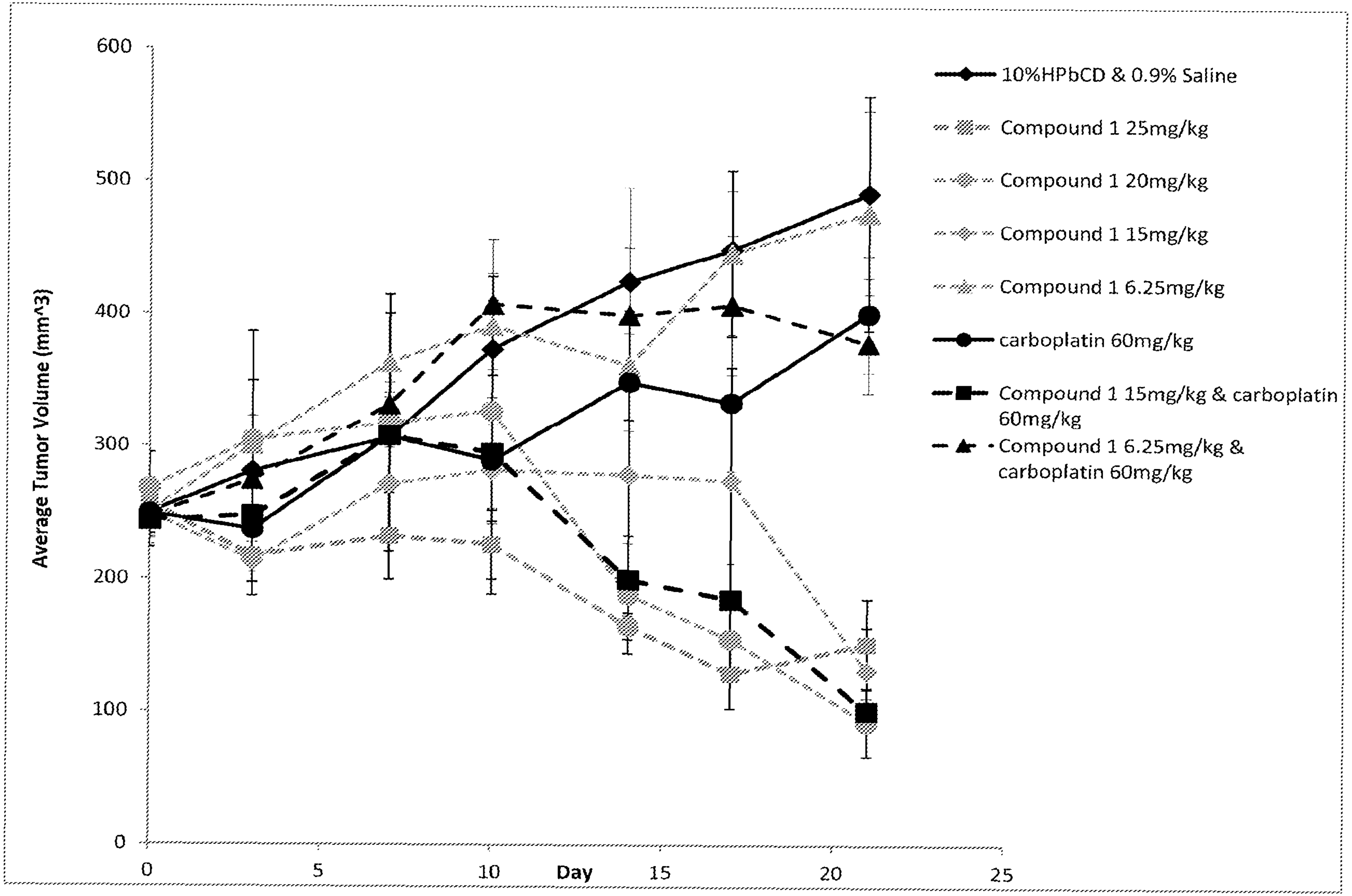

FIGURE 4: The anti-tumor activity of Compound 1 and oxaliplatin in the PHTX-24C xenograft model.

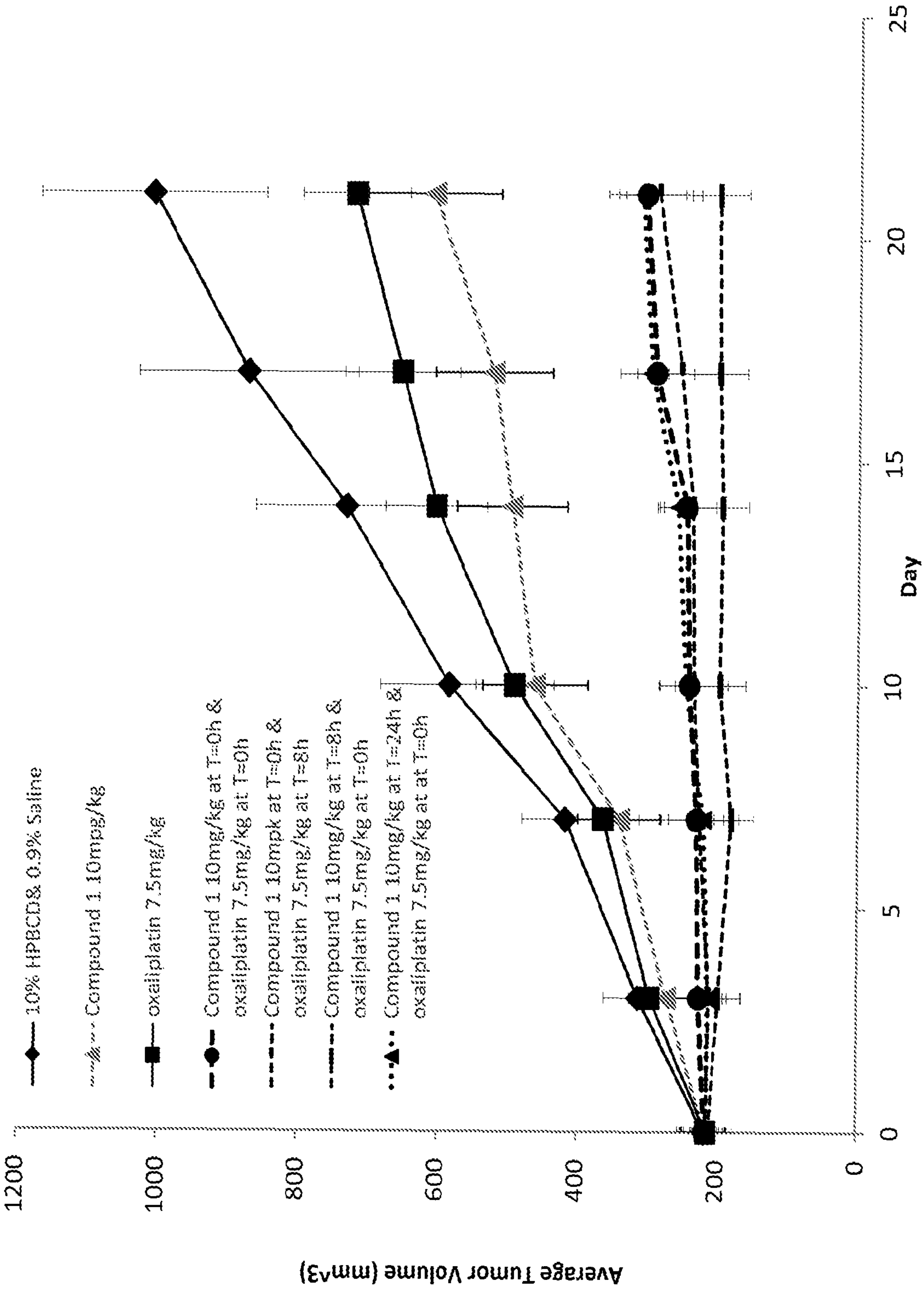

FIGURE 5: The anti-tumor activity of Compound 1 and docetaxel in the PHTX-02B xenograft model

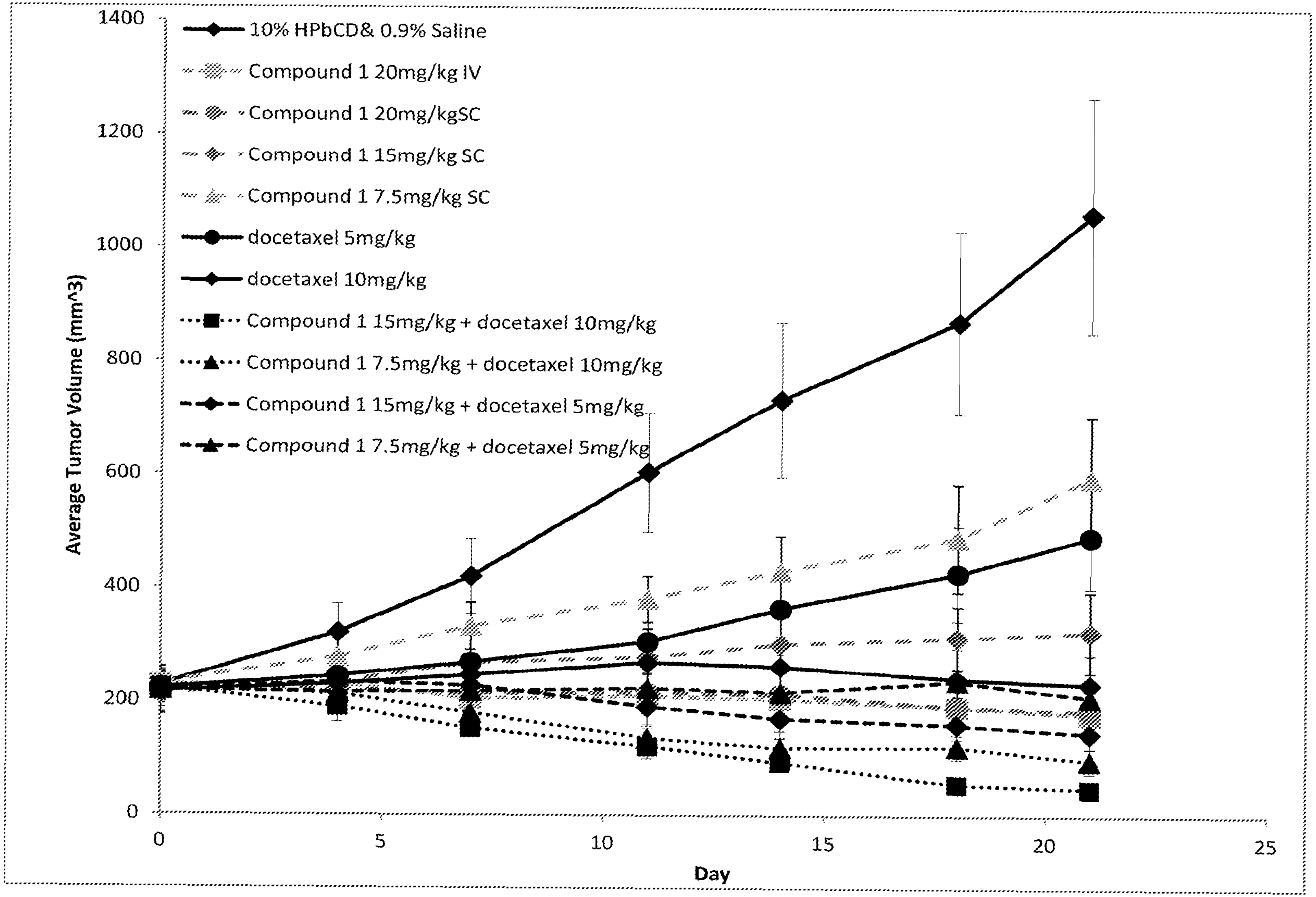

**FIGURE 6a: The anti-tumor activity of Compound 1 at 10 mg/kg and docetaxel in the Calu-6 xenograft model**

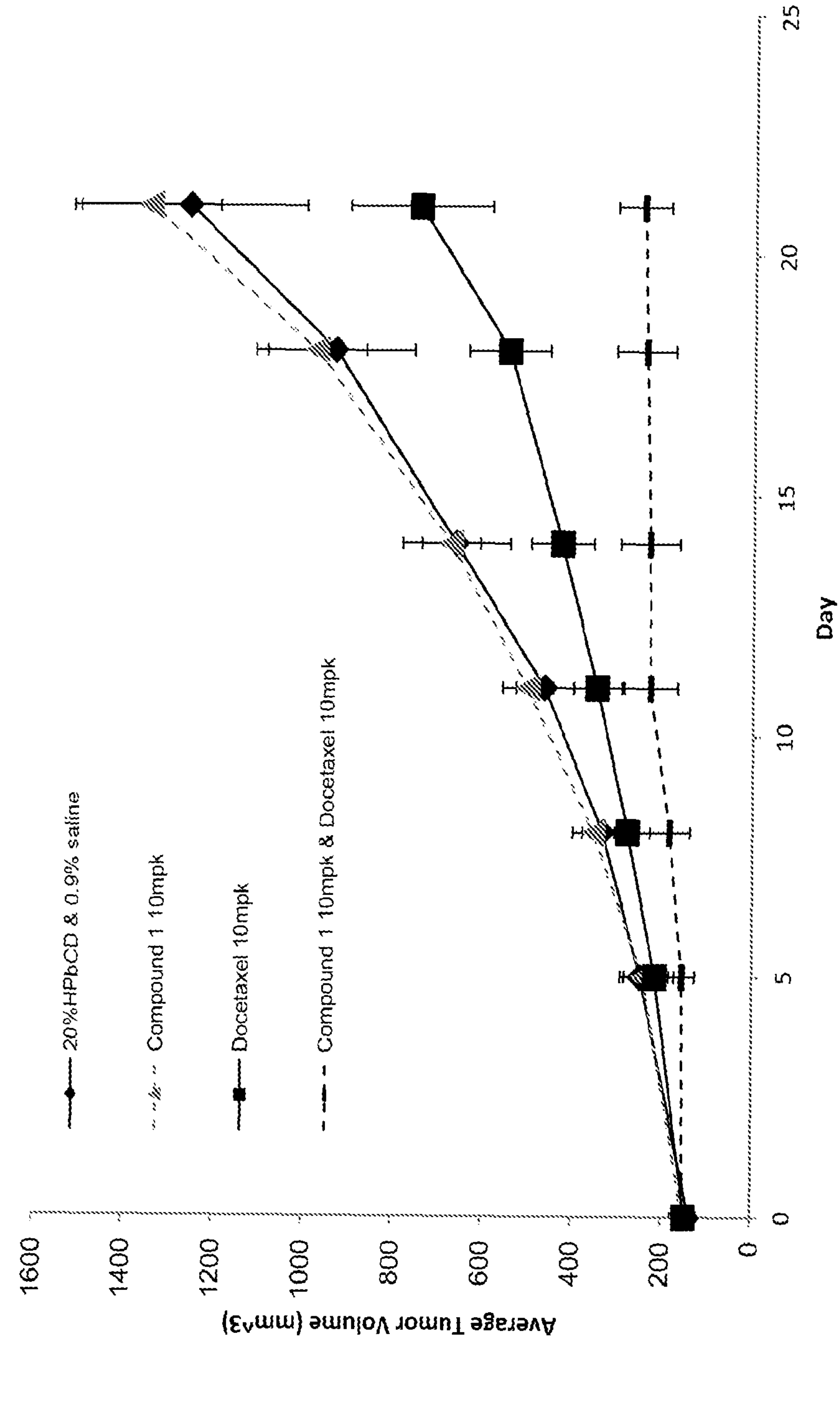

FIGURE 6b: The anti-tumor activity of Compound 1 and docetaxel in the Calu-6 xenograft model

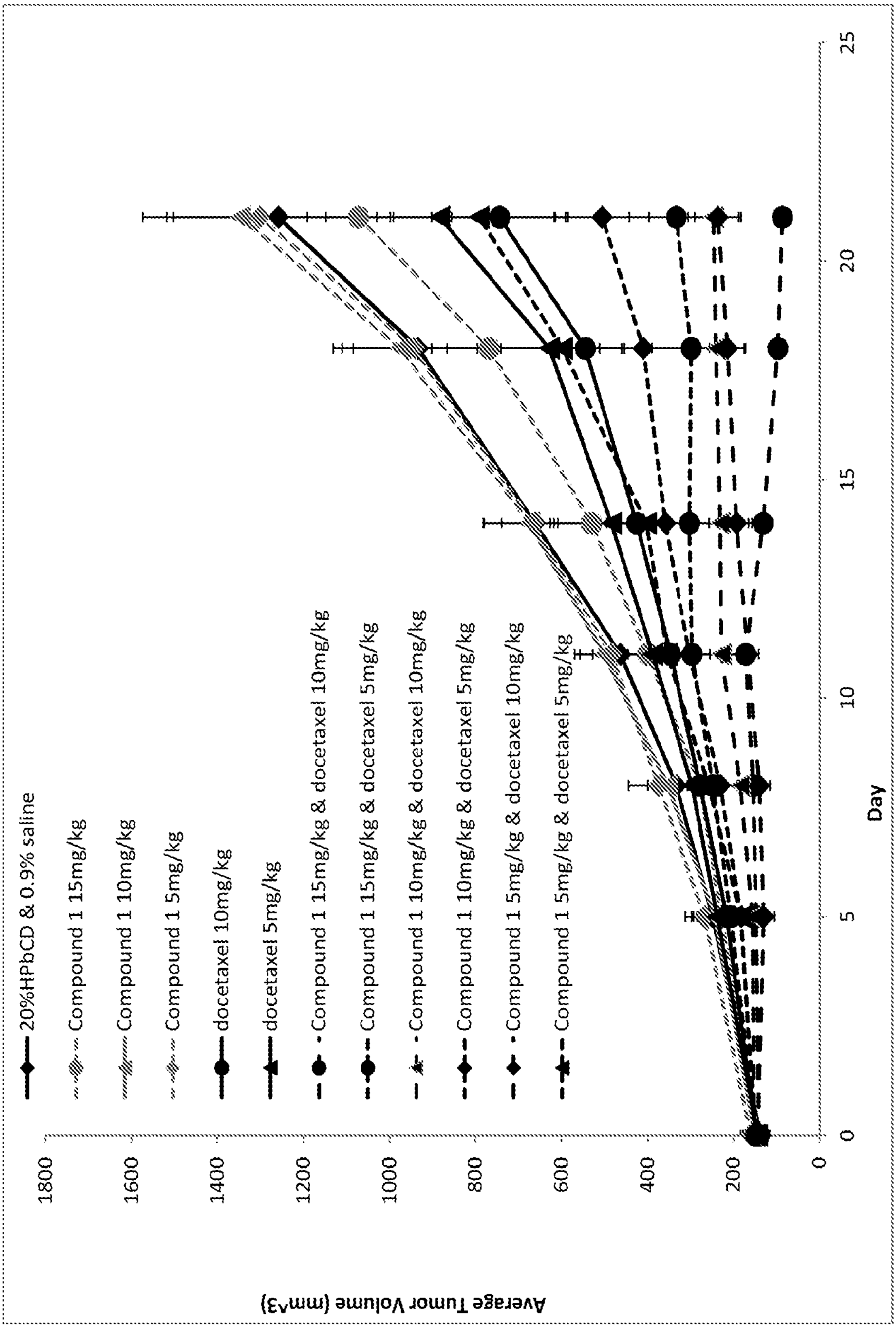

FIGURE 7: The anti-tumor activity of Compound 1 and docetaxel in the PHTX-14B xenograft model

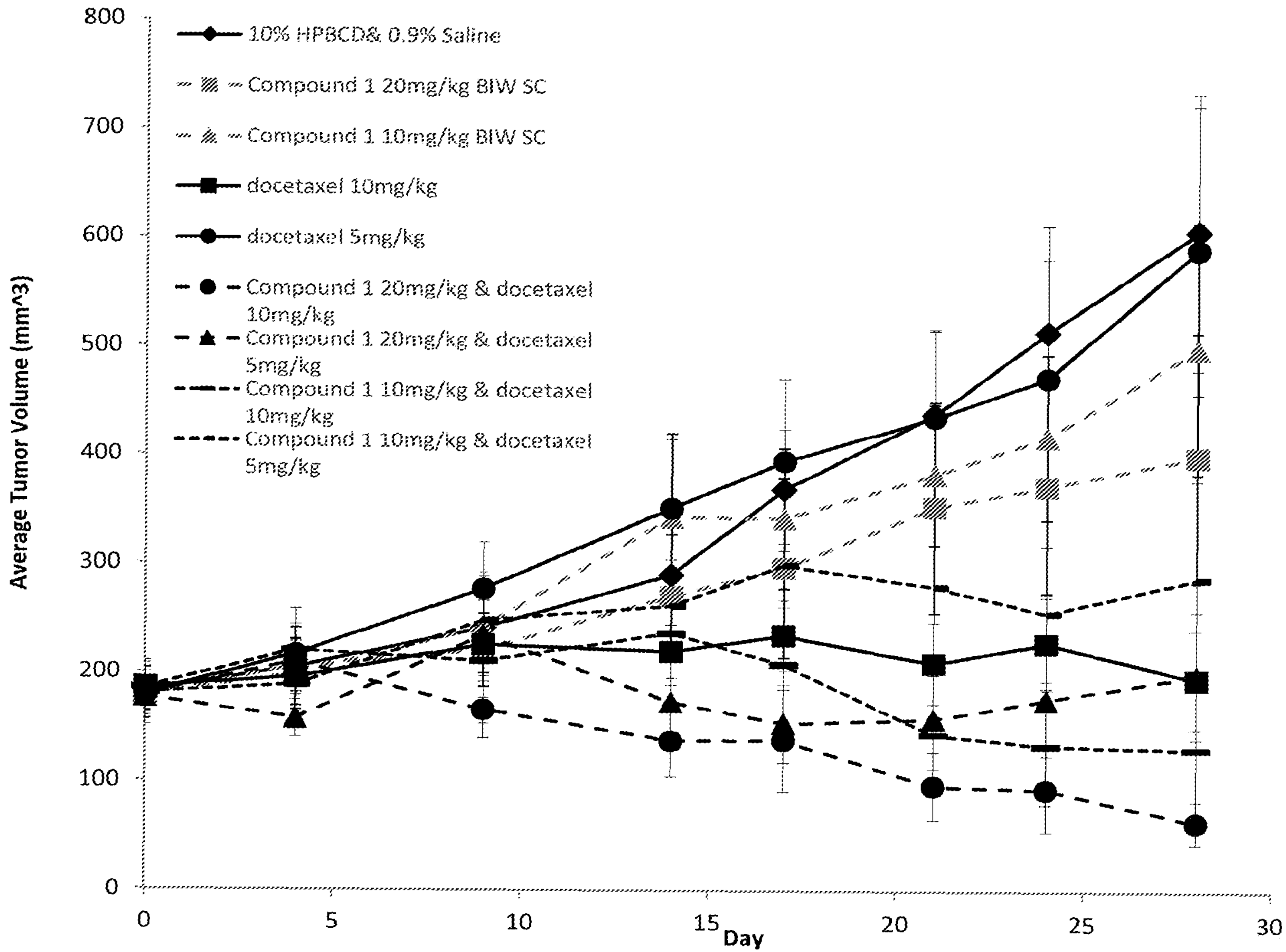

FIGURE 8: The anti-tumor activity of compound 1 and docetaxel in the CTG-0159 xenograft model

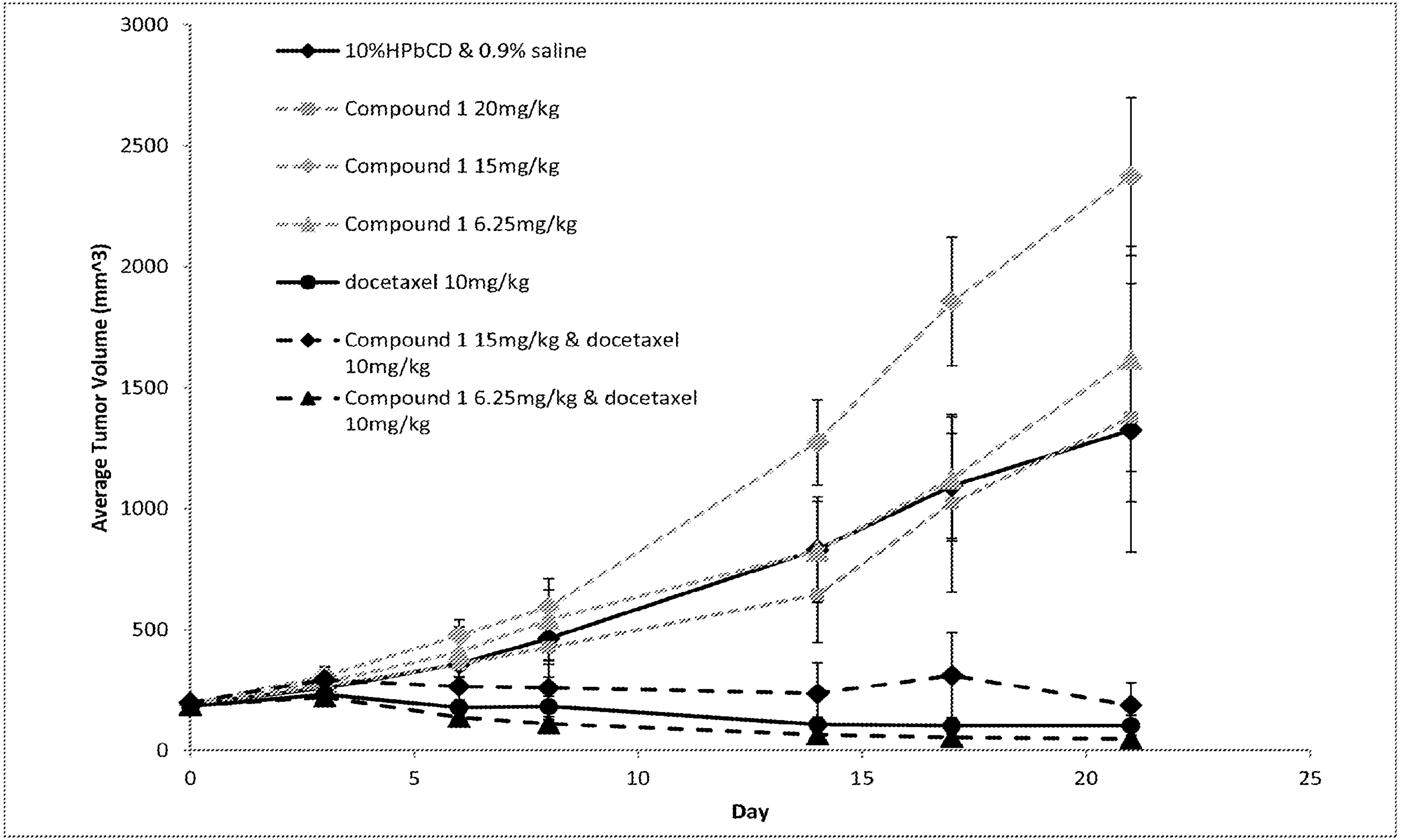

FIGURE 9: The anti-tumor activity of Compound 1 and paclitaxel in the PHTX-14B xenograft model

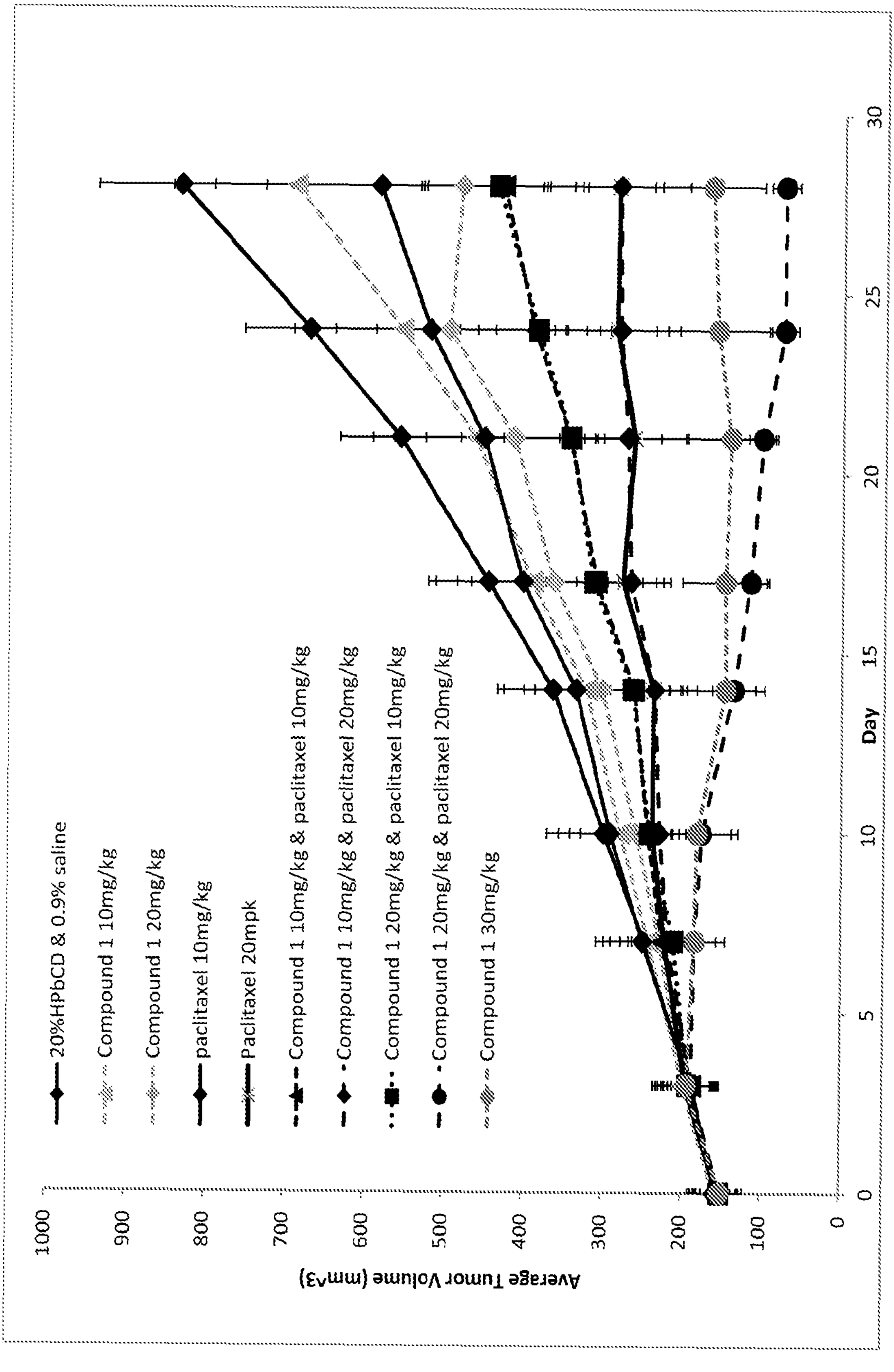

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20140088096 A
- WO 2013123169 A
- US 20130217682 A
- US 20090036678, Armitage, I.


### Non-patent literature cited in the description


- Cancer Facts and Figures. American Cancer Society, 2014
- **SCHULMAN, B.A. ; J.W. HARPER.** Ubiquitin-like protein activation by E1 enzymes: the apex for downstream signalling pathways. *Nat Rev Mol Cell Biol,* 2009, vol. 10, 319-331
- **DESHAIES, R.J. ; C.A. JOAZEIRO.** RING domain E3 ubiquitin ligases. *Annu Rev Biochem,* 2009, vol. 78, 399-434
- **LIPKOWITZ, S ; A.M. WEISSMAN.** RINGs of good and evil: RING finger ubiquitin ligases at the crossroads of tumour suppression and oncogenesis. *Nat Rev Cancer,* 2011, vol. 11, 629-643
- **ROTIN, D. ; S. KUMAR.** Physiological functions of the HECT family of ubiquitin ligases. *Nat Rev Mol Cell Biol,* 2009, vol. 10, 398-409
- **JIN, J et al.** Dual E1 activation systems for ubiquitin differentially regulate E2 enzyme charging. *Nature,* 2007, vol. 447, 1135-1138
- **CIECHANOVER, A. et al.** Ubiquitin dependence of selective protein degradation demonstrated in the mammalian cell cycle mutant ts85. *Cell,* 1984, vol. 37, 57-66
- **FINLEY, D., A. et al.** Thermolability of ubiquitin-activating enzyme from the mammalian cell cycle mutant ts85. *Cell,* 1984, vol. 37, 43-55
- **CHANDRASEKHARAN, M.B. et al.** Histone H2B ubiquitination and beyond: Regulation of nucleosome stability, chromatin dynamics and the trans-histone H3 methylation. *Epigenetics,* 2010, vol. 5, 460-468
- **TROTMAN, L.C. et al.** Ubiquitination regulates PTEN nuclear import and tumor suppression. *Cell,* 2007, vol. 128, 141-156
- **GREGORY, R.C. et al.** Regulation of the Fanconi anemia pathway by monoubiquitination. *Semin Cancer Biol,* 2003, vol. 13, 77-82
- **MOSESSON, Y. ; Y. YARDEN.** Monoubiquitylation: a recurrent theme in membrane protein transport. *Isr Med Assoc J,* 2006, vol. 8, 233-237
- **BEHRENDS, C. ; J.W. HARPER.** Constructing and decoding unconventional ubiquitin chains. *Nat Struct Mol Biol,* 2011, vol. 18, 520-528
- **BENNETT, E.J. ; J.W. HARPER.** DNA damage: ubiquitin marks the spot. *Nat Struct Mol Biol,* 2008, vol. 15, 20-22
- **KOMANDER, D.** The emerging complexity of protein ubiquitination. *Biochem Soc Trans,* 2009, vol. 37, 937-953
- **XU, W.G. et al.** The ubiquitin-activating enzyme E1 as a therapeutic target for the treatment of leukemia and multiple myeloma. *Blood,* 2010, vol. 115, 2251-59
- **COCKCROFT DW ; GAULT MH.** Prediction of creatinine clearance from serum creatinine. *Nephron,* 1976, vol. 16 (1), 31-41
- *U.S. Food and Drug Administration,* 1978
- **HARTMANN ; LIPP.** *Exper. Opin. Pharmacother.,* 2003, vol. 4 (6), 889-901
- **BERGE et al.** *J. Pharm. Sci.,* 1977, vol. 66, 1-19
- Remington: The Science and Practice of Pharmacy. Lippincott Williams & Wilkins, 2000
- **ARMITAGE, I. et al.** *Org. Lett.,* 2012, vol. 14, 2626-2629
- **SAKSENA, A. K.** *Tetrahedron Lett,* 1980, vol. 21, 133-136
- **HUTCHINSON, E. J. ; TAYLOR, B. F ; BLACKBURN, G. M.** *J. Chem. Soc., Chem. Commun.,* 1997, 1859-1860
- **TOKORO, Y.** *Chem. Commun,* 1999, 807-809
- **DOMINGUEZ, B. M. ; CULLIS, P. M.** *Tetrahedron Lett.,* 1999, vol. 40, 5783-5786